# EUROPEAN PATENT APPLICATION

(11) **EP 4 295 909 A2**
(43) Date of publication of application: **27.12.2023**
(21) Application number: 23192663.5
(22) Date of filing: 20.04.2017
(51) Int. Cl.: A61P 25/16

(54) **CARBIDOPA AND L-DOPA PRODRUGS AND METHODS OF USE**

(30) Priority: 20.04.2016 US 201662325200 P
(62) Divisional of application: 17721269.3
(71) Applicant: AbbVie Inc., North Chicago, IL 60064 (US)
(72) Inventor: KYM, Philip R., Libertyville, IL (US); VOIGHT, Eric, Pleasant Prairie, WI (US)
(74) Representative: Garner, Stephen

(57) **Abstract**

The present disclosure relates to (a) carbidopa prodrugs, (b) pharmaceutical combinations and compositions comprising a carbidopa prodrug and/or an L-dopa prodrug, and (c) methods of treating Parkinson's disease and associated conditions comprising administering a carbidopa prodrug and an L-dopa prodrug to a subject with Parkinson's disease.

## Description

### FIELD OF THE INVENTION

The present disclosure relates to (a) carbidopa prodrugs, (b) L-dopa prodrugs, (c) pharmaceutical combinations and compositions comprising a carbidopa prodrug and/or an L-dopa prodrug, and (d) methods of treating Parkinson's disease and associated conditions comprising administering a carbidopa prodrug and an L-dopa prodrug to a subject with Parkinson's disease.

### BACKGROUND OF THE INVENTION

Parkinson's disease is a chronic and progressive neurodegenerative condition characterized by reduced levels in the brain of the neurotransmitter dopamine (i.e., 3,4-dihydroxyphenethylamine). Administration of L-dopa (i.e., L-3,4-dihydroxyphenylalanine) currently is the most effective therapy for treating a patient with Parkinson's disease. L-dopa, which unlike dopamine can cross the blood-brain barrier, is enzymatically converted in the brain to dopamine resulting in an increase in dopamine levels:

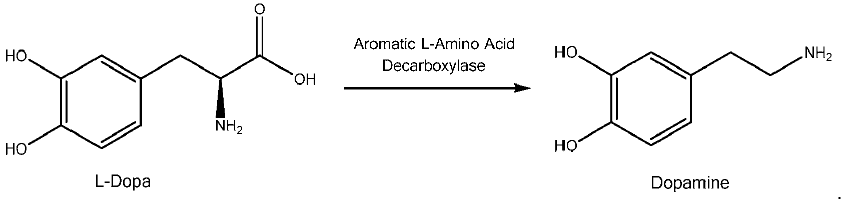

The conversion of L-dopa to dopamine is catalyzed by aromatic L-amino acid decarboxylase, a ubiquitous enzyme that promotes central as well as peripheral metabolism of L-dopa to dopamine. Due to the peripheral metabolism of L-dopa, a relatively large dose of L-dopa is required to achieve therapeutically effective dopamine levels in the brain. Administration of such large L-dopa doses results in elevated peripheral dopamine levels that can cause nausea in some patients. To overcome these problems, L-dopa generally is co-administered with a peripheral aromatic L-amino acid decarboxylase inhibitor such as carbidopa (i.e., (2S)-3-(3,4-dihydroxyphenyl)-2-hydrazino-2-methylpropanoic acid): 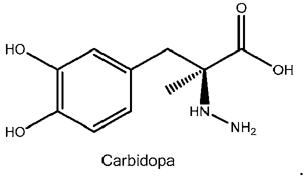 Co-administration of carbidopa with L-dopa inhibits the peripheral metabolism of L-dopa to dopamine, which significantly reduces the L-dopa dose required for a therapeutically effective response and reduces the associated side effects.

Even when L-dopa and carbidopa are co-administered, however, it is difficult to consistently maintain the desired dopamine levels in the brain due to the relatively short half-life of L-dopa in plasma. In addition, the tolerance of many patients to variability in dopamine levels in the brain decreases as the disease progresses. One approach that has been effective in reducing variability of dopamine levels is the continuous intestinal delivery of an adjustable dose of an L-dopa/carbidopa gel known by its commercial name, DuoDopa^{®} in Europe and Duopa^{®} in the United States. DuoDopa^{®}/Duopa^{®} is a suspension of L-dopa/carbidopa monohydrate (4:1 ratio of L-dopa to carbidopa monohydrate) in an aqueous gel (carboxymethyl cellulose sodium) having a viscosity that permits homogeneous distribution of micronized substance particles. The gel is delivered to the proximal small intestine through a jejunal tube inserted through a percutaneous endoscopic gastrostomy port. DuoDopa^{®}/Duopa^{®} is packaged in medication cassette reservoirs and continuously administered via a software-controlled ambulatory infusion pump. Although L-dopa and carbidopa have been co-administered to treat Parkinson's disease for several decades, a pharmacokinetically-consistent delivery system that does not require intestinal insertion is not commercially available.

A major challenge to the development of less invasive or otherwise improved modes of administering L-dopa and carbidopa has been the solubility of those compounds. They each have low aqueous solubility at the pH range required for infusion. Stable, more highly concentrated, and/or less viscous formulations comprising L-dopa and/or carbidopa (or compounds capable of *in vivo* bioconversion to L-dopa and/or carbidopa) are desirable. Such formulations can provide advantages over existing intestinal infusion therapy including: (a) decreasing the volume and improving the pumpability of the formulation to be delivered to the patient which also allows for a reduction of the size and weight of delivery device; (b) extending the shelf life of the formulation by reducing degradation and improving stability of the formulation; and/or (c) providing the patient with increased flexibility in managing their treatment by reducing or eliminating cold storage requirements for the formulation (e.g., longer times to handle the formulation outside of refrigerated storage). Such stable, more highly concentrated, and/or less viscous formulations also can be employed in less invasive modes of administration (e.g., subcutaneous infusion).

Accordingly, there is a continuing need for improved compositions and methods that can provide continuous and consistent dopamine levels in the brain to effectively treat movement disorders such as Parkinson's disease. The present disclosure provides such improved compositions and methods.

### SUMMARY OF THE INVENTION

In one aspect, the present disclosure relates to a compound corresponding in structure to Formula (I): 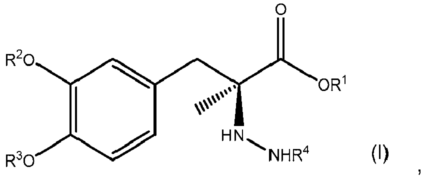 or a pharmaceutically acceptable salt thereof, wherein **R¹** is independently selected from the group consisting of hydrogen, 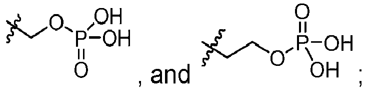 **R²** and **R³** are each independently selected from the group consisting of hydrogen, 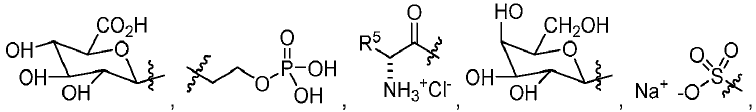 and 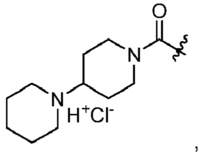 wherein **R⁵** is selected from the group consisting of hydrogen, methyl, and isopropyl; and **R⁴** is independently selected from the group consisting of hydrogen,

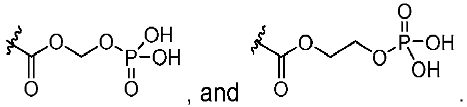

In another aspect, the present disclosure relates to a compound corresponding in structure Formula (II): 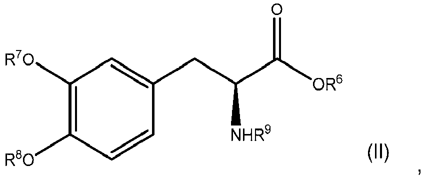 or a pharmaceutically acceptable salt thereof, wherein **R⁶** is independently selected from the group consisting of hydrogen, 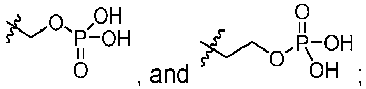 **R⁷** and **R⁸** are each independently selected from the group consisting of hydrogen, 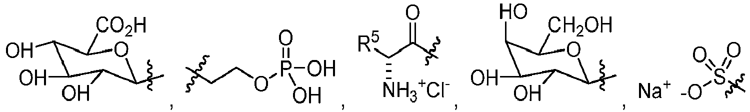 , and 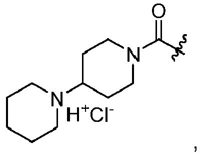 wherein **R⁵** is selected from the group consisting of hydrogen, methyl, and isopropyl; and **R⁹** is independently selected from the group consisting of hydrogen,

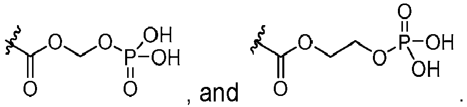

In another aspect, the present disclosure relates to a pharmaceutical combination comprising a first compound corresponding in structure to Formula (I), or a pharmaceutically acceptable salt thereof, and a second compound corresponding in structure to Formula (II) or a pharmaceutically acceptable salt thereof.

In another aspect, the present disclosure relates to a pharmaceutical composition comprising a first compound corresponding in structure to Formula (I), or a pharmaceutically acceptable salt thereof, and a pharmaceutically acceptable carrier. In certain aspects, the pharmaceutical composition may further comprise a second compound corresponding in structure to Formula (II) or a pharmaceutically acceptable salt thereof.

In another aspect, the present disclosure relates to a method of treating Parkinson's disease or an associated condition in a patient comprising administering to the patient a therapeutically effective amount of a pharmaceutical combination comprising a first compound corresponding in structure to Formula (I), or a pharmaceutically acceptable salt thereof, and a second compound corresponding in structure to Formula (II), or a pharmaceutically acceptable salt thereof. In certain aspects, the method comprises administering the first compound corresponding in structure to Formula (I), or a pharmaceutically acceptable salt thereof, and the second compound corresponding in structure to Formula (II) in a single pharmaceutical composition or in separate pharmaceutical compositions.

Further benefits of the present disclosure will be apparent to one skilled in the art from reading this patent application. The embodiments of the disclosure described in the following paragraphs are intended to illustrate the invention and should not be deemed to narrow the scope of the invention.

### DETAILED DESCRIPTION OF THE INVENTION

This written description uses examples to disclose the invention, including the best mode, and also to enable any person skilled in the art to practice the invention, including making and using any of the disclosed carbidopa prodrugs or pharmaceutical compositions, and performing any of the disclosed methods or processes. The patentable scope of the invention is defined by the claims, and may include other examples that occur to those skilled in the art. Such other examples are intended to be within the scope of the claims if they have elements that do not differ from the literal language of the claims, or if they include equivalent elements.

### I. Definitions

Section headings as used in this section and the entire disclosure are not intended to be limiting.

Where a numeric range is recited, each intervening number within the range is explicitly contemplated with the same degree of precision. For example, for the range 6 to 9, the numbers 7 and 8 are contemplated in addition to 6 and 9, and for the range 6.0 to 7.0, the numbers 6.0, 6.1, 6.2, 6.3, 6.4, 6.5, 6.6, 6.7, 6.8, 6.9 and 7.0 are explicitly contemplated. In the same manner, all recited ratios also include all sub-ratios falling within the broader ratio.

The singular forms "a," "an" and "the" include plural referents unless the context clearly dictates otherwise.

The term "and/or" as used in a phrase such as "A and/or B" herein is intended to include "A and B", "A or B", "A", and "B".

The term "about" generally refers to a range of numbers that one of skill in the art would consider equivalent to the recited value (*i.e.,* having the same function or result). In many instances, the term "about" may include numbers that are rounded to the nearest significant figure.

Unless the context requires otherwise, the terms "comprise," "comprises," and "comprising" are used on the basis and clear understanding that they are to be interpreted inclusively, rather than exclusively, and that Applicant intends each of those words to be so interpreted in construing this patent, including the claims below.

The terms "improve" and "improving" have their plain and ordinary meaning to one skilled in the art of pharmaceutical or medical sciences and specifically include ameliorating the effects of Parkinson's disease, or decreasing or lessening a side effect of Parkinson's disease.

The term "patient" includes mammals and humans, particularly humans.

The term "pharmaceutically acceptable carrier" or "pharmaceutically acceptable excipient" refers to any and all solvents, dispersion media, preservatives, antioxidants, coatings, isotonic and absorption delaying agents, and the like, that are compatible with pharmaceutical administration.

The term "pharmaceutically acceptable salt" refers to a salt of a compound that is pharmaceutically acceptable and that possesses the desired pharmacological activity of the parent compound. Such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1 ,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methyl-bicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; and (2) salts formed when an acidic proton present in the parent compound either is replaced by a metal ion, e.g., an alkali metal ion, an alkaline earth ion, or an aluminum ion; or coordinates with an organic base such as ethanolamine, diethanolamine, triethanolamine, N-methylglucamine, dicyclohexylamine, and the like.

The terms "reduce" and "reducing" have their plain and ordinary meanings to one skilled in the art of pharmaceutical or medical sciences and specifically include diminishing or decreasing the number of occurrences, the duration, or the intensity, of a Parkinson's disease side effect, such as dyskinesias or hallucinations.

The term "therapeutically effective amount" means an amount of a compound that, when administered to a patient suffering from or susceptible to Parkinson's disease or an associated condition is sufficient, either alone or in combination with additional therapies, to effect treatment for Parkinson's disease or the associated condition. The "therapeutically effective amount" will vary depending, for example, on the compound, the condition treated and its severity, and the age and weight of the patient to be treated.

The terms "treat" and "treating" have their plain and ordinary meaning to one skilled in the art of pharmaceutical or medical sciences and specifically include improving the quality of life or reducing the symptoms or side effects of Parkinson's disease.

### II. Carbidopa and L-Dopa Prodrugs

As previously noted, the inherently low aqueous solubility of L-dopa and carbidopa at physiologically acceptable pH for infusion presents a significant technical challenge to the development of improved pharmaceutical compositions and methods of treatment. Such challenges include, for example, difficulties in achieving appropriate dosing volume and formulation stability within the required pH limitations. These challenges are further complicated by the requirement that the pharmaceutical compositions and methods of treatment provide pharmacokinetically-appropriate and pharmacokinetically-consistent control of dopamine levels in the patient's brain.

Prior prodrug approaches have failed for a number of reasons due to these technical challenges (including insufficient chemical stability, insufficient solubility, *in vivo* bioconversion issues, and the like) and no L-dopa prodrugs or carbidopa prodrugs for infusion have been successfully commercialized. The prodrugs, pharmaceutical combinations and compositions, and methods of treatment of the present disclosure, however, have overcome these challenges. They can be used to treat patients suffering from Parkinson's disease and associated conditions and do not always require invasive surgery. In various embodiments of the present disclosure, the compositions comprise L-dopa and carbidopa prodrugs that convert to L-dopa and carbidopa *in vivo* which allows for delivery by continuous administration methods including intragastric, intramuscular, intravenous, and subcutaneous administration. These novel prodrugs, combinations, compositions, and methods of the present disclosure represent an advancement in the treatment of Parkinson's disease and other related conditions.

### A. Carbidopa Prodrugs

In one embodiment, therefore, the present disclosure relates to a compound corresponding in structure to Formula (I): 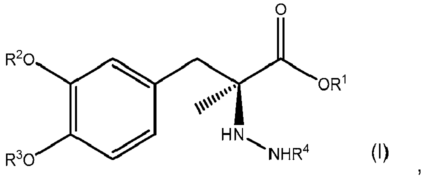 or a pharmaceutically acceptable salt thereof, wherein **R¹** is independently selected from the group consisting of hydrogen, 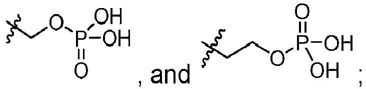 **R²** and **R³** are each independently selected from the group consisting of hydrogen, 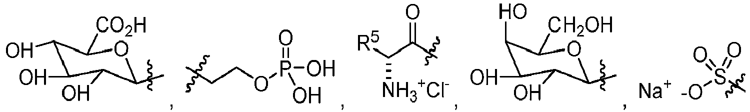 , and 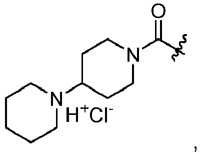 wherein **R⁵** is selected from the group consisting of hydrogen, methyl, and isopropyl; and **R⁴** is independently selected from the group consisting of hydrogen, 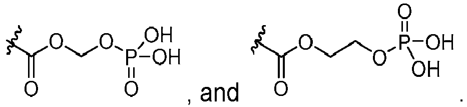 In one aspect, the compound corresponds in structure to Formula (I). In another aspect, the compound is a pharmaceutically acceptable salt of a compound corresponding in structure to Formula (I).

In one embodiment, **R**² and **R³** are both the same (e*.g.,* both hydrogen, both 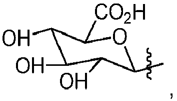 etc.). In another embodiment **R²** and **R³** are different (*e.g.,* **R²** is hydrogen and **R³** is 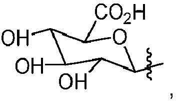 *etc*.).

Additionally or alternatively, **R²** is hydrogen and **R³** is selected from the group consisting of hydrogen, 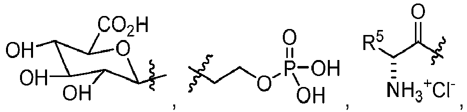 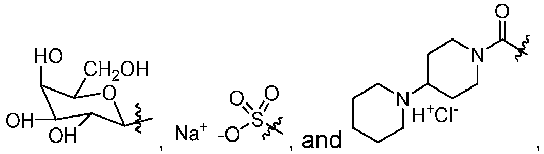 wherein **R⁵** is selected from the group consisting of hydrogen, methyl, and isopropyl.

Additionally or alternatively, **R²** is hydrogen and **R³** is selected from the group consisting of 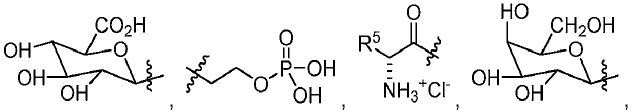 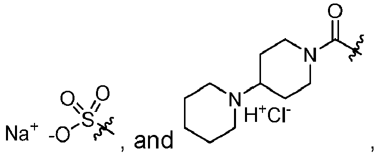 wherein **R⁵** is selected from the group consisting of hydrogen, methyl, and isopropyl.

Additionally or alternatively, **R²** is hydrogen and **R³** is

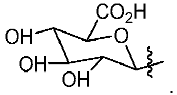

Additionally or alternatively, **R²** is hydrogen and **R³** is

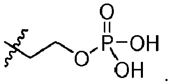

Additionally or alternatively, **R²** is hydrogen and **R³** is 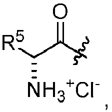 wherein **R⁵** is selected from the group consisting of hydrogen, methyl, and isopropyl.

Additionally or alternatively, **R²** is hydrogen and **R³** is

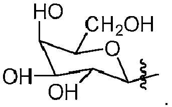

Additionally or alternatively, **R²** is hydrogen and **R³** is

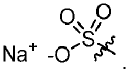

Additionally or alternatively, **R²** is hydrogen and **R³** is

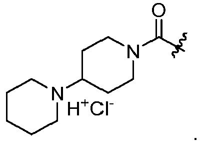

Additionally or alternatively, **R²** is hydrogen and **R³** is hydrogen.

Additionally or alternatively, **R²** is selected from the group consisting of hydrogen, 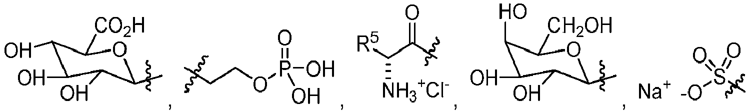 , and 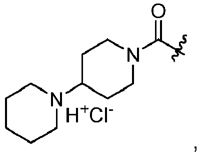 wherein **R⁵** is selected from the group consisting of hydrogen, methyl, and isopropyl and **R³** is hydrogen.

Additionally or alternatively, **R²** selected from the group consisting of 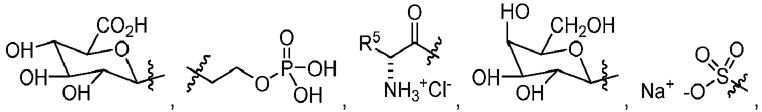 and 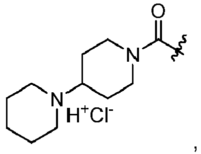 wherein **R⁵** is selected from the group consisting of hydrogen, methyl, and isopropyl and **R³** is hydrogen.

Additionally or alternatively, **R²** is 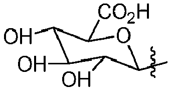 and **R³** is hydrogen.

Additionally or alternatively, **R²** is 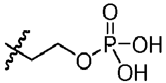 and **R³** is hydrogen.

Additionally or alternatively, **R²** is 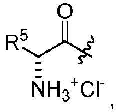 wherein **R⁵** is selected from the group consisting of hydrogen, methyl, and isopropyl and **R³** is hydrogen.

Additionally or alternatively, **R²** is 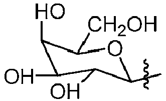 and **R³** is hydrogen.

Additionally or alternatively, **R²** is 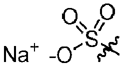 and **R³** is hydrogen.

Additionally or alternatively, **R²** is 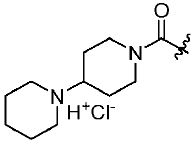 and **R³** is hydrogen.

In another embodiment, the present disclosure relates to a compound corresponding in structure to Formula (I) or a pharmaceutically acceptable salt thereof, wherein **R¹** is independently selected from the group consisting of hydrogen, 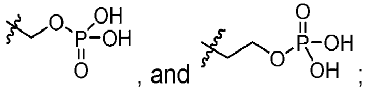 **R²** and **R³** are each hydrogen, and **R⁴** is independently selected from the group consisting of hydrogen, 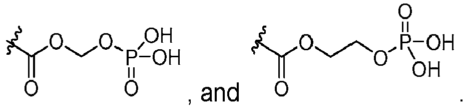 In one aspect, the compound corresponds in structure to Formula (I). In another aspect, the compound is a pharmaceutically acceptable salt of a compound corresponding in structure to Formula (I).

In another embodiment, the present disclosure relates to a compound corresponding in structure to Formula (I) or a pharmaceutically acceptable salt thereof, wherein **R¹** is hydrogen; **R²** and **R³** are each independently selected from the group consisting of hydrogen, 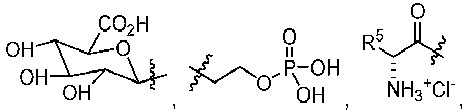 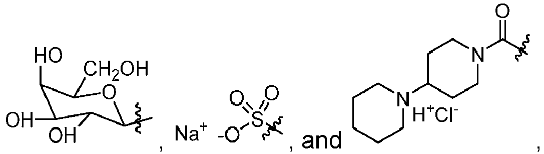 wherein **R⁵** is selected from the group consisting of hydrogen, methyl, and isopropyl; and **R⁴** is independently selected from the group consisting of hydrogen, 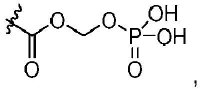 and 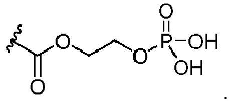 In one aspect, the compound corresponds in structure to Formula (I). In another aspect, the compound is a pharmaceutically acceptable salt of a compound corresponding in structure to Formula (I).

In another embodiment, the present disclosure relates to a compound corresponding in structure to Formula (I) or a pharmaceutically acceptable salt thereof, wherein **R¹** is hydrogen; **R²** and **R³** are hydrogen; and **R⁴** is independently selected from the group consisting of hydrogen, 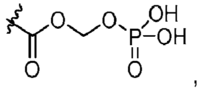 and 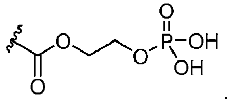 In one aspect, the compound corresponds in structure to Formula (I). In another aspect, the compound is a pharmaceutically acceptable salt of a compound corresponding in structure to Formula (I).

In another embodiment, the present disclosure relates to a compound corresponding in structure to Formula (I-a): 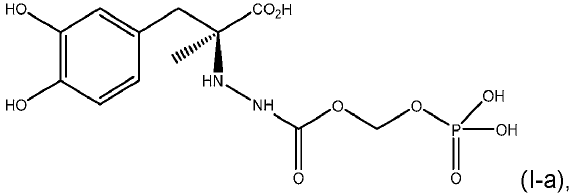 or a pharmaceutically acceptable salt thereof. In one aspect, the compound corresponds in structure to Formula (I-a). In another aspect, the compound is a pharmaceutically acceptable salt of a compound corresponding in structure to Formula (I-a).

In another embodiment, the present disclosure relates to a compound corresponding in structure to Formula (I-b): 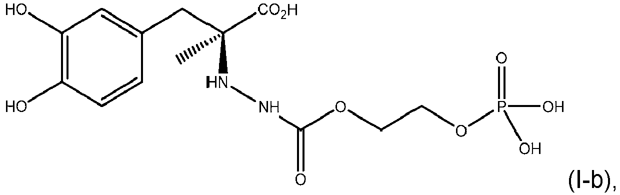 or a pharmaceutically acceptable salt thereof. In one aspect, the compound corresponds in structure to Formula (I-b). In another aspect, the compound is a pharmaceutically acceptable salt of a compound corresponding in structure to Formula (I-b).

In another embodiment, the present disclosure relates to a compound corresponding in structure to Formula (I) or a pharmaceutically acceptable salt thereof, wherein **R¹** is independently selected from the group consisting of hydrogen, 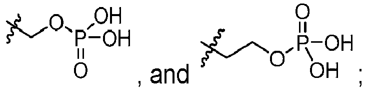 **R²** and **R³** are each independently selected from the group consisting of hydrogen, 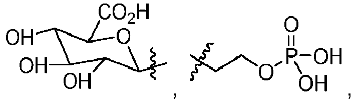 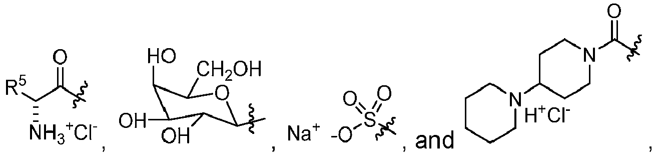 wherein **R⁵** is selected from the group consisting of hydrogen, methyl, and isopropyl; and **R⁴** is hydrogen. In one aspect, the compound corresponds in structure to Formula (I). In another aspect, the compound is a pharmaceutically acceptable salt of a compound corresponding in structure to Formula (I).

In another embodiment, the present disclosure relates to a compound corresponding in structure to Formula (I) or a pharmaceutically acceptable salt thereof, wherein **R¹** is hydrogen; **R²** and **R³** are each independently selected from the group consisting of hydrogen, 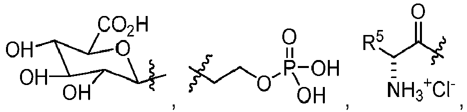 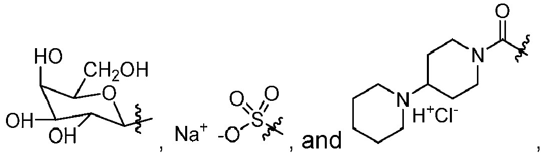 wherein **R⁵** is selected from the group consisting of hydrogen, methyl, and isopropyl; and **R⁴** is hydrogen. In one aspect, the compound corresponds in structure to Formula (I). In another aspect, the compound is a pharmaceutically acceptable salt of a compound corresponding in structure to Formula (I).

In another embodiment, the present disclosure relates to a compound corresponding in structure to Formula (I) or a pharmaceutically acceptable salt thereof, wherein **R¹** is independently selected from the group consisting of 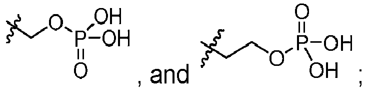 **R²** and **R³** are hydrogen; and **R⁴** is independently selected from the group consisting of hydrogen, 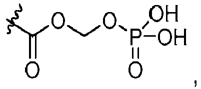 and 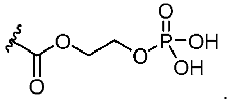 In one aspect, the compound corresponds in structure to Formula (I). In another aspect, the compound is a pharmaceutically acceptable salt of a compound corresponding in structure to Formula (I).

In another embodiment, the present disclosure relates to a compound corresponding in structure to Formula (I) or a pharmaceutically acceptable salt thereof, wherein **R¹** is independently selected from the group consisting of 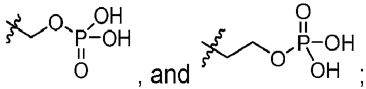 **R²** and **R³** are each independently selected from the group consisting of hydrogen, 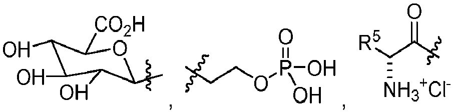 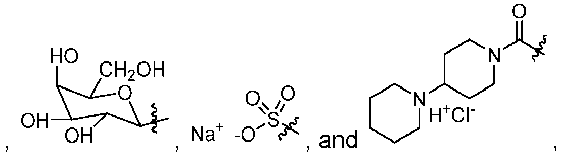 wherein **R⁵** is selected from the group consisting of hydrogen, methyl, and isopropyl; and **R⁴** hydrogen. In one aspect, the compound corresponds in structure to Formula (I). In another aspect, the compound is a pharmaceutically acceptable salt of a compound corresponding in structure to Formula (I).

In another embodiment, the present disclosure relates to a compound corresponding in structure to Formula (I) or a pharmaceutically acceptable salt thereof, wherein **R¹** is independently selected from the group consisting of 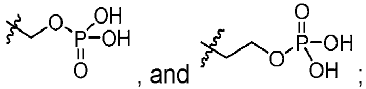 **R²** and **R³** are each independently selected from the group consisting of hydrogen, 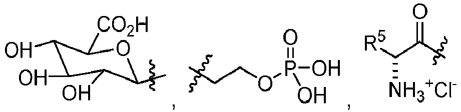 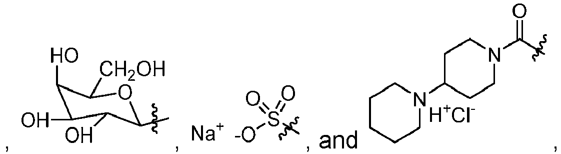 wherein **R⁵** is selected from the group consisting of hydrogen, methyl, and isopropyl; and **R⁴** hydrogen. In one aspect, the compound corresponds in structure to Formula (I). In another aspect, the compound is a pharmaceutically acceptable salt of a compound corresponding in structure to Formula (I).

In another embodiment, the present disclosure relates to a compound corresponding in structure to Formula (I) or a pharmaceutically acceptable salt thereof, wherein **R¹** is independently selected from the group consisting of 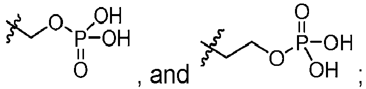 **R²** and **R³** are hydrogen; and **R⁴** hydrogen. In one aspect, the compound corresponds in structure to Formula (I). In another aspect, the compound is a pharmaceutically acceptable salt of a compound corresponding in structure to Formula (I).

Various other compounds corresponding in structure to Formula (I) or pharmaceutically acceptable salts thereof are contemplated herein. Examples of suitable compounds corresponding in structure to Formula (I) are provided below in Table 1, which provides R¹, R², R³, **R⁴** and/or R⁵ substituents for compounds corresponding in structure to Formula (I).

**Table 1**

| **Compound** | **R¹** | **R²** | **R³** | **R⁴** | **R⁵** |
|---|---|---|---|---|---|
| (I-c) | 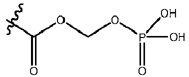 | hydrogen | hydrogen | hydrogen | |
| (I-d) | 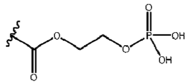 | hydrogen | hydrogen | hydrogen | |
| (I-e) | hydrogen | hydrogen | 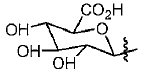 | hydrogen | |
| (I-f) | hydrogen | hydrogen | 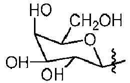 | hydrogen | |
| (I⁻g) | hydrogen | hydrogen | 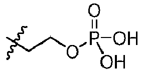 | hydrogen | |
| (I-h) | hydrogen | hydrogen | 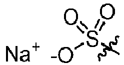 | hydrogen | |
| (I-i) | hydrogen | hydrogen | 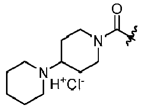 | hydrogen | |
| (I-j) | hydrogen | hydrogen | 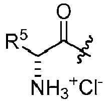 | hydrogen | hydrogen |
| (I-k) | hydrogen | hydrogen | 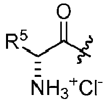 | hydrogen | Methyl |
| (I-l) | hydrogen | hydrogen | 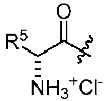 | hydrogen | Isopropyl |
| (I-m) | hydrogen | 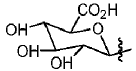 | hydrogen | hydrogen | |
| (I-n) | hydrogen | 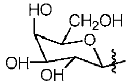 | hydrogen | hydrogen | |
| (I-o) | hydrogen | 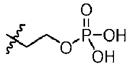 | hydrogen | hydrogen | |
| (I-p) | hydrogen | 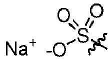 | hydrogen | hydrogen | |
| (I-q) | hydrogen | 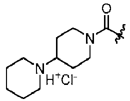 | hydrogen | hydrogen | |
| (I-r) | hydrogen | 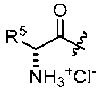 | hydrogen | hydrogen | hydrogen |
| (I-s) | hydrogen | 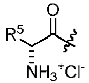 | hydrogen | hydrogen | Methyl |
| (I-t) | hydrogen | 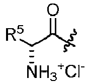 | hydrogen | hydrogen | Isopropyl |
| (I-u) | hydrogen | 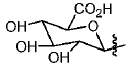 | 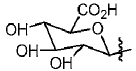 | hydrogen | |
| (I-v) | hydrogen | 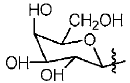 | 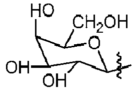 | hydrogen | |
| (I-w) | hydrogen | 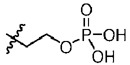 | 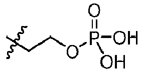 | hydrogen | |
| (I-x) | hydrogen | 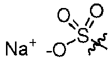 | 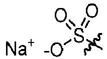 | hydrogen | |
| (I-y) | hydrogen | 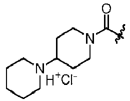 | 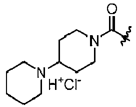 | hydrogen | |
| (I-z) | hydrogen | 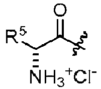 | 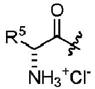 | hydrogen | hydrogen |
| (I-aa) | hydrogen | 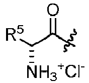 | 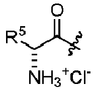 | hydrogen | Methyl |
| (I-ab) | hydrogen | 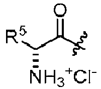 | 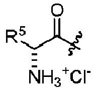 | hydrogen | Isopropyl |
| (I-ac) | hydrogen | hydrogen | hydrogen | hydrogen | |

In various embodiments, the present disclosure relates to a compound corresponding in structure to any one of Formula (I-c), (I-d), (I-e), (I-f), (I-g), (I-h), (I-i), (I-j), (I-k), (I-l), (I-m), (I-n), (I-o), (I-p), (I-q), (I-r), (I-s), (I-t), (I-u), (I-v), (I-w), (I-x), (I-y), (I-z), (I-aa), (I-ab), or (I-ac) or a pharmaceutically acceptable salt thereof. In one aspect, the compound corresponds in structure to any one of Formula (I-c), (I-d), (I-e), (I-f), (I-g), (I-h), (I-i), (I-j), (I-k), (I-l), (I-m), (I-n), (I-o), (I-p), (I-q), (I-r), (I-s), (I-t), (I-u), (I-v), (I-w), (I-x), (I-y), (I-z), (I-aa), (I-ab), or (I-ac). In another aspect, the compound is a pharmaceutically acceptable salt of a compound corresponding in structure to any one of Formula (I-c), (I-d), (I-e), (I-f), (I-g), (I-h), (I-i), (I-j), (I-k), (I-l), (I-m), (I-n), (I-o), (I-p), (I-q), (I-r), (I-s), (I-t), (I-u), (I-v), (I-w), (I-x), (I-y), (I-z), (I-aa), (I-ab), or (I-ac).

### B. L-Dopa Prodrugs

In another embodiment, the present disclosure relates to a compound corresponding in structure Formula (II): 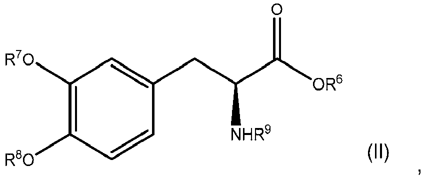 or a pharmaceutically acceptable salt thereof, wherein **R⁶** is independently selected from the group consisting of hydrogen, 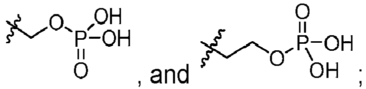 **R⁷** and **R⁸** are each independently selected from the group consisting of hydrogen, 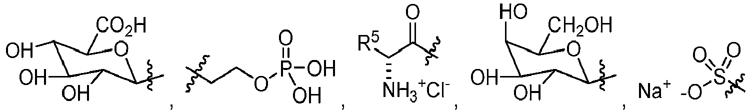 , and 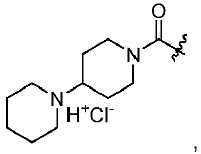 wherein **R⁵** is selected from the group consisting of hydrogen, methyl, and isopropyl; and **R⁹** is independently selected from the group consisting of hydrogen,

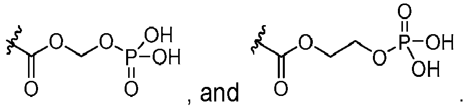

In one aspect, the compound corresponds in structure to Formula (II). In another aspect, the compound is a pharmaceutically acceptable salt of a compound corresponding in structure to Formula (II).

In one embodiment, **R⁷** and **R⁸** are both the same (*e.g.,* both hydrogen, both 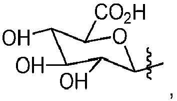 etc.). In another embodiment **R⁷** and **R⁸** are different (*e.g.,* **R⁷** is hydrogen and **R⁸** is 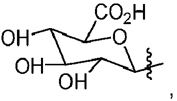 *etc*.).

Additionally or alternatively, **R⁷** is hydrogen and **R⁸** is selected from the group consisting of hydrogen, 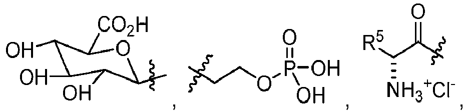 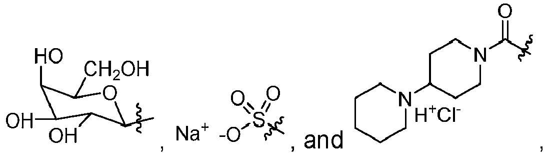 wherein **R⁵** is selected from the group consisting of hydrogen, methyl, and isopropyl.

Additionally or alternatively, **R⁷** is hydrogen and **R⁸** is selected from the group consisting of 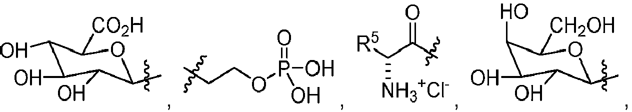 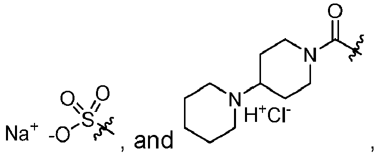 wherein **R⁵** is selected from the group consisting of hydrogen, methyl, and isopropyl.

Additionally or alternatively, **R⁷** is hydrogen and **R⁸** is

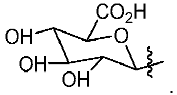

Additionally or alternatively, **R⁷** is hydrogen and **R⁸** is

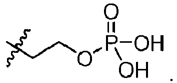

Additionally or alternatively, **R⁷** is hydrogen and **R⁸** is 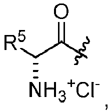 wherein **R⁵** is selected from the group consisting of hydrogen, methyl, and isopropyl.

Additionally or alternatively, **R⁷** is hydrogen and **R⁸** is

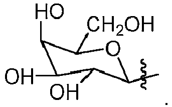

Additionally or alternatively, **R⁷** is hydrogen and **R⁸** is

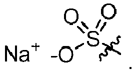

Additionally or alternatively, **R⁷** is hydrogen and **R⁸** is

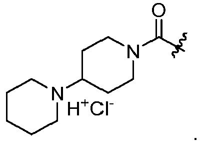

Additionally or alternatively, **R⁷** is hydrogen and **R⁸** is hydrogen.

Additionally or alternatively, **R⁷** is selected from the group consisting of hydrogen, 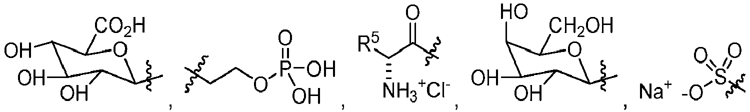 , and 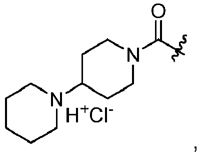 wherein **R⁵** is selected from the group consisting of hydrogen, methyl, and isopropyl and **R⁸** is hydrogen.

Additionally or alternatively, **R⁷** selected from the group consisting of 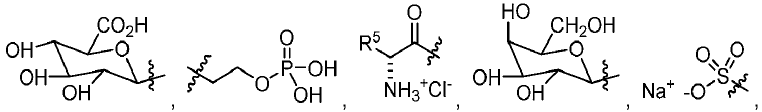 and 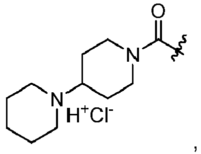 wherein **R⁵** is selected from the group consisting of hydrogen, methyl, and isopropyl and **R⁸** is hydrogen.

Additionally or alternatively, **R⁷** is 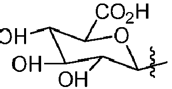 and **R⁸** is hydrogen.

Additionally or alternatively, **R⁷** is 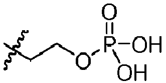 and **R⁸** is hydrogen.

Additionally or alternatively, **R⁷** is 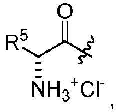 wherein **R⁵** is selected from the group consisting of hydrogen, methyl, and isopropyl and **R⁸** is hydrogen.

Additionally or alternatively, **R⁷** is 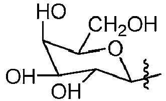 and **R⁸** is hydrogen.

Additionally or alternatively, **R⁷** is 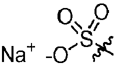 and **R⁸** is hydrogen.

Additionally or alternatively, **R⁷** is 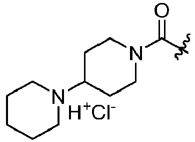 and **R⁸** is hydrogen.

In another embodiment, the present disclosure relates to a compound corresponding in structure to Formula (II) or a pharmaceutically acceptable salt thereof, wherein **R⁶** is independently selected from the group consisting of hydrogen, 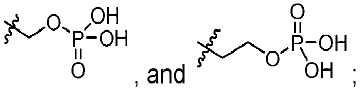 **R⁷** and **R⁸** are each hydrogen, and **R⁹** is independently selected from the group consisting of hydrogen, 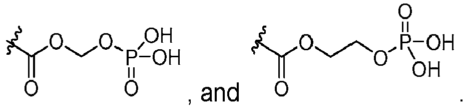 In one aspect, the compound corresponds in structure to Formula (II). In another aspect, the compound is a pharmaceutically acceptable salt of a compound corresponding in structure to Formula (II).

In another embodiment, the present disclosure relates to a compound corresponding in structure to Formula (II) or a pharmaceutically acceptable salt thereof, wherein **R⁶** is hydrogen; **R⁷** and **R⁸** are each independently selected from the group consisting of hydrogen, 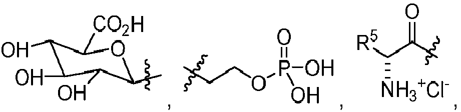 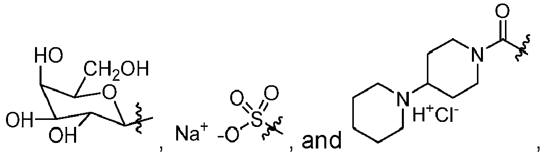 wherein **R⁵** is selected from the group consisting of hydrogen, methyl, and isopropyl; and **R⁹** is independently selected from the group consisting of hydrogen, 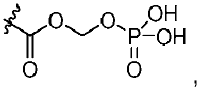 and 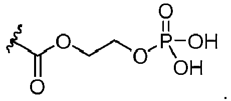 In one aspect, the compound corresponds in structure to Formula (II). In another aspect, the compound is a pharmaceutically acceptable salt of a compound corresponding in structure to Formula (II).

In another embodiment, the present disclosure relates to a compound corresponding in structure to Formula (II) or a pharmaceutically acceptable salt thereof, wherein **R⁶** is hydrogen; **R⁷** and **R⁸** are hydrogen; and **R⁹** is independently selected from the group consisting of hydrogen, 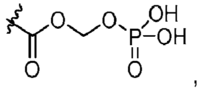 and 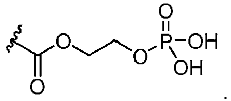 In one aspect, the compound corresponds in structure to Formula (II). In another aspect, the compound is a pharmaceutically acceptable salt of a compound corresponding in structure to Formula (II).

In another embodiment, the present disclosure relates to a compound corresponding in structure to Formula (II-a): 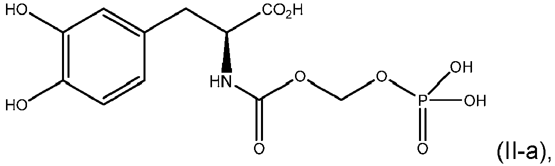 or a pharmaceutically acceptable salt thereof. In one aspect, the compound corresponds in structure to Formula (II-a). In another aspect, the compound is a pharmaceutically acceptable salt of a compound corresponding in structure to Formula (II-a).

In another embodiment, the present disclosure relates to a compound corresponding in structure to Formula (II-b): 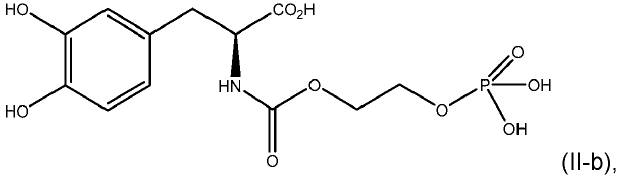 or a pharmaceutically acceptable salt thereof. In one aspect, the compound corresponds in structure to Formula (II-b). In another aspect, the compound is a pharmaceutically acceptable salt of a compound corresponding in structure to Formula (II-b).

In another embodiment, the present disclosure relates to a compound corresponding in structure to Formula (II) or a pharmaceutically acceptable salt thereof, wherein **R⁶** is hydrogen; **R⁷** and **R⁸** are each independently selected from the group consisting of hydrogen, 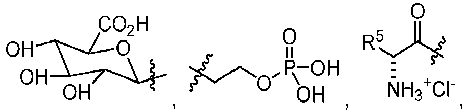 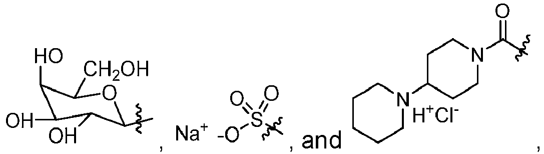 wherein **R⁵** is selected from the group consisting of hydrogen, methyl, and isopropyl; and **R⁹** is independently selected from the group consisting of hydrogen, 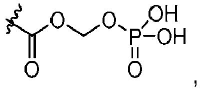 and 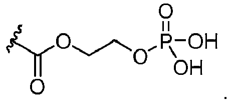 In one aspect, the compound corresponds in structure to Formula (II). In another aspect, the compound is a pharmaceutically acceptable salt of a compound corresponding in structure to Formula (II).

In another embodiment, the present disclosure relates to a compound corresponding in structure to Formula (II) or a pharmaceutically acceptable salt thereof, wherein **R⁶** is independently selected from the group consisting of hydrogen, 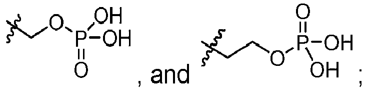 **R⁷** and **R⁸** are each independently selected from the group consisting of hydrogen, 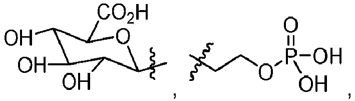 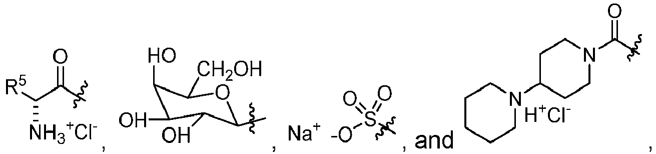 wherein **R⁵** is selected from the group consisting of hydrogen, methyl, and isopropyl; and **R⁹** is hydrogen. In one aspect, the compound corresponds in structure to Formula (II). In another aspect, the compound is a pharmaceutically acceptable salt of a compound corresponding in structure to Formula (II).

In another embodiment, the present disclosure relates to a compound corresponding in structure to Formula (II) or a pharmaceutically acceptable salt thereof, wherein **R⁶** is independently selected from the group consisting of hydrogen, 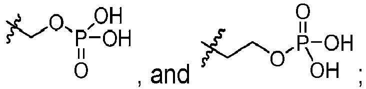 **R⁷** is selected from the group consisting of hydrogen, 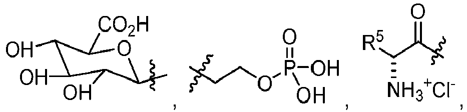 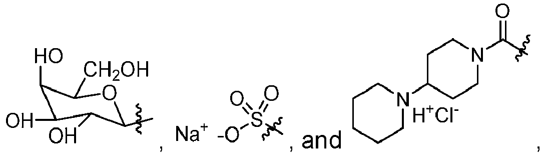 wherein **R⁵** is selected from the group consisting of hydrogen, methyl, and isopropyl; **R⁸** is hydrogen; and **R⁹** is independently selected from the group consisting of hydrogen, 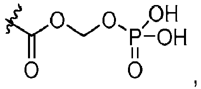 and 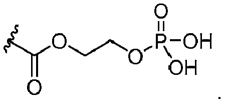 In one aspect, the compound corresponds in structure to Formula (II). In another aspect, the compound is a pharmaceutically acceptable salt of a compound corresponding in structure to Formula (II).

In another embodiment, the present disclosure relates to a compound corresponding in structure to Formula (II) or a pharmaceutically acceptable salt thereof, wherein **R⁶** is hydrogen; **R⁷** is selected from the group consisting of hydrogen, 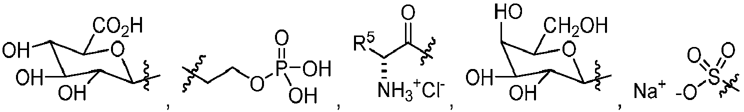 , and 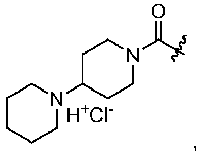 wherein **R⁵** is selected from the group consisting of hydrogen, methyl, and isopropyl; **R⁸** is hydrogen; and **R⁹** is hydrogen. In one aspect, the compound corresponds in structure to Formula (II). In another aspect, the compound is a pharmaceutically acceptable salt of a compound corresponding in structure to Formula (II).

In another embodiment, the present disclosure relates to a compound corresponding in structure to Formula (II) or a pharmaceutically acceptable salt thereof, wherein **R⁶** is hydrogen; **R⁷** and **R⁸** are each independently selected from the group consisting of hydrogen, 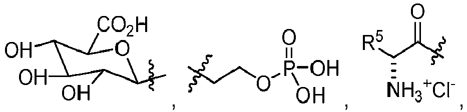 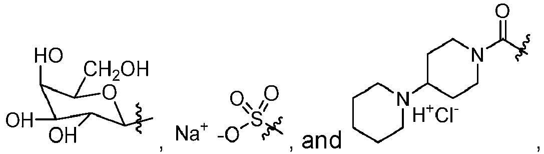 wherein **R⁵** is selected from the group consisting of hydrogen, methyl, and isopropyl; and **R⁹** is hydrogen. In one aspect, the compound corresponds in structure to Formula (II). In another aspect, the compound is a pharmaceutically acceptable salt of a compound corresponding in structure to Formula (II).

In another embodiment, the present disclosure relates to a compound corresponding in structure to Formula (II) or a pharmaceutically acceptable salt thereof, wherein **R⁶** is independently selected from the group consisting of hydrogen, 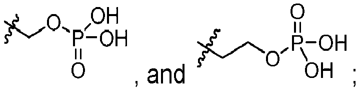 **R⁷** and **R⁸** are each independently selected from the group consisting of hydrogen, 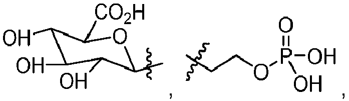 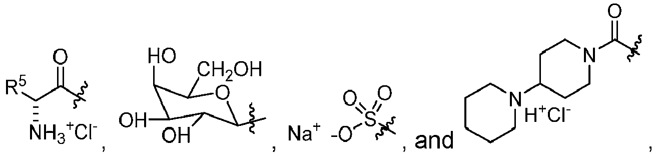 wherein **R⁵** is selected from the group consisting of hydrogen, methyl, and isopropyl; and **R⁹** is hydrogen. In one aspect, the compound corresponds in structure to Formula (II). In another aspect, the compound is a pharmaceutically acceptable salt of a compound corresponding in structure to Formula (II).

In another embodiment, the present disclosure relates to a compound corresponding in structure to Formula (II) or a pharmaceutically acceptable salt thereof, wherein **R⁶** is independently selected from the group consisting of hydrogen, 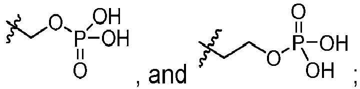 **R⁷** and **R⁸** are hydrogen; and **R⁹** is independently selected from the group consisting of hydrogen, 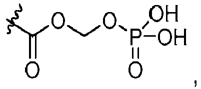 and 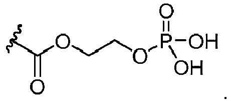 In one aspect, the compound corresponds in structure to Formula (II). In another aspect, the compound is a pharmaceutically acceptable salt of a compound corresponding in structure to Formula (II).

In another embodiment, the present disclosure relates to a compound corresponding in structure to Formula (II) or a pharmaceutically acceptable salt thereof, wherein **R⁶** is independently selected from the group consisting of hydrogen, 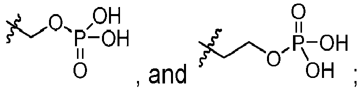 **R⁷** and **R⁸** are hydrogen; and **R⁹** is hydrogen. In one aspect, the compound corresponds in structure to Formula (II). In another aspect, the compound is a pharmaceutically acceptable salt of a compound corresponding in structure to Formula (II).

Various other compounds corresponding in structure to Formula (II) or pharmaceutically acceptable salts thereof are contemplated herein. Examples of suitable compounds corresponding in structure to Formula (I) are provided below in Table 2, which provides R⁶, R⁷, R⁸, **R⁹** and/or **R⁵** substituents for compounds corresponding in structure to Formula (II)

**Table 2**

| **Compound** | **R⁶** | **R⁷** | **R⁸** | **R⁹** | **R⁵** |
|---|---|---|---|---|---|
| (II-c) | 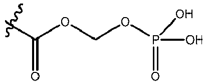 | hydrogen | hydrogen | hydrogen | |
| (II-d) | 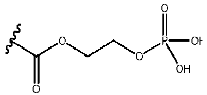 | hydrogen | hydrogen | hydrogen | |
| (II-e) | hydrogen | 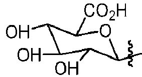 | hydrogen | hydrogen | |
| (II-f) | hydrogen | 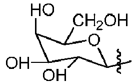 | hydrogen | hydrogen | |
| (II-g) | hydrogen | 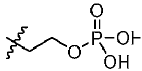 | hydrogen | hydrogen | |
| (II-h) | hydrogen | 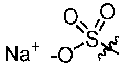 | hydrogen | hydrogen | |
| (II-i) | hydrogen | 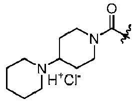 | hydrogen | hydrogen | |
| (II-j) | hydrogen | 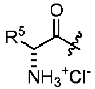 | hydrogen | hydrogen | hydrogen |
| (II-k) | hydrogen | 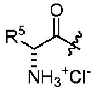 | hydrogen | hydrogen | methyl |
| (II-l) | hydrogen | 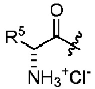 | hydrogen | hydrogen | isopropyl |
| (II-m) | hydrogen | 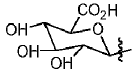 | 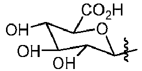 | hydrogen | |
| (II-n) | hydrogen | 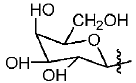 | 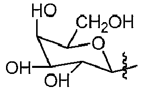 | hydrogen | |
| (II-o) | hydrogen | 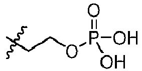 | 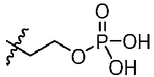 | hydrogen | |
| (II-p) | hydrogen | 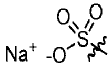 | 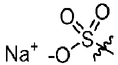 | hydrogen | |
| (II-q) | hydrogen | 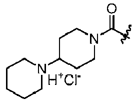 | 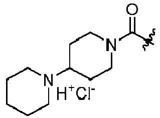 | hydrogen | |
| (II-r) | hydrogen | 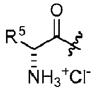 | 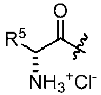 | hydrogen | hydrogen |
| (II-s) | hydrogen | 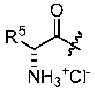 | 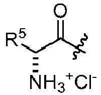 | hydrogen | methyl |
| (II-t) | hydrogen | 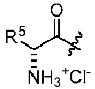 | 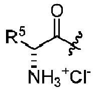 | hydrogen | isopropyl |
| (II-u) | hydrogen | hydrogen | 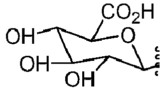 | hydrogen | |
| (II-v) | hydrogen | hydrogen | 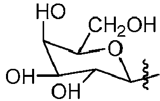 | hydrogen | |
| (II-w) | hydrogen | hydrogen | 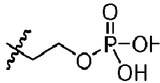 | hydrogen | |
| (II-x) | hydrogen | hydrogen | 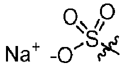 | hydrogen | |
| (II-y) | hydrogen | hydrogen | 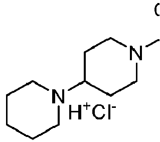 | hydrogen | |
| (II-z) | hydrogen | hydrogen | 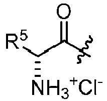 | hydrogen | |
| (II-aa) | hydrogen | hydrogen | 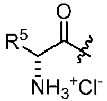 | hydrogen | |
| (II-ab) | hydrogen | hydrogen | 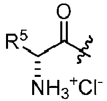 | hydrogen | |

In various embodiments, the present disclosure relates to a compound corresponding in structure to any one of Formula (II-c), (II-d), (II-e), (II-f), (II-g), (II-h), (II-i), (II-j), (II-k), (II-l), (II-m), (II-n), (II-o), (II-p), (II-q), (II-r), (II-s), (II-t), (II-u), (II-v), (II-x), (II-y), (II-z), (II-aa), or (II-ab) or a pharmaceutically acceptable salt thereof. In one aspect, the compound corresponds in structure to any one of Formula (II-c), (II-d), (II-e), (II-f), (II-g), (II-h), (II-i), (II-j), (II-k), (II-l), (II-m), (II-n), (II-o), (II-p), (II-q), (II-r), (II-s), (II-t), (II-u), (II-v), (II-x), (II-y), (II-z), (II-aa), or (II-ab). In another aspect, the compound is a pharmaceutically acceptable salt of a compound corresponding in structure to any one of Formula (II-c), (II-d), (II-e), (II-f), (II-g), (II-h), (II-i), (II-j), (II-k), (II-l), (II-m), (II-n), (II-o), (II-p), (II-q), (II-r), (II-s), (II-t), (II-u), (II-v), (II-x), (II-y), (II-z), (II-aa), or (II-ab).

### III. Pharmaceutical Combinations/Compositions

The present disclosure also relates to pharmaceutical combinations and compositions comprising a carbidopa prodrug and/or an L-dopa prodrug.

In some embodiments, the pharmaceutical compositions comprise a carbidopa prodrug. In other embodiments, the pharmaceutical compositions comprise an L-dopa prodrug. In still other embodiments, the pharmaceutical compositions comprise both a carbidopa prodrug and an L-dopa prodrug.

The carbidopa and L-dopa prodrugs disclosed herein (and their pharmaceutically acceptable salts) can be formulated in the same pharmaceutical composition or can be present in separate pharmaceutical compositions. For example, a pharmaceutical combination disclosed herein can comprise a carbidopa prodrug in a first pharmaceutical composition and an L-dopa prodrug in a separate, second pharmaceutical composition. Alternatively, the pharmaceutical combination can comprise a carbidopa prodrug and L-dopa prodrug in the same pharmaceutical composition.

### A. First Compound and Second Compound

In one embodiment, the pharmaceutical composition comprises a first compound corresponding in structure to Formula (I): 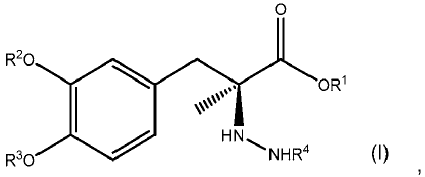 or a pharmaceutically acceptable salt thereof, wherein **R¹** is independently selected from the group consisting of hydrogen, 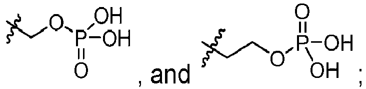 **R²** and **R³** are each independently selected from the group consisting of hydrogen, 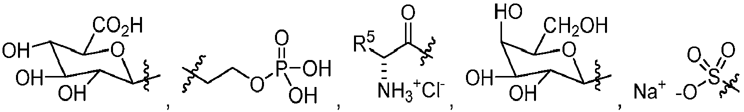 , and 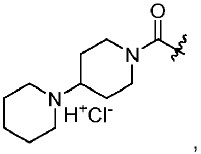 wherein **R⁵** is selected from the group consisting of hydrogen, methyl, and isopropyl; and **R⁴** is independently selected from the group consisting of hydrogen, 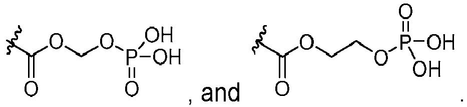 In one aspect, the composition comprises a first compound corresponding in structure to Formula (I). In another aspect, the composition comprises a pharmaceutically acceptable salt of the first compound corresponding in structure to Formula (I).

In another embodiment, the pharmaceutical composition comprises a first compound corresponding in structure to Formula (I-a) or a pharmaceutically acceptable salt thereof, and a pharmaceutically acceptable carrier. In one aspect, the composition comprises a first compound corresponding in structure to Formula (I-a). In another aspect, the composition comprises a pharmaceutically acceptable salt of the first compound corresponding in structure to Formula (I-a).

In another embodiment, the pharmaceutical composition comprises a first compound corresponding in structure to Formula (I-b) or a pharmaceutically acceptable salt thereof, and a pharmaceutically acceptable carrier. In one aspect, the composition comprises a first compound corresponding in structure to Formula (I-b). In another aspect, the composition comprises a pharmaceutically acceptable salt of the first compound corresponding in structure to Formula (I-b).

In another embodiment, the pharmaceutical composition comprises a first compound corresponding in structure to any one of Formula (I-c), (I-d), (I-e), (I-f), (I-g), (I-h), (I-i), (I-j), (I-k), (I-l), (I-m), (I-n), (I-o), (I-p), (I-q), (I-r), (I-s), (I-t), (I-u), (I-v), (I-w), (I-x), (I-y), (I-z), (I-aa), (I-ab), or (I-ac) or a pharmaceutically acceptable salt thereof, and a pharmaceutically acceptable carrier. In one aspect, the composition comprises a first compound corresponding in structure to any one of Formula (I-c), (I-d), (I-e), (I-f), (I-g), (I-h), (I-i), (I-j), (I-k), (I-l), (I-m), (I-n), (I-o), (I-p), (I-q), (I-r), (I-s), (I-t), (I-u), (I-v), (I-w), (I-x), (I-y), (I-z), (I-aa), (I-ab), or (I-ac). In another aspect, the composition comprises a pharmaceutically acceptable salt of the first compound corresponding in structure to any one of Formula (I-c), (I-d), (I-e), (I-f), (I-g), (I-h), (I-i), (I-j), (I-k), (I-l), (I-m), (I-n), (I-o), (I-p), (I-q), (I-r), (I-s), (I-t), (I-u), (I-v), (I-w), (I-x), (I-y), (I-z), (I-aa), (I-ab), or (I-ac).

In one embodiment, the pharmaceutical composition comprises a second compound corresponding in structure to Formula (II): 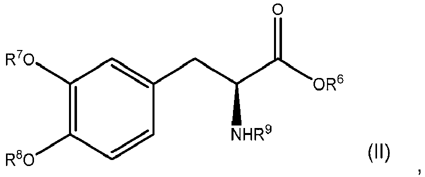 or a pharmaceutically acceptable salt thereof, wherein **R⁶** is independently selected from the group consisting of hydrogen, 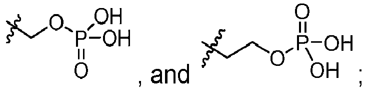 **R⁷** and **R⁸** are each independently selected from the group consisting of hydrogen, 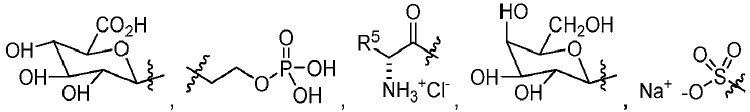 , and 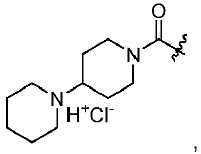 wherein **R⁵** is selected from the group consisting of hydrogen, methyl, and isopropyl; and **R⁹** is independently selected from the group consisting of hydrogen, 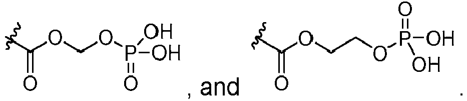 In one aspect, the composition comprises a second compound corresponding in structure to Formula (II). In another aspect, the composition comprises a pharmaceutically acceptable salt of the first compound corresponding in structure to Formula (II).

In another embodiment, the pharmaceutical composition comprises a second compound corresponding in structure to Formula (II-a) or a pharmaceutically acceptable salt thereof, and a pharmaceutically acceptable carrier. In one aspect, the composition comprises a second compound corresponding in structure to Formula (II-a). In another aspect, the composition comprises a pharmaceutically acceptable salt of the second compound corresponding in structure to Formula (II-a).

In another embodiment, the pharmaceutical composition comprises a second compound corresponding in structure to Formula (II-b) or a pharmaceutically acceptable salt thereof, and a pharmaceutically acceptable carrier. In one aspect, the composition comprises a second compound corresponding in structure to Formula (II-b). In another aspect, the composition comprises a pharmaceutically acceptable salt of the second compound corresponding in structure to Formula (II-b).

In another embodiment, the pharmaceutical composition comprises a second compound corresponding in structure to any one of Formula (II-c), (II-d), (II-e), (II-f), (II-g), (II-h), (II-i), (II-j), (II-k), (II-l), (II-m), (II-n), (II-o), (II-p), (II-q), (II-r), (II-s), (II-t), (II-u), (II-v), (II-x), (II-y), (II-z), (II-aa), or (II-ab) or a pharmaceutically acceptable salt thereof, and a pharmaceutically acceptable carrier. In one aspect, the composition comprises a second compound corresponding in structure to any one of Formula ((Il-c), (II-d), (II-e), (II-f), (II-g), (II-h), (II-i), (II-j), (II-k), (II-l), (II-m), (II-n), (II-o), (II-p), (II-q), (II-r), (II-s), (II-t), (II-u), (II-v), (II-x), (II-y), (II-z), (II-aa), or (II-ab). In another aspect, the composition comprises a pharmaceutically acceptable salt of the second compound corresponding in structure to any one of Formula (II-c), (II-d), (II-e), (II-f), (II-g), (II-h), (II-i), (II-j), (II-k), (II-l), (II-m), (II-n), (II-o), (II-p), (II-q), (II-r), (II-s), (II-t), (II-u), (II-v), (II-x), (II-y), (II-z), (II-aa), or (II-ab).

In another embodiment, the pharmaceutical composition comprises a first compound, a second compound, and a pharmaceutically acceptable carrier, wherein:
the first compound corresponds in structure to Formula (I): 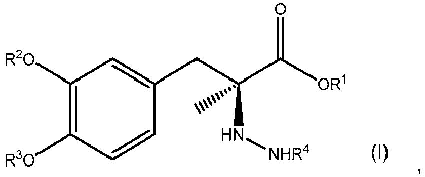 or a pharmaceutically acceptable salt thereof, wherein **R¹** is independently selected from the group consisting of hydrogen, 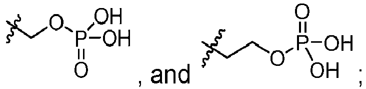 **R²** and **R³** are each independently selected from the group consisting of hydrogen, 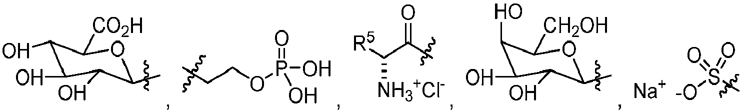 , and 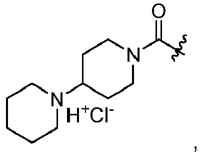 wherein **R⁵** is selected from the group consisting of hydrogen, methyl, and isopropyl; and **R⁴** is independently selected from the group consisting of hydrogen, 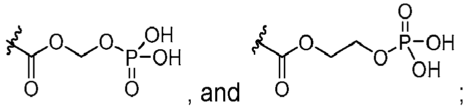 and
the second compound corresponds in structure to Formula (II): 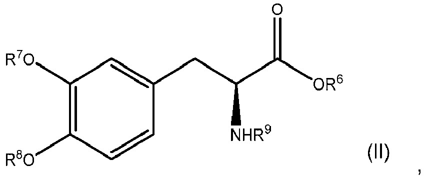 or a pharmaceutically acceptable salt thereof, wherein **R⁶** is independently selected from the group consisting of hydrogen, 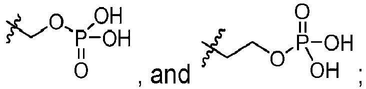 **R⁷** and **R⁸** are each independently selected from the group consisting of hydrogen, 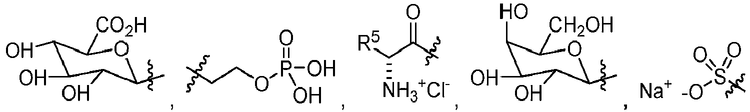 , and 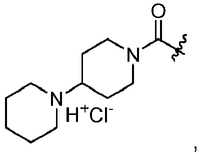 wherein **R⁵** is selected from the group consisting of hydrogen, methyl, and isopropyl; and **R⁹** is independently selected from the group consisting of hydrogen,

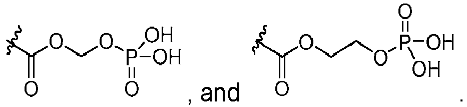

The composition may independently comprise the first compound and the second compound as either the free form of the compound or a pharmaceutically acceptable salt of the compound. In one aspect, the composition comprises the free form of the first compound. In another aspect, the composition comprises a pharmaceutically acceptable salt of the first compound. In another aspect, the composition comprises the free form of the second compound. In another aspect, the composition comprises a pharmaceutically acceptable salt of the second compound. In another aspect, the composition comprises the free form of the first compound and the free form of the second compound. In another aspect, the composition comprises a pharmaceutically acceptable salt of the first compound and a pharmaceutically acceptable salt of the second compound.

In another embodiment, the pharmaceutical composition comprises a first compound, a second compound, and a pharmaceutically acceptable carrier, wherein:
the first compound corresponds in structure to Formula (I-a) or a pharmaceutically acceptable salt thereof; and
the second compound corresponds in structure to Formula (II): 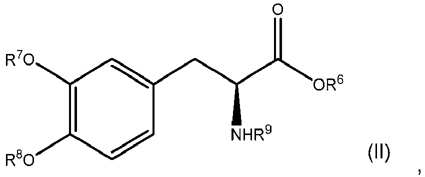 or a pharmaceutically acceptable salt thereof, wherein **R⁶** is independently selected from the group consisting of hydrogen, 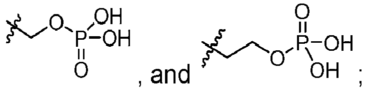 **R⁷** and **R⁸** are each independently selected from the group consisting of hydrogen, 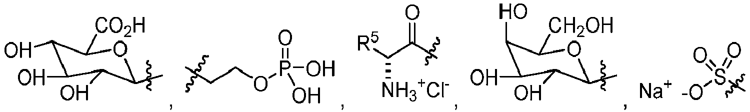 , and 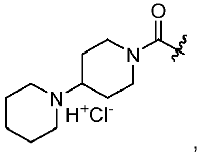 wherein **R⁵** is selected from the group consisting of hydrogen, methyl, and isopropyl; and **R⁹** is independently selected from the group consisting of hydrogen,

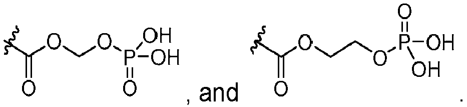

In another embodiment, the pharmaceutical composition comprises a first compound, a second compound, and a pharmaceutically acceptable carrier, wherein:
the first compound corresponds in structure to Formula (I-b) or a pharmaceutically acceptable salt thereof; and
the second compound corresponds in structure to Formula (II): 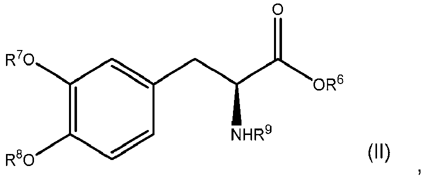 or a pharmaceutically acceptable salt thereof, wherein **R⁶** is independently selected from the group consisting of hydrogen, 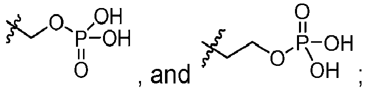 **R⁷** and **R⁸** are each independently selected from the group consisting of hydrogen, 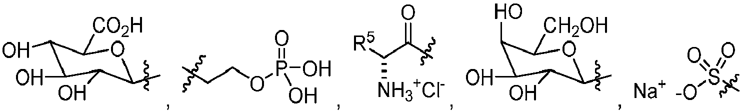 , and 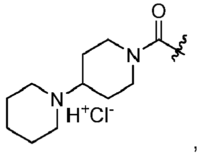 wherein **R⁵** is selected from the group consisting of hydrogen, methyl, and isopropyl; and **R⁹** is independently selected from the group consisting of hydrogen,

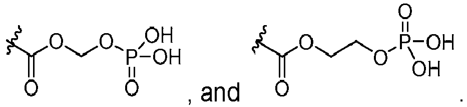

In another embodiment, the pharmaceutical composition comprises a first compound, a second compound, and a pharmaceutically acceptable carrier, wherein:
the first compound corresponds in structure to any one of Formula I-c), (I-d), (I-e), (I-f), (I-g), (I-h), (I-i), (I-j), (I-k), (I-l), (I-m), (I-n), (I-o), (I-p), (I-q), (I-r), (I-s), (I-t), (I-u), (I-v), (I-w), (I-x), (I-y), (I-z), (I-aa), (I-ab), or (I-ac) or a pharmaceutically acceptable salt thereof; and
the second compound corresponds in structure to Formula (II): 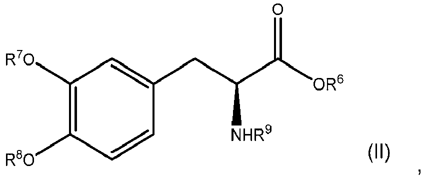 or a pharmaceutically acceptable salt thereof, wherein **R⁶** is independently selected from the group consisting of hydrogen, 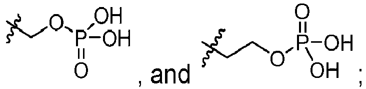 **R⁷** and **R⁸** are each independently selected from the group consisting of hydrogen, 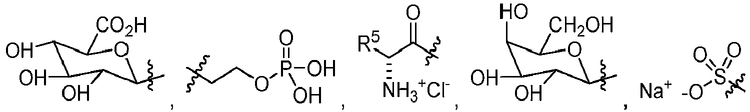 , and 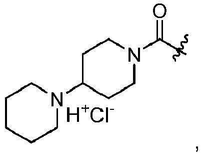 wherein **R⁵** is selected from the group consisting of hydrogen, methyl, and isopropyl; and **R⁹** is independently selected from the group consisting of hydrogen,

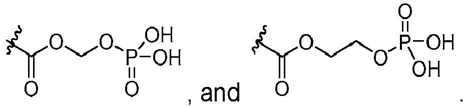

In another embodiment, the pharmaceutical composition comprises a first compound, a second compound, and a pharmaceutically acceptable carrier, wherein:
the first compound corresponds in structure to Formula (I): 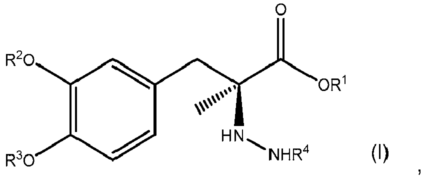 or a pharmaceutically acceptable salt thereof, wherein **R¹** is independently selected from the group consisting of hydrogen, 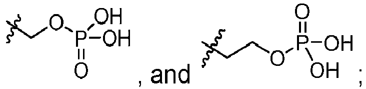 **R²** and **R³** are each independently selected from the group consisting of hydrogen, 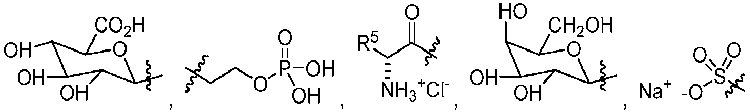 , and 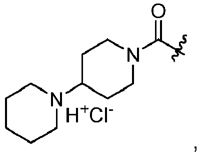 wherein **R⁵** is selected from the group consisting of hydrogen, methyl, and isopropyl; and **R⁴** is independently selected from the group consisting of hydrogen, 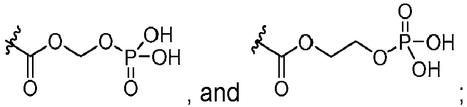 and
the second compound corresponds in structure to Formula (II-a) or a pharmaceutically acceptable salt thereof.

In another embodiment, the pharmaceutical composition comprises a first compound, a second compound, and a pharmaceutically acceptable carrier, wherein:
the first compound corresponds in structure to Formula (I): 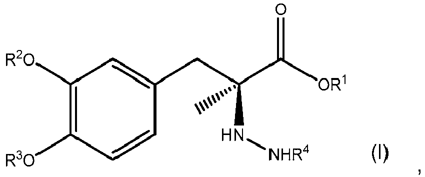 or a pharmaceutically acceptable salt thereof, wherein **R¹** is independently selected from the group consisting of hydrogen, 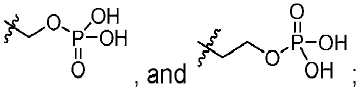 **R²** and **R³** are each independently selected from the group consisting of hydrogen, 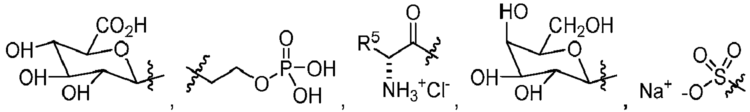 , and 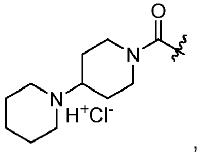 wherein **R⁵** is selected from the group consisting of hydrogen, methyl, and isopropyl; and **R⁴** is independently selected from the group consisting of hydrogen, 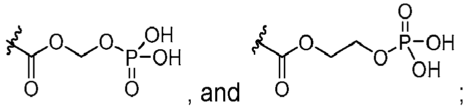 and
the second compound corresponds in structure to Formula (II-b) or a pharmaceutically acceptable salt thereof.

In another embodiment, the pharmaceutical composition comprises a first compound, a second compound, and a pharmaceutically acceptable carrier, wherein:
the first compound corresponds in structure to Formula (I): 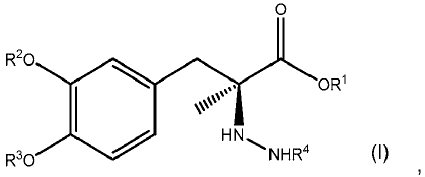 or a pharmaceutically acceptable salt thereof, wherein **R¹** is independently selected from the group consisting of hydrogen, 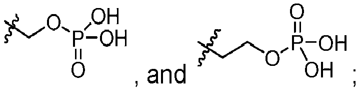 **R²** and **R³** are each independently selected from the group consisting of hydrogen, 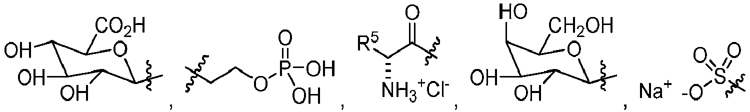 , and 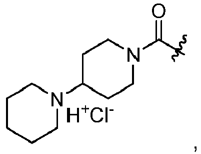 wherein **R⁵** is selected from the group consisting of hydrogen, methyl, and isopropyl; and **R⁴** is independently selected from the group consisting of hydrogen, 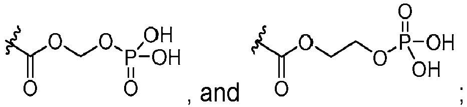 and
the second compound corresponds in structure to Formula (II-c), (II-d), (II-e), (II-f), (II-g), (II-h), (II-i), (II-j), (II-k), (II-l), (II-m), (II-n), (II-o), (II-p), (II-q), (II-r), (II-s), or (II-t) or a pharmaceutically acceptable salt thereof.

In another embodiment, the pharmaceutical composition comprises a first compound, a second compound, and a pharmaceutically acceptable carrier, wherein:
the first compound corresponds in structure to Formula (I-a):or a pharmaceutically acceptable salt thereof; and
the second compound corresponds in structure to Formula (II-a) or a pharmaceutically acceptable salt thereof.

In another embodiment, the pharmaceutical composition comprises a first compound, a second compound, and a pharmaceutically acceptable carrier, wherein:
the first compound corresponds in structure to Formula (I-b) or a pharmaceutically acceptable salt thereof; and
the second compound corresponds in structure to Formula (II-a) or a pharmaceutically acceptable salt thereof.

In another embodiment, the pharmaceutical composition comprises a first compound, a second compound, and a pharmaceutically acceptable carrier, wherein:
the first compound corresponds in structure to Formula (I-c) or a pharmaceutically acceptable salt thereof; and
the second compound corresponds in structure to Formula (II-a) or a pharmaceutically acceptable salt thereof.

In another embodiment, the pharmaceutical composition comprises a first compound, a second compound, and a pharmaceutically acceptable carrier, wherein:
the first compound corresponds in structure to Formula (I-d) or a pharmaceutically acceptable salt thereof; and
the second compound corresponds in structure to Formula (II-a) or a pharmaceutically acceptable salt thereof.

In another embodiment, the pharmaceutical composition comprises a first compound, a second compound, and a pharmaceutically acceptable carrier, wherein:
the first compound corresponds in structure to Formula (I-e) or a pharmaceutically acceptable salt thereof; and
the second compound corresponds in structure to Formula (II-a) or a pharmaceutically acceptable salt thereof.

In another embodiment, the pharmaceutical composition comprises a first compound, a second compound, and a pharmaceutically acceptable carrier, wherein:
the first compound corresponds in structure to Formula or a pharmaceutically acceptable salt thereof; and
the second compound corresponds in structure to Formula (II-a) or a pharmaceutically acceptable salt thereof.

In another embodiment, the pharmaceutical composition comprises a first compound, a second compound, and a pharmaceutically acceptable carrier, wherein:
the first compound corresponds in structure to Formula (I-a) or a pharmaceutically acceptable salt thereof; and
the second compound corresponds in structure to Formula (II-b) or a pharmaceutically acceptable salt thereof.

In another embodiment, the pharmaceutical composition comprises a first compound, a second compound, and a pharmaceutically acceptable carrier, wherein:
the first compound corresponds in structure to Formula (I-b) or a pharmaceutically acceptable salt thereof; and
the second compound corresponds in structure to Formula (II-b) or a pharmaceutically acceptable salt thereof.

In another embodiment, the pharmaceutical composition comprises a first compound, a second compound, and a pharmaceutically acceptable carrier, wherein:
the first compound corresponds in structure to Formula (I-c) or a pharmaceutically acceptable salt thereof; and
the second compound corresponds in structure to Formula (II-b) or a pharmaceutically acceptable salt thereof.

In another embodiment, the pharmaceutical composition comprises a first compound, a second compound, and a pharmaceutically acceptable carrier, wherein:
the first compound corresponds in structure to Formula (I-d) or a pharmaceutically acceptable salt thereof; and
the second compound corresponds in structure to Formula (II-b) or a pharmaceutically acceptable salt thereof.

In another embodiment, the pharmaceutical composition comprises a first compound, a second compound, and a pharmaceutically acceptable carrier, wherein:
the first compound corresponds in structure to Formula (I-e) or a pharmaceutically acceptable salt thereof; and
the second compound corresponds in structure to Formula (II-b) or a pharmaceutically acceptable salt thereof.

In another embodiment, the pharmaceutical composition comprises a first compound, a second compound, and a pharmaceutically acceptable carrier, wherein:
the first compound corresponds in structure to Formula or a pharmaceutically acceptable salt thereof; and
the second compound corresponds in structure to Formula (II-b) or a pharmaceutically acceptable salt thereof.

In another embodiment, the pharmaceutical composition comprises a first compound, a second compound, and a pharmaceutically acceptable carrier, wherein:
the first compound corresponds in structure to Formula (I-a) or a pharmaceutically acceptable salt thereof; and
the second compound corresponds in structure to Formula (II-c) or a pharmaceutically acceptable salt thereof.

In another embodiment, the pharmaceutical composition comprises a first compound, a second compound, and a pharmaceutically acceptable carrier, wherein:
the first compound corresponds in structure to Formula (I-b) or a pharmaceutically acceptable salt thereof; and
the second compound corresponds in structure to Formula (II-c) or a pharmaceutically acceptable salt thereof.

In another embodiment, the pharmaceutical composition comprises a first compound, a second compound, and a pharmaceutically acceptable carrier, wherein:
the first compound corresponds in structure to Formula (I-c) or a pharmaceutically acceptable salt thereof; and
the second compound corresponds in structure to Formula (II-c) or a pharmaceutically acceptable salt thereof.

In another embodiment, the pharmaceutical composition comprises a first compound, a second compound, and a pharmaceutically acceptable carrier, wherein:
the first compound corresponds in structure to Formula (I-d) or a pharmaceutically acceptable salt thereof; and
the second compound corresponds in structure to Formula (II-c) or a pharmaceutically acceptable salt thereof.

In another embodiment, the pharmaceutical composition comprises a first compound, a second compound, and a pharmaceutically acceptable carrier, wherein:
the first compound corresponds in structure to Formula (I-e) or a pharmaceutically acceptable salt thereof; and
the second compound corresponds in structure to Formula (II-c) or a pharmaceutically acceptable salt thereof.

In another embodiment, the pharmaceutical composition comprises a first compound, a second compound, and a pharmaceutically acceptable carrier, wherein:
the first compound corresponds in structure to Formula or a pharmaceutically acceptable salt thereof; and
the second compound corresponds in structure to Formula (II-c) or a pharmaceutically acceptable salt thereof.

In another embodiment, the pharmaceutical composition comprises a first compound, a second compound, and a pharmaceutically acceptable carrier, wherein:
the first compound corresponds in structure to Formula (I-a) or a pharmaceutically acceptable salt thereof; and
the second compound corresponds in structure to Formula (II-d) or a pharmaceutically acceptable salt thereof.

In another embodiment, the pharmaceutical composition comprises a first compound, a second compound, and a pharmaceutically acceptable carrier, wherein:
the first compound corresponds in structure to Formula (I-b) or a pharmaceutically acceptable salt thereof; and
the second compound corresponds in structure to Formula (II-d) or a pharmaceutically acceptable salt thereof.

In another embodiment, the pharmaceutical composition comprises a first compound, a second compound, and a pharmaceutically acceptable carrier, wherein:
the first compound corresponds in structure to Formula (I-c) or a pharmaceutically acceptable salt thereof; and
the second compound corresponds in structure to Formula (II-d) or a pharmaceutically acceptable salt thereof.

In another embodiment, the pharmaceutical composition comprises a first compound, a second compound, and a pharmaceutically acceptable carrier, wherein:
the first compound corresponds in structure to Formula (I-d) or a pharmaceutically acceptable salt thereof; and
the second compound corresponds in structure to Formula (II-d) or a pharmaceutically acceptable salt thereof.

In another embodiment, the pharmaceutical composition comprises a first compound, a second compound, and a pharmaceutically acceptable carrier, wherein:
the first compound corresponds in structure to Formula (I-e) or a pharmaceutically acceptable salt thereof; and
the second compound corresponds in structure to Formula (II-d) or a pharmaceutically acceptable salt thereof.

In another embodiment, the pharmaceutical composition comprises a first compound, a second compound, and a pharmaceutically acceptable carrier, wherein:
the first compound corresponds in structure to Formula or a pharmaceutically acceptable salt thereof; and
the second compound corresponds in structure to Formula (II-d) or a pharmaceutically acceptable salt thereof.

In another embodiment, the pharmaceutical composition comprises a first compound, a second compound, and a pharmaceutically acceptable carrier, wherein:
the first compound corresponds in structure to Formula (I-a) or a pharmaceutically acceptable salt thereof; and
the second compound corresponds in structure to Formula (II-e) or a pharmaceutically acceptable salt thereof.

In another embodiment, the pharmaceutical composition comprises a first compound, a second compound, and a pharmaceutically acceptable carrier, wherein:
the first compound corresponds in structure to Formula (I-b) or a pharmaceutically acceptable salt thereof; and
the second compound corresponds in structure to Formula (II-e) or a pharmaceutically acceptable salt thereof.

In another embodiment, the pharmaceutical composition comprises a first compound, a second compound, and a pharmaceutically acceptable carrier, wherein:
the first compound corresponds in structure to Formula (I-c) or a pharmaceutically acceptable salt thereof; and
the second compound corresponds in structure to Formula (II-e) or a pharmaceutically acceptable salt thereof.

In another embodiment, the pharmaceutical composition comprises a first compound, a second compound, and a pharmaceutically acceptable carrier, wherein:
the first compound corresponds in structure to Formula (I-d) or a pharmaceutically acceptable salt thereof; and
the second compound corresponds in structure to Formula (II-e) or a pharmaceutically acceptable salt thereof.

In another embodiment, the pharmaceutical composition comprises a first compound, a second compound, and a pharmaceutically acceptable carrier, wherein:
the first compound corresponds in structure to Formula (I-e) or a pharmaceutically acceptable salt thereof; and
the second compound corresponds in structure to Formula (II-e) or a pharmaceutically acceptable salt thereof.

In another embodiment, the pharmaceutical composition comprises a first compound, a second compound, and a pharmaceutically acceptable carrier, wherein:
the first compound corresponds in structure to Formula or a pharmaceutically acceptable salt thereof; and
the second compound corresponds in structure to Formula (II-e) or a pharmaceutically acceptable salt thereof.

The pharmaceutical compositions of the present disclosure comprising both the first compound and the second compound generally will comprise the first compound and the second compound at a weight ratio from about 1:1 to about 1:50. In one aspect, the weight ratio is from about 1:2 to about 1:15. In another aspect, the weight ratio is from about 1:4 to about 1:10. In another aspect, the weight ratio is about 1:4. In another aspect, the weight ratio is about 1:7.5. In another aspect, the weight ratio is about 1:10.

### B. Additional Excipients

The pharmaceutical compositions of the present disclosure optionally comprise one or more additional pharmaceutically acceptable excipients. The term "excipient" refers to any substance, not itself a therapeutic agent, used as a carrier or vehicle for delivery of a therapeutic agent to a subject or added to a pharmaceutical composition to improve its handling or storage properties or to permit or facilitate formation of a unit dose of the composition.

Excipients include, for example, antioxidants, agents to adjust the pH and osmolarity, preservatives, thickening agents, colorants, buffering agents, bacteriostats, and stabilizers. A given excipient, if present, generally will be present in an amount of about 0.001% to about 95%, about 0.01% to about 80%, about 0.02% to about 25%, or about 0.3% to about 10%, by weight.

In one embodiment, the pharmaceutical compositions optionally comprise an antioxidant. Suitable antioxidants for use in the pharmaceutical compositions include, for example, butylated hydroxytoluene, butylated hydroxyanisole, potassium metabisulfite, and the like.

In one embodiment, the pharmaceutical compositions optionally comprise a buffering agent. Buffering agents include agents that reduce pH changes. Suitable classes of buffering agents for use in various embodiments of the present invention comprise a salt of a Group IA metal including, for example, a bicarbonate salt of a Group IA metal, a carbonate salt of a Group IA metal, an alkaline or alkali earth metal buffering agent, an aluminum buffering agent, a calcium buffering agent, a sodium buffering agent, or a magnesium buffering agent. Suitable buffering agents further include carbonates, phosphates, bicarbonates, citrates, borates, acetates, phthalates, tartrates, succinates of any of the foregoing, for example, sodium or potassium phosphate, citrate, borate, acetate, bicarbonate and carbonate.

### C. Dosage Forms

### Solid Composition

In one embodiment, the pharmaceutical composition is a solid composition.

In another embodiment, the pharmaceutical composition is a solid composition that is suitable for oral administration. The first and second compound may be present as independent, separate solid dosage forms or combined in the same solid dosage form. Suitable solid dosage forms include capsules, tablets, pills, powders and granules. In such solid dosage forms, the first and/or second compound may be mixed with at least one inert, pharmaceutically acceptable excipient or carrier, such as sodium citrate or dicalcium phosphate and/or a) fillers or extenders such as starches, lactose, sucrose, glucose, mannitol and silicic acid; b) binders such as carboxymethylcellulose, alginates, gelatin, polyvinylpyrrolidone, sucrose and acacia; c) humectants such as glycerol; d) disintegrating agents such as agar-agar, calcium carbonate, potato or tapioca starch, alginic acid, certain silicates and sodium carbonate; e) solution retarding agents such as paraffin; f) absorption accelerators such as quaternary ammonium compounds; g) wetting agents such as cetyl alcohol and glycerol monostearate; h) absorbents such as kaolin and bentonite clay and i) lubricants such as talc, calcium stearate, magnesium stearate, solid polyethylene glycols, sodium lauryl sulfate and mixtures thereof. In the case of capsules, tablets and pills, the dosage form may also comprise buffering agents.

Solid compositions of a similar type may also be employed as fillers in soft and hard-filled gelatin capsules using such carriers as lactose or milk sugar as well as high molecular weight polyethylene glycols and the like.

The solid dosage forms of tablets, dragees, capsules, pills and granules can be prepared with coatings and shells such as enteric coatings and other coatings well-known in the pharmaceutical formulating art. They may optionally contain opacifying agents and may also be of a composition such that they release the active ingredient(s) only, or preferentially, in a certain part of the intestinal tract, optionally, in a delayed manner. Examples of embedding compositions which can be used include polymeric substances and waxes.

The first and/or second compounds can also be in micro-encapsulated form (separately or together), if appropriate, with one or more of the above-mentioned carriers.

### Liquid Composition

In one embodiment, the pharmaceutical composition is a liquid composition. In one aspect, the composition comprises water and is suitable for infusion.

In another embodiment, the pharmaceutical composition is a liquid composition that is suitable for intragastric, intestinal (*e.g.,* intraduodenum, intrajejunum), intranasal, subcutaneous, intramuscular or intravenous administration. In one aspect, the composition is suitable for intragastric administration. In another aspect, the composition is suitable for subcutaneous administration. In another aspect, the composition is suitable for intramuscular administration. In another aspect, the composition is suitable for intravenous administration. In another aspect, the composition is suitable for intestinal administration. In another aspect, the composition is suitable for intraduodenum administration. In another aspect, the composition is suitable for intrajejunum administration. In another aspect, the composition is suitable for intranasal administration.

In another embodiment, the pharmaceutical composition is an aqueous pharmaceutical composition having an L-dopa prodrug concentration of at least about 5 mg/mL. In one aspect, the L-dopa prodrug concentration is at least about 10 mg/mL. In another aspect, the L-dopa prodrug concentration is at least about 20 mg/mL. In another aspect, the L-dopa prodrug concentration is at least about 30 mg/mL. In another aspect, the L-dopa prodrug concentration is at least about 50 mg/mL. In another aspect, the L-dopa prodrug concentration is at least about 100 mg/mL. In another aspect, the L-dopa prodrug concentration is at least about 150 mg/ mL. In another aspect, the L-dopa prodrug concentration is at least about 200 mg/mL. In another aspect, the L-dopa prodrug concentration is at least about 250 mg/mL. In another aspect, the L-dopa prodrug concentration is at least about 300 mg/mL. In another aspect, the L-dopa prodrug concentration is at least about 350 mg/mL. In another aspect, the L-dopa prodrug concentration is at least about 400 mg/mL. In particular, the above L-dopa prodrug concentrations may be L-dopa phosphate prodrug concentrations, more particularly a compound corresponding in structure to Formula (II-a) concentrations and/or a compound corresponding in structure to Formula (II-b) concentrations.

In another embodiment, the pharmaceutical composition is an aqueous pharmaceutical composition having a carbidopa prodrug concentration of at least about 5 mg/mL. In one aspect, the carbidopa prodrug concentration is at least about 10 mg/mL. In another aspect, the carbidopa prodrug concentration is at least about 20 mg/mL. In another aspect, the carbidopa prodrug concentration is at least about 30 mg/mL. In another aspect, the carbidopa prodrug concentration is at least about 50 mg/mL. In another aspect, the carbidopa prodrug concentration is at least about 100 mg/mL. In another aspect, the carbidopa prodrug concentration is at least about 150 mg/ mL. In another aspect, the carbidopa prodrug concentration is at least about 200 mg/mL. In particular, the above carbidopa prodrug concentrations may be carbidopa phosphate prodrug concentrations, more particularly a compound corresponding in structure to Formula (I-a) concentrations and/or a compound corresponding in structure to Formula (I-b) concentrations.

### D. pH Level

In one embodiment, the pharmaceutical compositions may have a pH of ≥ ~2.0, ≥ ~2.5, ≥ ~3.0, ≥ ~3.5, ≥ ~4.0, ≥ ~4.5, ≥ ~5.0, ≥ ~5.5, ≥ ~6.0, ≥ ~6.2, ≥ ~6.4, ≥ ~6.5, ≥ ~6.6, ≥ ~6.8, ≥ ~7.0, ≥ ~7.1, ≥ ~7.2, ≥ ~7.3, ≥ ~7.4, ≥ ~7.5, ≥ ~7.6, ≥ ~7.7, ≥ ~7.8, ≥ ~7.9, ≥ ~8.0, ≥ ~8.2, ≥ ~8.4, ≥ ~8.6, ≥ ~8.8, or ≥ ~9.0. Particularly, the pH is ≥ ~7.4. Ranges expressly disclosed include combinations of any of the above-enumerated values, *e.g.,* ~2.0 to ~7.5, ~6.0 to ~9.0, ~6.4 to ~7.7, ~7.0 to ~7.9, ~7.3 to ~8.2, *etc.* In one aspect the pH is from about 2 to about 8. In one aspect, the pH is from about 2.0 to about 7.5. In another aspect, the pH is from about 3.0 to about 7.5. In another aspect, the pH is from about 4.0 to about 7.5. In another aspect, the pH is from about 5.0 to about 7.5. In another aspect, the pH is from about 6.0 to about 7.5.

### E. Stability

In another embodiment, the first compound (*e.g.,* the phosphate prodrugs) and the second compound (*e.g.,* the phosphate prodrugs) in the pharmaceutical compositions advantageously may remain stable in liquid compositions (*e.g,* aqueous solutions) at the above-described pHs for ≥ ~24 hours, ≥ ~36 hours, ≥ ~48 hours, ≥ ~60 hours, ≥ ~72 hours, ≥ ~84 hours, ≥ ~96 hours, ≥ ~108 hours, ≥ ~120 hours, ≥ ~132 hours, ≥ ~136 hours, ≥ ~144 hours, ≥ -156 hours, ≥ ~168 hours, or ≥ ~180 hours. Particularly, the pharmaceutical compositions may remain stable in aqueous solutions for ≥ ~24 hours at a pH of ~6 to ~8. Ranges expressly disclosed include combinations of any of the above-enumerated values, e.g., -24 hours to -180 hours, ~24 hours to -168 hours, ~36 hours to -72 hours, etc. Such increased stability is important for liquid compositions of the pharmaceutical compositions because typically the liquid compositions are stored prior to administration (e.g., intragastric, subcutaneous, intrajejunum, intranasal, intramuscular and/or intravenous), and thus, the first compound and the second compound must remain stable and not degrade significantly during the course of storage.

### F. Solubility

In another embodiment, the first compound (*e.g.,* the phosphate prodrugs) and the second compound (*e.g.,* the phosphate prodrugs) in the pharmaceutical compositions unexpectedly have increased solubility in liquid compositions (*e.g,* aqueous solutions). For example, the first compound and/or the second compound may have a solubility at a pH of about ~5 to -8, or more particularly at about a neutral pH of about 6.9 to about 7.5, of ≥ ~90 mg/mL, ≥ ~100 mg/mL, ≥ -110 mg/mL, ≥ -120 mg/mL, ≥ ~130 mg/mL, ≥ -140 mg/mL, ≥ -150 mg/mL, ≥ ~160 mg/mL, ≥ ~170 mg/mL, ≥ ~180 mg/mL, ≥ ~190 mg/mL, ≥ ~200 mg/mL, ≥ ~210 mg/mL, ≥ ~220 mg/mL, ≥ ~230 mg/mL, ≥ ~240 mg/mL, ≥ -250 mg/mL, ≥ ~260 mg/mL, ≥ -270 mg/mL, ≥ ~280 mg/mL, ≥ ~290 mg/mL, ≥ ~300 mg/mL, ≥ ~310 mg/mL, ≥ ~320 mg/mL, ≥ -330 mg/mL, ≥ ~340 mg/mL, ≥ -350 mg/mL, ≥ ~360 mg/mL, ≥ ~370 mg/mL, ≥ ~380 mg/mL, ≥ ~390 mg/mL, ≥ -400 mg/mL, ≥ ~410 mg/mL, ≥ ~420 mg/mL, ≥ ~430 mg/mL, ≥ ~440 mg/mL, ≥ -450 mg/mL, ≥ ~460 mg/mL, ≥ ~470 mg/mL, ≥ ~480 mg/mL, ≥ ~490 mg/mL, or ≥ -500 mg/mL. Ranges expressly disclosed include combinations of any of the above-enumerated values, e.g., ~90 mg/mL to -500 mg/mL, -100 mg/mL to -300 mg/mL, ~200 mg/mL to -500 mg/mL, *etc.* In particular, the first compound has a solubility at a neutral pH, for example of about 7.4, of ≥ ~160 mg/mL, particularly ≥ -200 mg/mL. In particular, the second compound has a solubility at a neutral pH, for example of about 7.4, of ≥ ~370 mg/mL, particularly ≥ ~400 mg/mL. This increased solubility allows for higher concentrations of the first compound and/or second compound in the pharmaceutical composition, which leads to more effective and higher systemic levels of the first compound and/or second compound once administered to a patient.

### G. Ready-to-Use

In still other embodiments, the present disclosure relates to a ready-to-use vial or cartridge or container or enclosure suitable for liquid pharmaceutical dosage formulation containment. Such containment may serve the function of holding a liquid formulation containing one or more carbidopa prodrugs and/or one or more L-dopa prodrugs. The vials can also serve as storage for powder forms of the carbidopa prodrug(s) and/or L-dopa prodrug(s) such that the vial can be in a ready to use format wherein reconstitution with an aqueous vehicle results in a ready to withdraw or load injection to the patient.

### H. Pharmaceutical Combinations

As mentioned above, a pharmaceutical combination comprising the first compound and the second compound is also disclosed herein. The first compound or pharmaceutically acceptable salt thereof, and the second compound or pharmaceutically acceptable salt thereof can both be present in one pharmaceutical composition or can be present in separate pharmaceutical compositions. If separate they can be co-administered as more fully discussed herein.

Thus, in one embodiment a pharmaceutical combination comprising a first compound corresponding in structure to Formula (I): 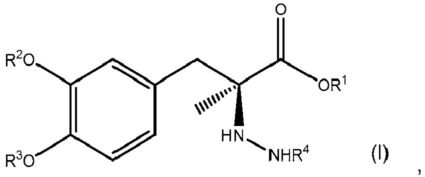 or a pharmaceutically acceptable salt thereof, wherein **R¹** is independently selected from the group consisting of hydrogen, 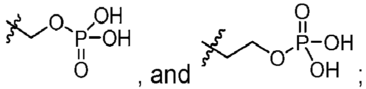 **R²** and **R³** are each independently selected from the group consisting of hydrogen, 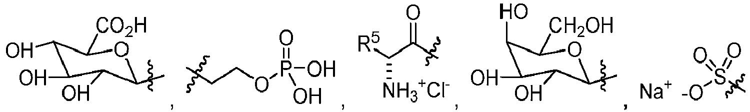 , and 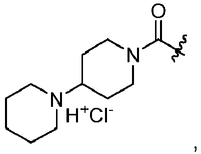 wherein **R⁵** is selected from the group consisting of hydrogen, methyl, and isopropyl; and **R⁴** is independently selected from the group consisting of hydrogen, 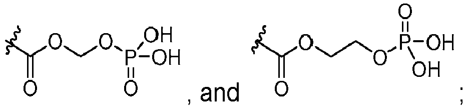 and
a second compound corresponding in structure Formula (II): 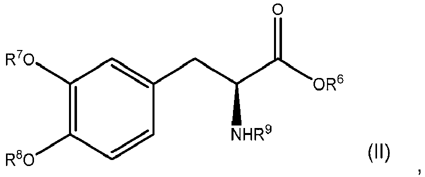 or a pharmaceutically acceptable salt thereof, wherein **R⁶** is independently selected from the group consisting of hydrogen, 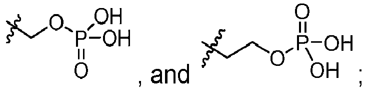 **R⁷** and **R⁸** are each independently selected from the group consisting of hydrogen, 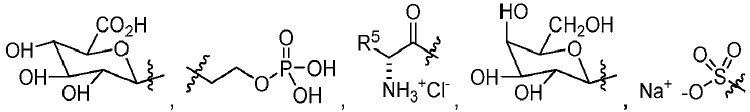 , and 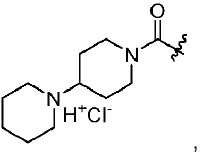 wherein **R⁵** is selected from the group consisting of hydrogen, methyl, and isopropyl; and **R⁹** is independently selected from the group consisting of hydrogen, 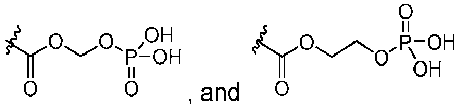 is provided herein.

### IV. Methods of Treatment

The present disclosure further relates to methods of treating Parkinson's disease and associated conditions comprising administering an effective amount of a carbidopa prodrug and an L-dopa prodrug to a patient.

In some embodiments, the methods of treating Parkinson's disease and associated conditions include providing a rescue therapy for treatment of Parkinson's disease and associated conditions. The term "rescue therapy" as used herein is any acute and intermittent therapy that may be used to treat the sudden re-immergence of motor symptoms (e.g. sudden "off" episode or "end-of-dose wearing off" and unpredictable "on/off" episodes). Patients with disabling motor complications can cycle between "off" time, which is defined as periods of poor mobility, slowness, and stiffness, and "on" time, which is defined as periods of good motor system control without troublesome dyskinesia.

In some embodiments, the carbidopa phosphate prodrug and the L-dopa prodrug are administered to the patient in the form of a pharmaceutical composition comprising both prodrugs. In other embodiments, the carbidopa prodrug and the L-dopa prodrug are separately administered to the patient.

### A. First Compound and Second Compound and Combinations Thereof

In one embodiment, the present disclosure relates to a method of treating a condition in a subject (e.g. patient) in need of such treatment, wherein the method comprises administering to the patient a pharmaceutical combination comprising a first compound and a second compound, wherein:
the first compound corresponds in structure to Formula (I): 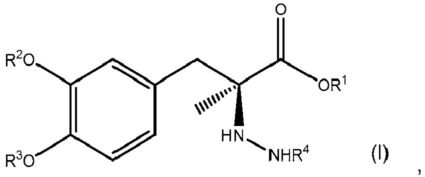 or a pharmaceutically acceptable salt thereof, wherein **R¹** is independently selected from the group consisting of hydrogen, 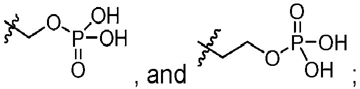 **R²** and **R³** are each independently selected from the group consisting of hydrogen, 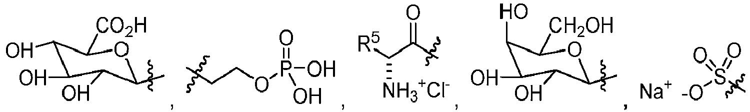 , and 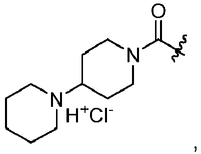 wherein **R⁵** is selected from the group consisting of hydrogen, methyl, and isopropyl; and **R⁴** is independently selected from the group consisting of hydrogen, 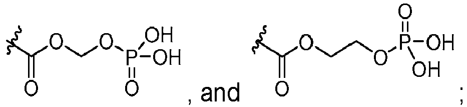 and
the second compound corresponds in structure to Formula (II): 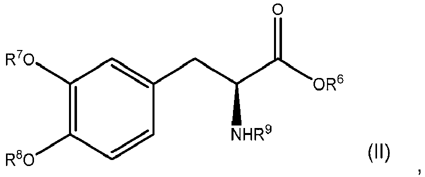 or a pharmaceutically acceptable salt thereof, wherein **R⁶** is independently selected from the group consisting of hydrogen, 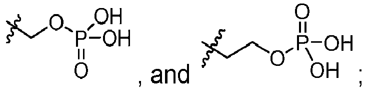 **R⁷** and **R⁸** are each independently selected from the group consisting of hydrogen, 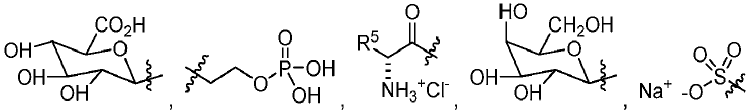 , and 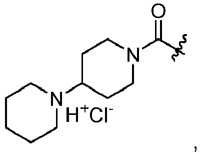 wherein **R⁵** is selected from the group consisting of hydrogen, methyl, and isopropyl; and **R⁹** is independently selected from the group consisting of hydrogen,

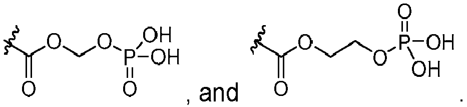

In one embodiment, the first compound and the second compound are administered in amounts that together provide a therapeutically effect for the subject (e.g. patient).

In one embodiment, the first compound corresponds in structure to Formula (I-a), and the second compound corresponds in structure to Formula (II-a).

In another embodiment, the first compound corresponds in structure to Formula (I-b), and the second compound corresponds in structure to Formula (II-a).

In another embodiment, the first compound corresponds in structure to Formula (I-c), and the second compound corresponds in structure to Formula (II-a).

In another embodiment, the first compound corresponds in structure to Formula (I-d), and the second compound corresponds in structure to Formula (II-a).

In another embodiment, the first compound corresponds in structure to Formula (I-e), and the second compound corresponds in structure to Formula (II-a).

In another embodiment, the first compound corresponds in structure to Formula (I-f), and the second compound corresponds in structure to Formula (II-a).

In another embodiment, the first compound corresponds in structure to Formula (I-a), and the second compound corresponds in structure to Formula (II-b).

In another embodiment, the first compound corresponds in structure to Formula (I-b), and the second compound corresponds in structure to Formula (II-b).

In another embodiment, the first compound corresponds in structure to Formula (I-c), and the second compound corresponds in structure to Formula (II-b).

In another embodiment, the first compound corresponds in structure to Formula (I-d), and the second compound corresponds in structure to Formula (II-b).

In another embodiment, the first compound corresponds in structure to Formula (I-e), and the second compound corresponds in structure to Formula (II-b).

In another embodiment, the first compound corresponds in structure to Formula (I-f), and the second compound corresponds in structure to Formula (II-b).

In another embodiment, the first compound corresponds in structure to Formula (I-a), and the second compound corresponds in structure to Formula (II-c).

In another embodiment, the first compound corresponds in structure to Formula (I-b), and the second compound corresponds in structure to Formula (II-c).

In another embodiment, the first compound corresponds in structure to Formula (I-c), and the second compound corresponds in structure to Formula (II-c).

In another embodiment, the first compound corresponds in structure to Formula (I-d), and the second compound corresponds in structure to Formula (II-c).

In another embodiment, the first compound corresponds in structure to Formula (I-e), and the second compound corresponds in structure to Formula (II-c).

In another embodiment, the first compound corresponds in structure to Formula (I-f), and the second compound corresponds in structure to Formula (II-c).

In another embodiment, the first compound corresponds in structure to Formula (I-a), and the second compound corresponds in structure to Formula (II-d).

In another embodiment, the first compound corresponds in structure to Formula (I-b), and the second compound corresponds in structure to Formula (II-d).

In another embodiment, the first compound corresponds in structure to Formula (I-c), and the second compound corresponds in structure to Formula (II-d).

In another embodiment, the first compound corresponds in structure to Formula (I-d), and the second compound corresponds in structure to Formula (II-d).

In another embodiment, the first compound corresponds in structure to Formula (I-e), and the second compound corresponds in structure to Formula (II-d).

In another embodiment, the first compound corresponds in structure to Formula (I-f), and the second compound corresponds in structure to Formula (II-d).

In another embodiment, the first compound corresponds in structure to Formula (I-a), and the second compound corresponds in structure to Formula (II-e).

In another embodiment, the first compound corresponds in structure to Formula (I-b), and the second compound corresponds in structure to Formula (II-e).

In another embodiment, the first compound corresponds in structure to Formula (I-c), and the second compound corresponds in structure to Formula (II-e).

In another embodiment, the first compound corresponds in structure to Formula (I-d), and the second compound corresponds in structure to Formula (II-e).

In another embodiment, the first compound corresponds in structure to Formula (I-e), and the second compound corresponds in structure to Formula (II-e).

In another embodiment, the first compound corresponds in structure to Formula (I-f), and the second compound corresponds in structure to Formula (II-e).

### B. Conditions Treated

In one embodiment, the condition treated by administering the first compound and the second compound is Parkinson's disease.

In another embodiment, the condition treated by administering the first compound and the second compound is sleep disturbance in a patient with Parkinson's disease (*i.e*., a method of reducing sleep disturbance in a patient with Parkinson's disease).

In another embodiment, the condition treated by administering the first compound and the second compound is impaired motor performance in a patient with Parkinson's disease (*i.e*., a method of improving motor performance in a patient with Parkinson's disease).

In another embodiment, the condition treated by administering the first compound and the second compound is nighttime disability in a patient with Parkinson's disease (*i.e*., a method of reducing nighttime disabilities in a patient with Parkinson's disease).

In another embodiment, the first compound and the second compound are administered to treat motor fluctuations in a patient with Parkinson's disease.

In another embodiment, the first compound and the second compound are administered to treat dyskinesia in a patient with Parkinson's disease.

In another embodiment, the first compound and the second compound are administered to delay the onset of motor fluctuations in a patient with Parkinson's disease.

In another embodiment, the first compound and the second compound are administered to delay the onset of dyskinesia in a patient with Parkinson's disease.

### C. Administering a Pharmaceutical Composition

In one embodiment, the present disclosure relates to a method of treating a condition in need of treatment, wherein the method comprises administering to a subject (e.g. patient) a therapeutically effective amount of a pharmaceutical composition of the present disclosure.

In one embodiment, the composition administered comprises a first compound corresponding in structure to Formula (I-a), and a second compound corresponding in structure to Formula (II-a).

In another embodiment, the composition administered comprises a first compound corresponding in structure to Formula (I-b), and a second compound corresponding in structure to Formula (II-a).

In another embodiment, the composition administered comprises a first compound corresponding in structure to Formula (I-c), and a second compound corresponding in structure to Formula (II-a).

In another embodiment, the composition administered comprises a first compound corresponding in structure to Formula (I-d), and a second compound corresponding in structure to Formula (II-a).

In another embodiment, the composition administered comprises a first compound corresponding in structure to Formula (I-e), and a second compound corresponding in structure to Formula (II-a).

In another embodiment, the composition administered comprises a first compound corresponding in structure to Formula (I-f), and a second compound corresponding in structure to Formula (II-a).

In another embodiment, the composition administered comprises a first compound corresponding in structure to Formula (I-a), and a second compound corresponding in structure to Formula (II-b).

In another embodiment, the composition administered comprises a first compound corresponding in structure to Formula (I-b), and a second compound corresponding in structure to Formula (II-b).

In another embodiment, the composition administered comprises a first compound corresponding in structure to Formula (I-c), and a second compound corresponding in structure to Formula (II-b).

In another embodiment, the composition administered comprises a first compound corresponding in structure to Formula (I-d), and a second compound corresponding in structure to Formula (II-b).

In another embodiment, the composition administered comprises a first compound corresponding in structure to Formula (I-e), and a second compound corresponding in structure to Formula (II-b).

In another embodiment, the composition administered comprises a first compound corresponding in structure to Formula (I-f), and a second compound corresponding in structure to Formula (II-b).

In another embodiment, the composition administered comprises a first compound corresponding in structure to Formula (I-a), and a second compound corresponding in structure to Formula (II-c).

In another embodiment, the composition administered comprises a first compound corresponding in structure to Formula (I-b), and a second compound corresponding in structure to Formula (II-c).

In another embodiment, the composition administered comprises a first compound corresponding in structure to Formula (I-c), and a second compound corresponding in structure to Formula (II-c).

In another embodiment, the composition administered comprises a first compound corresponding in structure to Formula (I-d), and a second compound corresponding in structure to Formula (II-c).

In another embodiment, the composition administered comprises a first compound corresponding in structure to Formula (I-e), and a second compound corresponding in structure to Formula (II-c).

In another embodiment, the composition administered comprises a first compound corresponding in structure to Formula (I-f), and a second compound corresponding in structure to Formula (II-c).

In another embodiment, the composition administered comprises a first compound corresponding in structure to Formula (I-a), and a second compound corresponding in structure to Formula (II-d).

In another embodiment, the composition administered comprises a first compound corresponding in structure to Formula (I-b), and a second compound corresponding in structure to Formula (II-d).

In another embodiment, the composition administered comprises a first compound corresponding in structure to Formula (I-c), and a second compound corresponding in structure to Formula (II-d).

In another embodiment, the composition administered comprises a first compound corresponding in structure to Formula (I-d), and a second compound corresponding in structure to Formula (II-d).

In another embodiment, the composition administered comprises a first compound corresponding in structure to Formula (I-e), and a second compound corresponding in structure to Formula (II-d).

In another embodiment, the composition administered comprises a first compound corresponding in structure to Formula (I-f), and a second compound corresponding in structure to Formula (II-d).

In another embodiment, the composition administered comprises a first compound corresponding in structure to Formula (I-a), and a second compound corresponding in structure to Formula (II-e).

In another embodiment, the composition administered comprises a first compound corresponding in structure to Formula (I-b), and a second compound corresponding in structure to Formula (II-e).

In another embodiment, the composition administered comprises a first compound corresponding in structure to Formula (I-c), and a second compound corresponding in structure to Formula (II-e).

In another embodiment, the composition administered comprises a first compound corresponding in structure to Formula (I-d), and a second compound corresponding in structure to Formula (II-e).

In another embodiment, the composition administered comprises a first compound corresponding in structure to Formula (I-e), and a second compound corresponding in structure to Formula (II-e).

In another embodiment, the composition administered comprises a first compound corresponding in structure to Formula (I-f), and a second compound corresponding in structure to Formula (II-e).

### D. Conditions Treated

In one embodiment, the condition treated by administering the pharmaceutical composition is Parkinson's disease.

In another embodiment, the condition treated by administering the pharmaceutical composition is sleep disturbance in a patient with Parkinson's disease (*i.e*., a method of reducing sleep disturbance in a patient with Parkinson's disease).

In another embodiment, the condition treated by administering the pharmaceutical composition is impaired motor performance in a patient with Parkinson's disease (*i.e*., a method of improving motor performance in a patient with Parkinson's disease).

In another embodiment, the pharmaceutical composition is administered to treat motor fluctuations in a patient with Parkinson's disease.

In another embodiment, the pharmaceutical composition is administered to treat dyskinesia in a patient with Parkinson's disease.

In another embodiment, the pharmaceutical composition is administered to delay the onset of motor fluctuations in a patient with Parkinson's disease.

In another embodiment, the pharmaceutical composition is administered to delay the onset of dyskinesia in a patient with Parkinson's disease.

In another embodiment, the condition treated by administering the pharmaceutical composition is nighttime disability in a patient with Parkinson's disease (*i.e.*, a method of reducing nighttime disabilities in a patient with Parkinson's disease).

### E. Weight ratios and administration routes

In general, the weight ratio of the first compound (*e.g*., the phosphate prodrugs) and the second compound (*e.g*., the phosphate prodrugs) administered to the patient (either separately or together in a single pharmaceutical composition) is from about 1:1 to about 1:50. In one aspect, the weight ratio is from about 1:2 to about 1:15. In another aspect, the weight ratio is from about 1:4 to about 1:10. In another aspect, the weight ratio is about 1:4. In another aspect, the weight ratio is about 1:7.5. In another aspect, the weight ratio is about 1:10.

In one embodiment, the first compound (*e.g*., the phosphate prodrugs) and the second compound (*e.g*., the phosphate prodrugs) are administered to the patient in the form of a solid composition (or solid compositions). In one aspect, the composition is suitable for oral administration.

In one embodiment, the first compound (*e.g.*, the phosphate prodrugs) and the second compound (*e.g.*, the phosphate prodrugs) are administered to the patient in the form of a liquid composition (or liquid compositions). In one aspect, the composition comprises water and is suitable for infusion.

In another embodiment, the first compound (*e.g.*, the phosphate prodrugs) and the second compound (*e.g.*, the phosphate prodrugs) are administered to the patient as a liquid composition (either separately or in the same pharmaceutical composition) that is suitable for intragastric, subcutaneous, intranasal, intramuscular or intravenous administration. In one aspect, the liquid composition(s) is suitable for intragastric administration. In another aspect, the liquid composition(s) is suitable for subcutaneous administration. In another aspect, the liquid composition(s) is suitable for intramuscular administration. In another aspect, the liquid composition(s) is suitable for intravenous administration. In another aspect, the liquid composition(s) is suitable for intranasal administration.

In another embodiment, the first compound (*e.g.*, the phosphate prodrugs) and the second compound (*e.g.*, the phosphate prodrugs) are administered via intestinal administration (*e.g.*, intrajejunum, intraduodenum) (either separately or in the same pharmaceutical composition). They can be administered (or "infused") directly into the intestine, *e.g.*, duodenum or the jejunum by a permanent tube inserted via percutaneous endoscopic gastrostomy, for example, with an outer transabdominal tube and an inner intestinal tube. In one aspect, the first compound (*e.g.*, the phosphate prodrugs) and the second compound (*e.g.*, the phosphate prodrugs) are administered via a tube inserted by radiological gastrojejunostomy. In another aspect, the first compound (*e.g.*, the phosphate prodrugs) and the second compound (*e.g.*, the phosphate prodrugs) are administered via a temporary nasoduodenal tube that is inserted into the patient initially to determine if the patient responds favorably to the treatment method before the permanent tube is inserted.

In some embodiments where the first compound (*e.g*., the phosphate prodrugs) and the second compound (*e.g.*, the phosphate prodrugs) are administered via intestinal administration, administration can be carried out using a portable pump, such as the pump sold under the trade name, CADD-Legacy Duodopa.RTM. pump^{®}. Specifically, a cassette, pouch, or vial comprising the first compound (*e.g.*, the phosphate prodrugs) and the second compound (*e.g.*, the phosphate prodrugs) can be attached to the pump to create the delivery system. The delivery system is then connected to the nasoduodenal tube, the transabdominal port, the duodenal tube, or the jejunum tube for intestinal administration.

In one embodiment, the method comprises administering the first compound (*e.g.*, the phosphate prodrugs) and the second compound (*e.g.*, the phosphate prodrugs) (either together or separately) to the patient substantially continuously over a period of at least about 12 hours. In additional aspects, the first compound (*e.g.*, the phosphate prodrugs) and the second compound (*e.g*., the phosphate prodrugs) are administered substantially continuously over a period of at least about 16 hours, at least about 24 hours, about 2 days, about 3 days, about 4 days, about 5 days, about 6 days, about one week, or longer. In particular, the first compound (*e.g.*, the phosphate prodrugs) and the second compound (*e.g.*, the phosphate prodrugs) may be subcutaneously administered substantially continuously over a period of at least about 16 hours.

### F. Dosing and Plasma Concentrations

In one embodiment, the dosing of the first compound (*e.g.*, the phosphate prodrugs) and the second compound (*e.g.*, the phosphate prodrugs) administered to the patient is adjusted to optimize the clinical response achieved by a subject (e.g. patient), which means maximizing the functional ON-time during the day by minimizing the number and duration of OFF-time episodes (*i.e*., bradykinesia) and minimizing ON-time with disabling dyskinesia.

In one embodiment, the daily dose of the L-dopa prodrug (*i.e.*, the second compound) administered to the patient according to methods of the present disclosure may be, for example, about 20 to about 1000000 mg, about 20 to about 100000 mg, about 20 to about 10000 mg, about 20 to about 5000 mg, about 20 mg to about 4000 mg, about 20 mg to about 3000 mg, about 20 mg to about 2000 mg, or about 20 mg to about 1000 mg per day. In particular, L-dopa phosphate prodrug, more particularly a compound corresponding in structure to Formula (II-a) and/or a compound corresponding in structure to Formula (II-b) are administered in the above daily doses.

In one embodiment, the daily dose of the carbidopa prodrug (i.e., the first compound) administered to the patient according to methods of the present disclosure may be, for example, 0 mg to about 2500 mg, 0 mg to about 1250 mg, 0 mg to about 1000 mg, 0 mg to about 750 mg, 0 mg to about 625 mg, 0 mg to about 500 mg, 0 mg to about 375 mg, 0 mg to about 250 mg, or 5 mg to about 125 mg per day. In particular, carbidopa phosphate prodrug, more particularly a compound corresponding in structure to Formula (I-a) and/or a compound corresponding in structure to Formula (I-b) are administered in the above daily doses.

In some embodiments, an amount of the first compound and an amount of the second compound are administered such that in combination they are sufficient to achieve an L-dopa plasma level in the patient of at least about 100 ng/mL. In one aspect, the L-dopa plasma level is at least about 200 ng/mL. In another aspect, the L-dopa plasma level is at least about 300 ng/mL. In another aspect, the L-dopa plasma level is at least about 400 ng/mL. In another aspect, the L-dopa plasma level is at least about 500 ng/mL. In another aspect, the L-dopa plasma level is at least about 600 ng/mL. In another aspect, the L-dopa plasma level is at least about 700 ng/mL. In another aspect, the L-dopa plasma level is at least about 800 ng/mL. In another aspect, the L-dopa plasma level is at least about 900 ng/mL. In another aspect, the L-dopa plasma level is at least about 1,000 ng/mL. In another aspect, the L-dopa plasma level is at least about 1,500 ng/mL. In another aspect, the L-dopa plasma level is at least about 2,000 ng/mL. In another aspect, the L-dopa plasma level is at least about 3,000 ng/mL. In another aspect, the L-dopa plasma level is at least about 4,000 ng/mL. In another aspect, the L-dopa plasma level is at least about 5,000 ng/mL. In another aspect, the L-dopa plasma level is at least about 6,000 ng/mL. In another aspect, the L-dopa plasma level is at least about 7,000 ng/mL. In another aspect, the L-dopa plasma level is at least about 8,000 ng/mL. In another aspect, the L-dopa plasma level is at least about 9,000 ng/mL. In particular, the first compound may be a carbidopa phosphate prodrug, more particularly a compound corresponding in structure to Formula (I-a) and/or a compound corresponding in structure to Formula (I-b). In particular, the second compound may be a L-dopa phosphate prodrug, more particularly a compound corresponding in structure to Formula (II-a) and/or a compound corresponding in structure to Formula (II-b).

In some embodiments, an amount of the first compound and an amount of the second compound are administered such that in combination they are sufficient to achieve an L-dopa plasma level from about 10 ng/mL to about 9,000 ng/mL. In one aspect, the L-dopa plasma level is from about 10 ng/mL to about 8,000 ng/mL In another aspect, the L-dopa plasma level is from about 25 ng/mL to about 6,000 ng/mL. In another aspect, the L-dopa plasma level is from about 50 ng/mL to about 4,000 ng/mL. In another aspect, the L-dopa plasma level is from about 100 ng/mL to about 2,000 ng/mL. In another aspect, the L-dopa plasma level is from about 25 ng/mL to about 1,200 ng/mL. In another aspect, the L-dopa plasma level is from about 10 ng/mL to about 500 ng/mL. In another aspect, the L-dopa plasma level is from about 25 ng/mL to about 500 ng/mL. In particular, the first compound may be a carbidopa phosphate prodrug, more particularly a compound corresponding in structure to Formula (I-a) and/or a compound corresponding in structure to Formula (I-b). In particular, the second compound may be a L-dopa phosphate prodrug, more particularly a compound corresponding in structure to Formula (II-a) and/or a compound corresponding in structure to Formula (II-b).

In some embodiments, the above-described L-dopa concentration ranges can be maintained for at least about a 1 hour interval, a 2 hour interval, a 3 hour interval, a 4 hour interval, a 5 hour interval, a 6 hour interval, a 7 hour interval, an 8 hour interval, a 9 hour interval, a 10 hour interval, an 11 hour interval, a 12 hour interval, a 13 hour interval, a 14 hour interval, a 15 hour interval, a 16 hour interval, a 17 hour interval, an 18 hour interval, a 19 hour interval, a 20 hour interval, a 21 hour interval, a 22 hour interval, a 23 hour interval, or a 24 hour interval.

In some embodiments, an amount of the first compound and an amount of the second compound are administered such that they are sufficient to maintain a carbidopa plasma level less than about 2500 ng/mL. In one aspect, the carbidopa plasma level is less than about 2000 ng/mL. In another aspect, the carbidopa plasma level is less than about 1500 ng/mL. In another aspect, the carbidopa plasma level is less than about 1000 ng/mL. In another aspect, the carbidopa plasma level is less than about 500 ng/mL. In another aspect, the carbidopa plasma level is less than about 250 ng/mL. In another aspect, the carbidopa plasma level is less than about 100 ng/mL. In another aspect, the carbidopa plasma level is less than about 50 ng/mL. In another aspect, the carbidopa plasma level is less than about 25 ng/mL.

In some embodiments, the above-described carbidopa plasma concentration ranges are maintained for at least about: a 1 hour interval, a 2 hour interval, a 3 hour interval, a 4 hour interval, a 5 hour interval, a 6 hour interval, a 7 hour interval, an 8 hour interval, a 9 hour interval, a 10 hour interval, an 11 hour interval, a 12 hour interval, a 13 hour interval, a 14 hour interval, a 15 hour interval, a 16 hour interval, a 17 hour interval, an 18 hour interval, a 19 hour interval, a 20 hour interval, a 21 hour interval, a 22 hour interval, a 23 hour interval, or a 24 hour interval.

### V. Co-Administration and/or Add-On Therapy

The methods of treatment of the present disclosure optionally can further comprise administration of one or more therapeutic agents for the treatment of Parkinson's disease (e.g. an anti-Parkinson's agent) in addition to the L-dopa prodrug and the carbidopa prodrug. In one embodiment, the additional therapeutic agent(s) is selected from the group consisting of decarboxylase inhibitors other than carbidopa (e.g., benserazide), catechol-0-methyl transferase ("COMT") inhibitors (e.g., entacapone and tolcapone), and monoamine oxidase A ("MAO-A") or monoamine oxidase B ("MAO-B") inhibitors (e.g., moclobemide, rasagiline, selegiline, and safinamide). In one aspect, the additional therapeutic agent(s) is selected from the group consisting of decarboxylase inhibitors other than carbidopa. In another aspect, the additional therapeutic agent(s) is selected from the group consisting of COMT inhibitors, such as entacapone. In another aspect, the additional therapeutic agent(s) is selected from the group consisting of MAO-A inhibitors. In another aspect, the additional therapeutic agent(s) is selected from the group consisting of MAO-B inhibitors.

The additional therapeutic agents and the first and second compound can be administered together or separately; and substantially simultaneously or subsequent to each other. Additionally, the additional therapeutic agents and the first and second compound can be in separate dosage forms which can be the same or different. For example, entacapone can be used adjunctively and can be orally delivered, and the first and the second compound discussed herein can be subcutaneously administered (separately or together in the same pharmaceutical composition). Further, the therapeutic agents and the first and the second compound can optionally be co-packaged, for example in a single container or in a plurality of containers within a single outer package, or co-presented in separate packaging ("common presentation").

In a similar manner, the pharmaceutical compositions of the present disclosure optionally can further comprise one or more additional therapeutic agents for the treatment of Parkinson's disease as described above.

### VI. Kits

The present disclosure also relates to kits comprising one or more pharmaceutical dosage forms comprising a carbidopa phosphate prodrug; kits comprising one or more pharmaceutical dosage forms comprising a an L-dopa phosphate prodrug; and kits comprising one or more pharmaceutical dosage forms comprising both a carbidopa phosphate prodrug and an L-dopa phosphate prodrug. The kit optionally can comprise one or more additional therapeutic agents and/or instructions, for example, instructions for using the kit to treat a patient having Parkinson's disease and an associated condition.

In one embodiment, the kit comprises a first pharmaceutical dosage form, wherein the first pharmaceutical dosage form comprises a first compound corresponding in structure to Formula (I), or a pharmaceutically acceptable salt thereof. In one aspect, the kit comprises a second pharmaceutical dosage form comprising a second compound corresponding in structure to Formula (II), or a pharmaceutically acceptable salt thereof. In another aspect, the first pharmaceutical dosage form further comprises a second compound corresponding in structure to Formula (II), or a pharmaceutically acceptable salt thereof. In another aspect, the first pharmaceutical dosage form and, where applicable, the second pharmaceutical dosage form are liquid pharmaceutical dosage forms.

As dopamine is an achiral compound, the various embodiments discussed above potentially could be adapted for use with a D-dopa phosphate prodrug or a racemate of D-dopa phosphate prodrug and L-dopa phosphate prodrug in place of the L-dopa phosphate prodrug.

### VII. Examples

The following non-limiting examples are provided to further illustrate the present disclosure. Abbreviations used in the examples below include the following:
"DBU" means1,8-diazabicyclo[5.4.0]-undec-7-ene.
"DCM" means dichloromethane.
"EDTA" means ethylenediaminetetraacetic acid.
"FCC" means flash column chromatography.
"HPLC" means high pressure liquid chromatography
"IPA" means isopropanol.
"LC-MS" means liquid chromatography-mass spectrometry.
"m-CPBA" means meta-chloroperoxybenzoic acid.
"MTBE" means methyl tertiary butyl ether.
"pa" means peak area.
"THF" means tetrahydrofuran.
"TLC" means thin layer chromatography.
"t_{1/2}" means biological half-life, *i.e.*, the time required for half the quantity of a drug or other substance administered to a living organism to be metabolized or eliminated by normal biological processes.

### Example 1: Synthesis of L-Dopa R⁷ or Monoprodrugs

L-dopa 3'-monoprodrugs and L-dopa 4'-monoprodrugs were prepared as shown in Scheme 1 below:

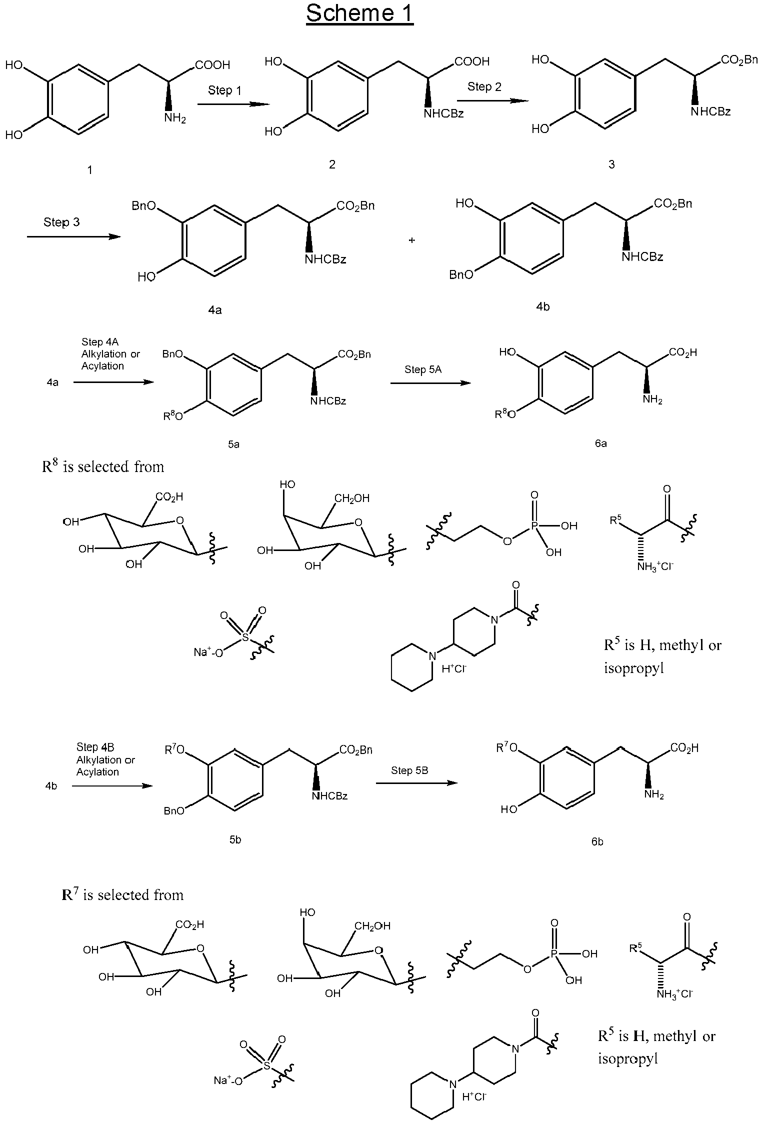

Specifically, L-dopa 3'-monoprodrugs and L-dopa 4'-monoprodrugs were prepared as described in Steps 1 through 5B below.

### Step 1

A solution of sodium hydroxide (40 g, 1.0 mol) in water (300 mL) was added drop-wise to a suspension of **Compound 1** (100 g, 0.5 mol) in water (300 mL) over a period of 20 minutes at 0°C. Benzylchloroformate (103.9 g, 0.6 mol) in dioxane (400 mL) was added drop-wise to the suspension over a period of 30 minutes at 0°C and then the reaction mass was stirred at room temperature for 16 hours. Reaction completion was monitored by TLC. After the complete consumption of starting material, the reaction mass was basified to pH = 10 using 10% sodium hydroxide (200 mL) and extracted with MTBE (500 mL). The organic layer was separated and discarded. The aqueous layer was acidified to pH = 2 using 6 N HCl (150 mL) and extracted with MTBE (500 mL × 2). The combined organic layer was washed with water (500 mL), saturated sodium chloride solution (500 mL), dried over sodium sulfate, and concentrated under vacuum at 45°C to 50°C to provide crude **Compound 2** as a viscous liquid (120 g, 72%).

### Step 2

Cesium carbonate (123 g, 0.37 mol) was added in two lots to a solution of **Compound 2** (250 g, 0.75 mol) in dimethylformamide (2 L) at 0° C. Benzyl bromide (90.3 mL, 0.75 mol) was added drop-wise to this mixture over a period of 30 minutes at 0°C and then the reaction mass was stirred at room temperature for 16 hours. Reaction completion was monitored by TLC. After the complete consumption of starting material, the reaction mass was diluted with water (5 L) and extracted with MTBE (1 L × 2). The combined organic layer was washed with water (1 L), saturated sodium chloride solution (0.5 L), dried over sodium sulfate, and concentrated under vacuum at 45°C to 50°C to provide crude **Compound 3** as a viscous liquid (250 g).

### Step 3

Cesium carbonate (698.5 g, 2.14 mol) was added in four lots to a solution of **Compound 3** (900 g, 2.14 mol) in dimethylformamide (7.2 L) at 0° C. Benzyl bromide (512 mL, 4.28 mol) was added drop-wise to this mixture over a period of one hour at 0°C and then the reaction mass was stirred at room temperature for 16 hours. Reaction completion was monitored by TLC. After the complete consumption of starting material, the reaction mass was diluted with water (15 L) and extracted with MTBE (3 L × 2). The combined organic layer was washed with water (3 L), saturated sodium chloride solution (1.5 L), dried over sodium sulfate, and concentrated under vacuum at 45°C to 50°C to provide a crude product as a viscous liquid (1 Kg).

The crude product obtained was blended with the crude product from previous batches (total of 1.6 Kg) and was repeatedly purified by flash column chromatography over silica gel (230-400 mesh) using 10-20 % ethyl acetate in petroleum ether to provide **Compounds 4a** (270 g) and **4b** (255 g).

### Step 4A

An appropriate base was added to a solution of **Compound 4a** in tetrahydrofuran at 0°C. A solution alkylating or acylating agent was added drop-wise to this mixture over a period of 30 minutes at 0°C and then the reaction mass was stirred at room temperature for 2 hours. Reaction completion was monitored by thin layer chromatography. After the complete consumption of starting material, the reaction mass was cooled to 0°C to 5°C and quenched with water. The organic layer was separated and the aqueous layer was extracted with toluene. The combined organic layer was washed with water, saturated NaCl solution, dried over sodium sulfate, and concentrated under vacuum at 45°C to 50°C. The crude product obtained was purified by column chromatography over silica gel (230-400 mesh) using 30%-40% ethyl acetate in petroleum ether to yield **Compound 5a.**

### Step 4B

An appropriate base was added to a solution of **Compound 4b** in tetrahydrofuran at 0°C. A solution of alkylating agent or acylating agent was added drop-wise to this mixture over a period of 30 minutes at 0°C. After complete addition, the reaction mass was stirred at room temperature for 2 hours. Reaction completion was monitored by thin layer chromatography. After reaction completion, the reaction mass was cooled to 0°C to 5°C and quenched with water. The organic layer was separated and the aqueous layer was extracted with toluene. The combined organic layer was washed with water, saturated NaCl solution, dried over sodium sulfate, and concentrated under vacuum at 45°C to 50°C. The crude product was purified by column chromatography over silica gel (230-400 mesh) using 30%-40% ethyl acetate in petroleum ether to yield **Compound 5b.**

### Step 5A

10% Pd/C was added to a solution of **Compound 5a** in ethanol and water (4:1 ratio) under nitrogen atmosphere. The reaction flask was evacuated and purged with hydrogen gas three times and then hydrogenated at 4 Kg/cm² pressure (approximately 4 atmospheres) for 16 hours. After the reaction was complete, water was added to the reaction mixture and the catalyst was removed by filtration through K100 cellulose filter pad. The filtrate was concentrated under reduced pressure. The crude product obtained was stirred with ethanol, filtered, and dried under suction to give the L-Dopa prodrug **Compound 6A.**

### Step 5B

10% Pd/C was added to a solution of **Compound 5b** in ethanol and water (4:1 ratio) under nitrogen atmosphere. The reaction flask was evacuated and purged with hydrogen gas three times and then hydrogenated at 4 Kg/cm² pressure (approximately 4 atmospheres) for 16 hours. After the reaction was complete, water was added to the reaction mixture and the catalyst was removed by filtration through K100 cellulose filter pad. The filtrate was concentrated under reduced pressure. The crude product obtained was stirred with ethanol filtered, and dried under suction to give the L-dopa prodrug **Compound 6B.**

### Example 2: Synthesis of L-Dopa Diprodrugs

L-dopa 3',4'-diprodrug was prepared as shown in Scheme 2 below: 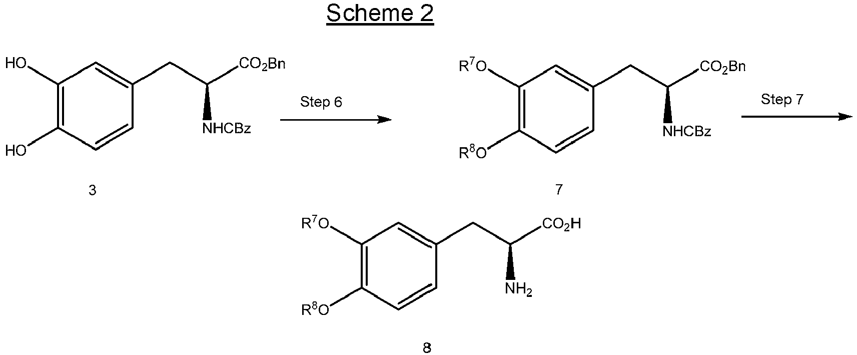 R⁷ and are the same group selected from the following: 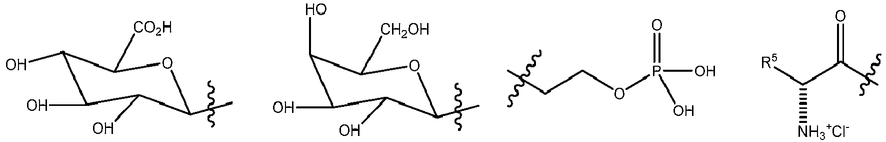 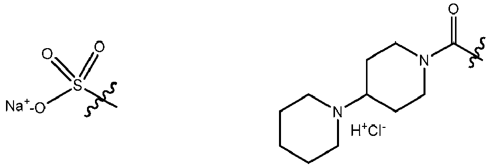 R⁵ is H, methyl or isopropyl Specifically, L-dopa diprodrugs were prepared as described in Steps 6 and 7 below.

### Step 6

Cesium carbonate was added in two lots to a solution of **Compound 3** in dimethylformamide at 0°C. A solution of alkylating or acylating agent was added drop-wise to this mixture over a period of one hour at 0°C and then the reaction mass was stirred at room temperature for 2 hours. Reaction completion was monitored by TLC. After the complete consumption of the starting material, the reaction mass was cooled to 0°C to 5°C and quenched with water. The organic layer was separated and the aqueous layer was extracted with toluene. The combined organic layer was washed with water, saturated sodium chloride solution, dried over sodium sulfate, filtered, and concentrated under vacuum at 45°C to 50°C. The crude product obtained was purified by column chromatography over silica gel (230-400 mesh) using 10%-15 % ethyl acetate in petroleum ether to provide **Compound 7.**

### Step 7

10 % Pd/C was added to a solution of **Compound 7** in THF under nitrogen atmosphere. The reaction flask was evacuated and purged with hydrogen gas three times and then hydrogenated at 6 Kg pressure for 8 hours. After the reaction was complete, water was added to the reaction mixture and the catalyst was removed by filtration through K100 cellulose filter pad. The filtrate was concentrated under reduced pressure to provide L-dopa diprodrug **Compound 8.**

### Example 3: Synthesis of Carbidopa Monoprodrugs and Carbidopa Diprodrugs

Carbidopa prodrugs were prepared as shown in Scheme 3 below: 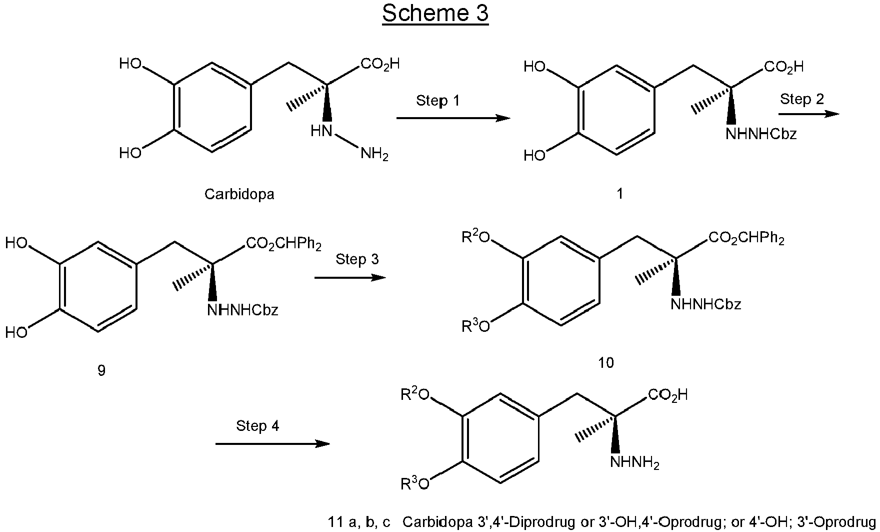 R² and R³ are both H or the same group selected from the following: 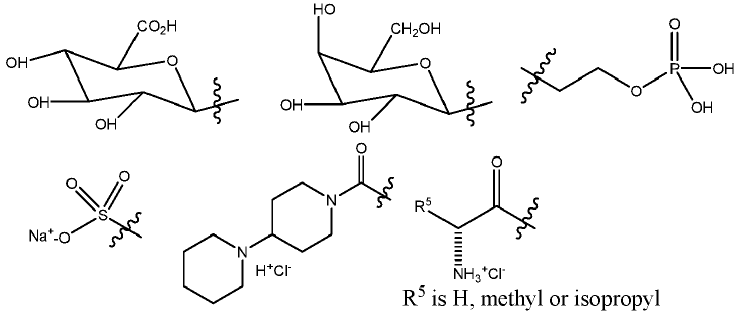 R² and R³ can also independently be H or a group selected from above.

Specifically, carbidopa prodrugs were prepared as described in Steps 1 through 4 below.

### Step 1

A slurry of carbidopa monohydrate (20.0 g, 82 mmol), sodium bicarbonate (7.57 g, 90 mmol), water (200 mL), and THF (100 mL) was cooled to 5°C to10°C and N-(benzyloxycarbonyloxy)succinimide (20.4 g, 82 mmol) was added. The mixture was warmed to ambient temperature and became a nearly homogeneous solution over 5 hours, when LC-MS showed nearly complete reaction. The solution was diluted with MTBE (100 mL), the layers separated, and organic layer extracted with saturated aqueous NaHCOs (100 mL). The aqueous layers were acidified with 2 N HCl (160 mL) and the acidic aqueous layer was extracted with MTBE (2 × 100 mL). During the second back-extraction, a small amount of product began to precipitate. The combined organic layers were washed with brine (20 mL) and residual solid rinsed out of the separatory funnel with MTBE (20 mL). The resulting mixture was concentrated to 43 g total mass and 10% THF/MTBE (60 mL) was added. The mixture was too thick to stir, so additional MTBE (60 mL to 6 vol 5% THF/MTBE) was added. The resulting white slurry was then heated to 50°C. The slurry was cooled to ambient temperature over one hour and then stirred for 14 hours. The white solid was filtered, washed with 5% THF/MTBE (20 mL), and dried in a vacuum oven (50°C), giving (S)-2-(2-((benzyloxy)carbonyl)-hydrazinyl)-3-(3,4-dihydroxyphenyl)-2-methylpropanoic acid compound with THF (4:3) (31.1 g, 71.9 mmol, 91 % yield) as a white solid. ¹H NMR (400 MHz, DMSO-*d*₆) δ 8.66 (d, *J =* 9.0 Hz, 2H), 8.18 (br s, 1H), 7.49 - 7.17 (m, 5H), 6.59 (dd, *J* = 5.0, 3.0 Hz, 2H), 6.44 (dd, *J* = 8.0, 2.0 Hz, 1H), 5.04 (s, 2H), 2.73 (d, *J =* 13.4 Hz, 1H), 2.59 (d, *J* = 13.3 Hz, 1H), 1.07 (s, 3H); MS (ESI) m/z 361 [M+H]+.

### Step 2

A solution of benzophenone hydrazone (20.0 g, 102 mmol) in DCM (100 mL) was cooled to < 0 °C and iodine (0.052 g, 0.204 mmol) and 1,1,3,3-tetramethylguanidine (25.6 mL, 204 mmol) were added. *m*-CPBA (30.5 g, 132 mmol) was added portion-wise between -10°C and 0°C over 5 minutes (exothermic, dry ice/acetone bath to control). The mixture was stirred between 0°C and 12°C for 15 minutes and then washed with water (3 × 200 mL). The resulting mixture was dried (Na₂SO₄), concentrated to 76 mL total volume, and rinsed into a 125 mL Erlenmeyer flask with an additional 16 mL of DCM, to make an approximately 1 M dark purple solution of (diazomethylene)dibenzene. In a separate flask, a slurry of (S)-2-(2-((benzyloxy)carbonyl)hydrazinyl)-3-(3,4-dihydroxyphenyl)-2-methylpropanoic acid compound with tetrahydrofuran (4:3) (30.7 g, 74.0 mmol) in IPA (300 mL) was cooled to less than 10°C and the (diazomethylene)dibenzene solution (78 mL, 78 mmol) was added. The resulting mixture was warmed to room temperature and LC-MS showed a stalled reaction after 30 minutes. Additional diphenyldiazomethane (0.2 eq, 14 mL) was added and stirring continued at room temperature. After 35 minutes, the remaining diphenyldiazomethane solution (9 mL) was added. After 2hours, 20 minutes, a purple color persisted and LC-MS showed that the reaction was complete. The reaction mixture was concentrated to approximately 60 mL and 20% aq. CH₃CN (300 mL) was added. The mixture was washed with cyclohexane (10 × 300 mL), ethyl acetate (450 mL) added, and the mixture was washed with saturated aqueous NaHCOs (150 mL) and brine (60 mL). The mixture was dried (Na₂SO₄) and concentrated, giving **Compound 9,** (S)-benzyl 2-(1-(benzhydryloxy)-3-(3,4-dihydroxyphenyl)-2-methyl-1-oxopropan-2-yl)hydrazine-carboxylate (39.4 g, 74.8 mmol, >99 % yield). ¹H NMR (400 MHz, DMSO-*d*₆) δ 8.65 (br s, 2H), 8.19 (br s, 1H), 7.43 - 7.20 (m, 15H), 6.70 (s, 1H), 6.55 (d, *J* = 2.0 Hz, 1H), 6.45 (d, *J =* 8.0 Hz, 1H), 6.20 (dd, *J* = 7.9, 2.0 Hz, 1H), 4.95 (d, *J =* 3.4 Hz, 2H), 2.81 (d, *J* = 13.6 Hz, 1H), 2.67 (d, *J* = 13.7 Hz, 1H), 1.17 (d, *J=* 3.1 Hz, 3H); MS (ESI) m/z 549 [M+Na]+.

### Step 3

A solution of (S)-benzyl 2-(1-(benzhydryloxy)-3-(3,4-dihydroxyphenyl)-2-methyl-1-oxopropan-2-yl)hydrazinecarboxylate and CH₃CN was cooled to less than 0°C and DBU and a prodrug alkylating or acylating agent were added at less than 0°C. After 40 minutes, water was added giving a biphasic solution. The layers were separated; the bottom layer was washed with cold 1:1 CH₃CN/water, then diluted with ethyl acetate, and washed with brine. the mixture was dried (Na₂SO₄) and concentrated. FCC (50-100% MTBE/heptanes) gave the mixture of carbidopa prodrugs **Compound 10.**

### Step 4

A solution of the mixture of carbidopa prodrugs prepared in step 3 in THF was added to 5% Pd/C (wet JM#9) in a stainless steel pressure bottle. The mixture was shaken under 60 psi of hydrogen at 22°C for 2 hours. Water was then added and the hydrogenation was continued for another 17 hours. The mixture was filtered through a nylon membrane with 100 mL of washing with 1:1 THF-water. The mixture was diluted with MTBE and the layers were separated. The aqueous layer was washed with MTBE then concentrated on a rotary evaporator to total mass, and lyophilized for 3 days to a white glass. The amorphous solid was broken up and lyophilized for one day to remove traces of additional water, giving the carbidopa prodrugs, **Compounds 11a, 11b, and 11c.**

### Example 4: Synthesis of L-Dopa Amine Prodrugs

L-dopa amine prodrugs were prepared as shown in Scheme 4 below:

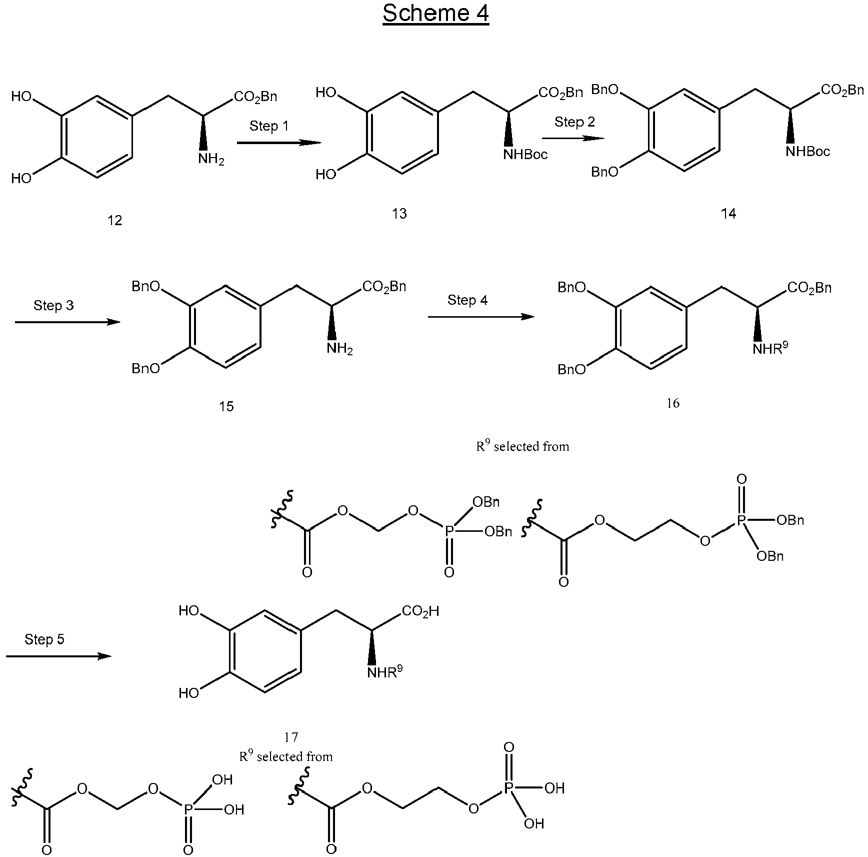

Specifically, L-dopa amine prodrugs in Steps 1 through 5 below.

### Step 1

**Compound 12** was combined with triethylamine and BOC anhydride in methylene chloride at RT for 1 hr. Reaction completion was monitored by TLC. After the complete consumption of starting material, the reaction mass was washed with saturated sodium chloride solution, dried over sodium sulfate, and concentrated under vacuum at 45°C to 50°C to provide crude **Compound 13.**

### Step 2

Cesium carbonate was added to a solution of **Compound 13** in dimethylformamide at 0° C. Benzyl bromide was added drop-wise to this mixture over a period of 30 minutes at 0°C and then the reaction mass was stirred at room temperature for 16 hours. Reaction completion was monitored by TLC. After the complete consumption of starting material, the reaction mass was diluted with water and extracted with MTBE. The combined organic layer was washed with water, saturated sodium chloride solution, dried over sodium sulfate, and concentrated under vacuum at 45°C to 50°C to provide crude **Compound 14** as a viscous liquid.

### Step 3

**Compound 14** was dissolved in dichloromethane and treated with trifluoroacetic acid to provide **Compound 15.** Reaction completion was monitored by TLC. After the complete consumption of starting material, the reaction mass was quenched with a saturated solution of sodium bicarbonate and extracted with MTBE. The combined organic layer was washed with water, saturated sodium chloride solution, dried over sodium sulfate, and concentrated under vacuum at 45°C to 50°C to provide a crude product.

### Step 4

An appropriate base was added to a solution of **Compound 15** in tetrahydrofuran at 0°C. A solution acylating agent was added drop-wise to this mixture over a period of 30 minutes at 0°C and then the reaction mass was stirred at room temperature for 2 hours. Reaction completion was monitored by thin layer chromatography. After the complete consumption of starting material, the reaction mass was cooled to 0°C to 5°C and quenched with water. The organic layer was separated and the aqueous layer was extracted with toluene (500 mL). The combined organic layer was washed with water, saturated NaCl solution, dried over sodium sulfate, and concentrated under vacuum at 45°C to 50°C. The crude product obtained was purified by column chromatography over silica gel (230-400 mesh) using 30%-40% ethyl acetate in petroleum ether to yield

### Compound 16.

### Step 5

10% Pd/C was added to a solution of **Compound 16** in ethanol and water (4:1) under nitrogen atmosphere. The reaction flask was evacuated and purged with hydrogen gas three times and then hydrogenated at 4 Kg/cm² pressure (approximately 4 atmospheres) for 16 hours. After the reaction was complete, water was added to the reaction mixture and the catalyst was removed by filtration through K100 cellulose filter pad. The filtrate was concentrated under reduced pressure. The crude product obtained was stirred with ethanol, filtered, and dried under suction to give the L-Dopa prodrug **Compound 17.**

### Example 5: Synthesis of Carbidopa Amine prodrugs

Carbidopa prodrugs were prepared as shown in Scheme 5 below: 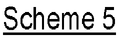 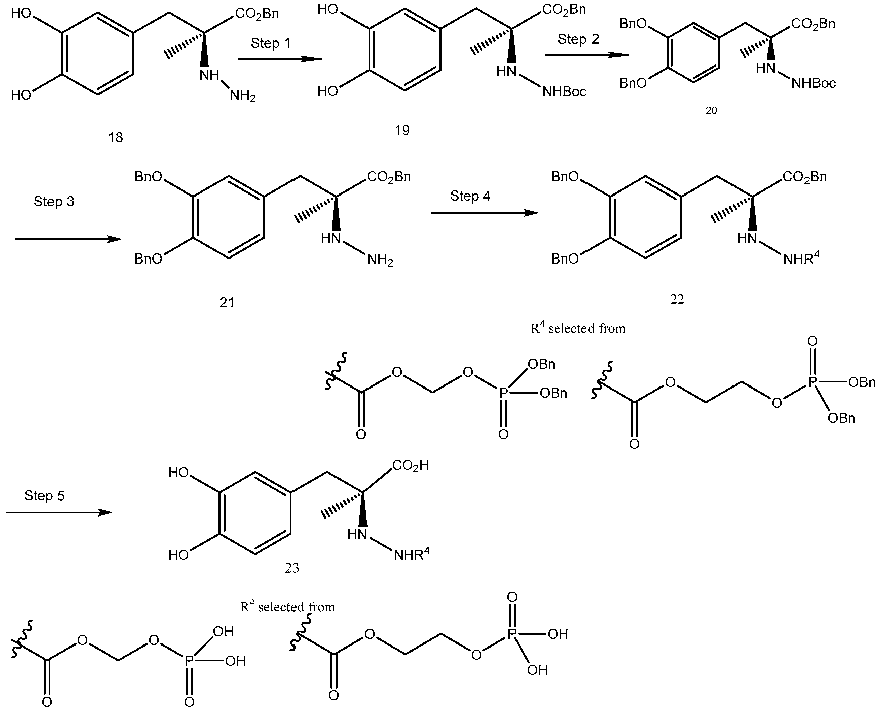 Specifically, Carbidopa amine prodrugs in Steps 1 through 5 below.

### Step 1

**Compound 18** was combined with triethylamine and BOC anhydride in methylene chloride at RT for 1 hr. Reaction completion was monitored by TLC. After the complete consumption of starting material, the reaction mass was washed with saturated sodium chloride solution, dried over sodium sulfate, and concentrated under vacuum at 45°C to 50°C to provide crude **Compound 19.**

### Step 2

Cesium carbonate was added to a solution of **Compound 19** in dimethylformamide at 0° C. Benzyl bromide was added drop-wise to this mixture over a period of 30 minutes at 0°C and then the reaction mass was stirred at room temperature for 16 hours. Reaction completion was monitored by TLC. After the complete consumption of starting material, the reaction mass was diluted with water and extracted with MTBE. The combined organic layer was washed with water, saturated sodium chloride solution, dried over sodium sulfate, and concentrated under vacuum at 45°C to 50°C to provide crude **Compound 20** as a viscous liquid.

### Step 3

**Compound 20** was dissolved in dichloromethane and treated with trifluoroacetic acid to provide **Compound 21.** Reaction completion was monitored by TLC. After the complete consumption of starting material, the reaction mass was quenched with a saturated solution of sodium bicarbonate and extracted with MTBE. The combined organic layer was washed with water, saturated sodium chloride solution, dried over sodium sulfate, and concentrated under vacuum at 45°C to 50°C to provide a crude product.

### Step 4

An appropriate base was added to a solution of **Compound 21** in tetrahydrofuran at 0°C. A solution acylating agent was added drop-wise to this mixture over a period of 30 minutes at 0°C and then the reaction mass was stirred at room temperature for 2 hours. Reaction completion was monitored by thin layer chromatography. After the complete consumption of starting material, the reaction mass was cooled to 0°C to 5°C and quenched with water. The organic layer was separated and the aqueous layer was extracted with toluene. The combined organic layer was washed with water, saturated NaCl solution, dried over sodium sulfate, and concentrated under vacuum at 45°C to 50°C. The crude product obtained was purified by column chromatography over silica gel (230-400 mesh) using 30%-40% ethyl acetate in petroleum ether to yield **Compound 22.**

### Step 5

10% Pd/C was added to a solution of **Compound 22** in ethanol and water (4:1) under nitrogen atmosphere. The reaction flask was evacuated and purged with hydrogen gas three times and then hydrogenated at 4 Kg/cm² pressure (approximately 4 atmospheres) for 16 hours. After the reaction was complete, water was added to the reaction mixture and the catalyst was removed by filtration through K100 cellulose filter pad. The filtrate was concentrated under reduced pressure. The crude product obtained was stirred with ethanol, filtered, and dried under suction to give the Carbidopa prodrug **Compound 23**.

### Example 6: Synthesis of Levodopa Carboxylic Acid Prodrugs

Levodopa carboxylic acid prodrugs were prepared as shown in Scheme 6 below:

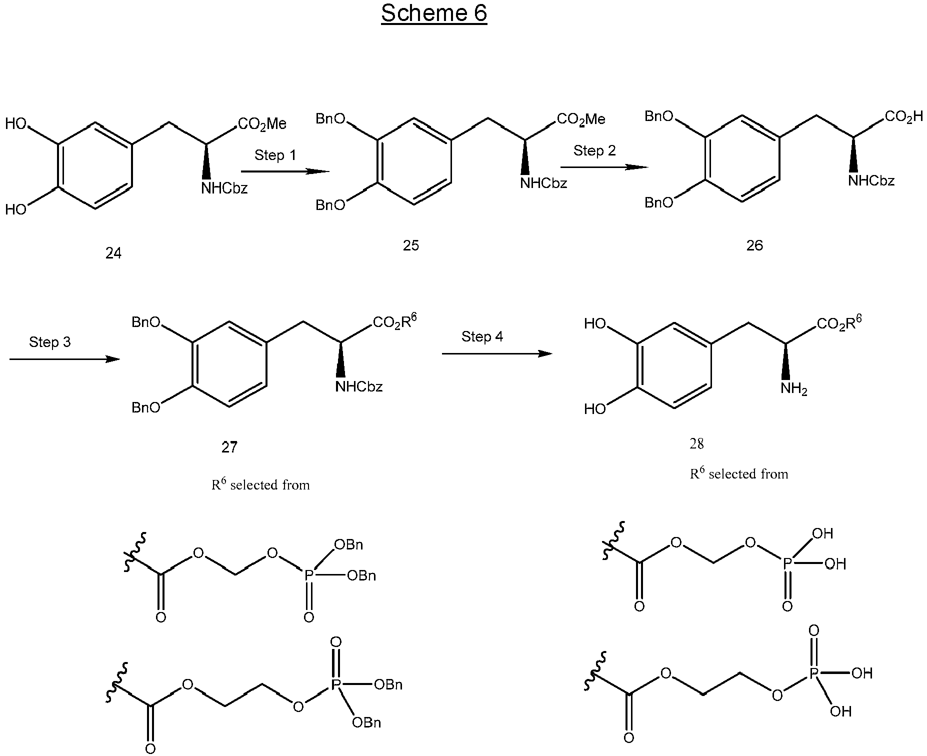

Specifically, Levodopa carboxylic acid prodrugs were prepared in Steps 1 through 4 below.

### Step 1

Cesium carbonate was added to a solution of **Compound 24** in dimethylformamide at 0° C. Benzyl bromide was added drop-wise to this mixture over a period of 30 minutes at 0°C and then the reaction mass was stirred at room temperature for 16 hours. Reaction completion was monitored by TLC. After the complete consumption of starting material, the reaction mass was diluted with water and extracted with MTBE. The combined organic layer was washed with water, saturated sodium chloride solution, dried over sodium sulfate, and concentrated under vacuum at 45°C to 50°C to provide crude **Compound 25** as a viscous liquid.

### Step 2

**Compound 25** was dissolved in a mixture of THF and water (3:1), and treated with a solution of lithium hydroxide. After the complete consumption of starting material, the reaction mass was quenched with a solution of 1N hydrochloric acid, and extracted with MTBE. The combined organic layer was washed with water, saturated sodium chloride solution, dried over sodium sulfate, and concentrated under vacuum at 45°C to 50°C to provide crude **Compound 26** as a viscous liquid.

### Step 3

An appropriate base was added to a solution of **Compound 26** in tetrahydrofuran at 0°C. A solution acylating agent was added drop-wise to this mixture over a period of 30 minutes at 0°C and then the reaction mass was stirred at room temperature for 2 hours. Reaction completion was monitored by thin layer chromatography. After the complete consumption of starting material, the reaction mass was cooled to 0°C to 5°C and quenched with water. The organic layer was separated and the aqueous layer was extracted with toluene. The combined organic layer was washed with water, saturated NaCl solution, dried over sodium sulfate, and concentrated under vacuum at 45°C to 50°C. The crude product obtained was purified by column chromatography over silica gel (230-400 mesh) using 30%-40% ethyl acetate in petroleum ether to yield **Compound 27.**

### Step 4

10% Pd/C was added to a solution of **Compound 27** in ethanol and water (4:1) under nitrogen atmosphere. The reaction flask was evacuated and purged with hydrogen gas three times and then hydrogenated at 4 Kg/cm² pressure (approximately 4 atmospheres) for 16 hours. After the reaction was complete, water was added to the reaction mixture and the catalyst was removed by filtration through K100 cellulose filter pad. The filtrate was concentrated under reduced pressure. The crude product obtained was stirred with ethanol, filtered, and dried under suction to give the Levodopa carboxylic acid prodrug **Compound 28.**

### Example 7: Synthesis of Carbidopa Carboxylic Acid Prodrugs

Carbidopa carboxylic acid prodrugs were prepared as shown in Scheme 7 below:

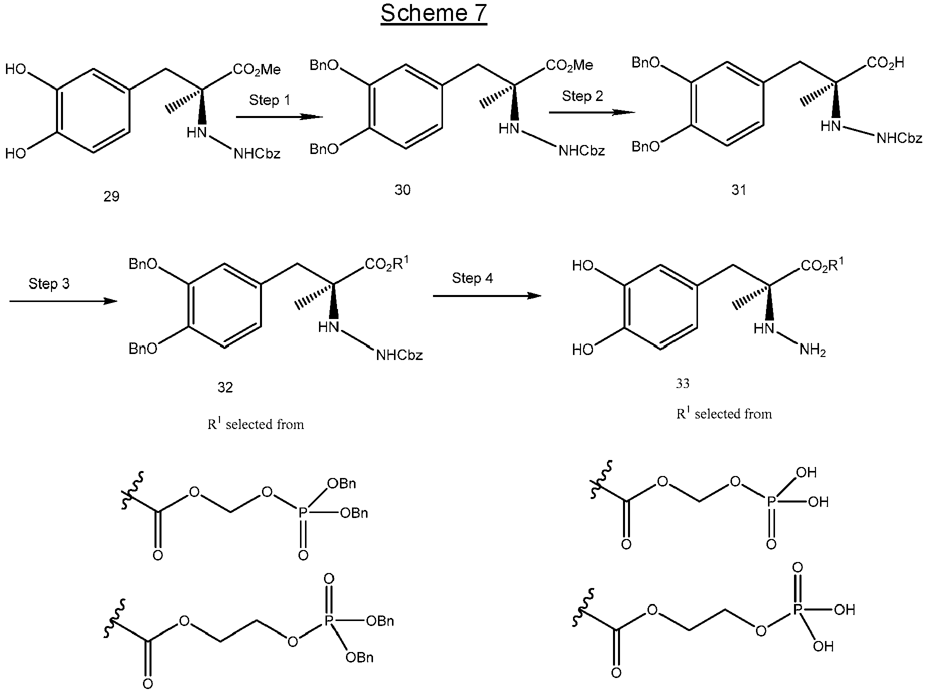

Specifically, Carbidopa carboxylic acid prodrugs were prepared in Steps 1 through 4 below.

### Step 1

Cesium carbonate was added to a solution of **Compound 29** in dimethylformamide at 0° C. Benzyl bromide was added drop-wise to this mixture over a period of 30 minutes at 0°C and then the reaction mass was stirred at room temperature for 16 hours. Reaction completion was monitored by TLC. After the complete consumption of starting material, the reaction mass was diluted with water and extracted with MTBE. The combined organic layer was washed with water, saturated sodium chloride solution, dried over sodium sulfate, and concentrated under vacuum at 45°C to 50°C to provide crude **Compound 30** as a viscous liquid.

### Step 2

**Compound 30** was dissolved in a mixture of THF and water (3:1), and treated with a solution of lithium hydroxide. After the complete consumption of starting material, the reaction mass was quenched with a solution of 1N hydrochloric acid, and extracted with MTBE. The combined organic layer was washed with water, saturated sodium chloride solution, dried over sodium sulfate, and concentrated under vacuum at 45°C to 50°C to provide crude **Compound 31** as a viscous liquid.

### Step 3

An appropriate base was added to a solution of **Compound 31** in tetrahydrofuran at 0°C. A solution acylating agent was added drop-wise to this mixture over a period of 30 minutes at 0°C and then the reaction mass was stirred at room temperature for 2 hours. Reaction completion was monitored by thin layer chromatography. After the complete consumption of starting material, the reaction mass was cooled to 0°C to 5°C and quenched with water. The organic layer was separated and the aqueous layer was extracted with toluene. The combined organic layer was washed with water, saturated NaCl solution, dried over sodium sulfate, and concentrated under vacuum at 45°C to 50°C. The crude product obtained was purified by column chromatography over silica gel (230-400 mesh) using 30%-40% ethyl acetate in petroleum ether to yield **Compound 32.**

### Step 4

10% Pd/C was added to a solution of **Compound 32** in ethanol and water (4:1) under nitrogen atmosphere. The reaction flask was evacuated and purged with hydrogen gas three times and then hydrogenated at 4 Kg/cm² pressure (approximately 4 atmospheres) for 16 hours. After the reaction was complete, water was added to the reaction mixture and the catalyst was removed by filtration through K100 cellulose filter pad. The filtrate was concentrated under reduced pressure. The crude product obtained was stirred with ethanol, filtered, and dried under suction to give the Carbidopa carboxylic acid prodrug **Compound 33.**

### VIII. Further Embodiments

Embodiment 1. A pharmaceutical combination comprising a first compound corresponding in structure to Formula (I): 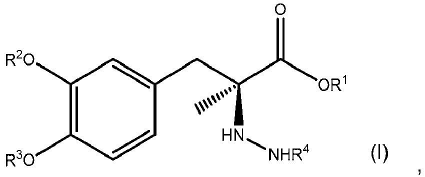 or a pharmaceutically acceptable salt thereof, wherein **R¹** is independently selected from the group consisting of hydrogen, 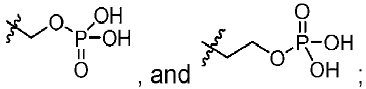 **R²** and **R³** are each independently selected from the group consisting of hydrogen, 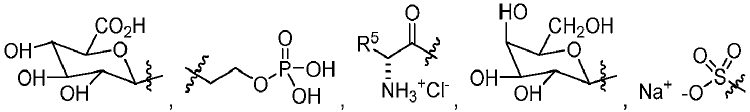 , and 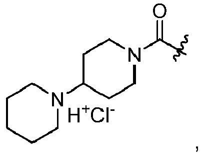 wherein **R⁵** is selected from the group consisting of hydrogen, methyl, and isopropyl; and **R⁴** is independently selected from the group consisting of hydrogen, 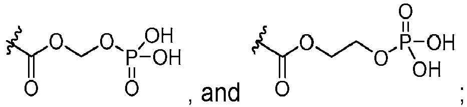 and a second compound corresponding in structure Formula (II): 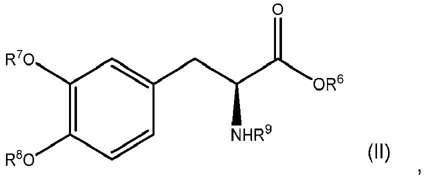 or a pharmaceutically acceptable salt thereof, wherein **R⁶** is independently selected from the group consisting of hydrogen, 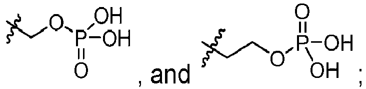 **R⁷** and **R⁸** are each independently selected from the group consisting of hydrogen, 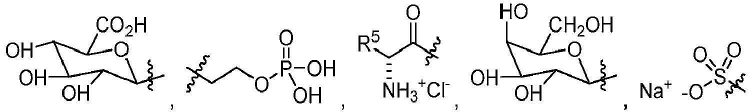 , and 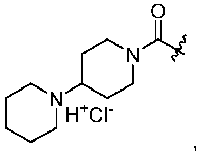 wherein **R⁵** is selected from the group consisting of hydrogen, methyl, and isopropyl; and **R⁹** is independently selected from the group consisting of hydrogen,

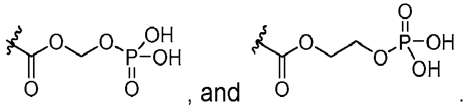

Embodiment 2. The pharmaceutical combination of Embodiment 1, wherein the first compound is 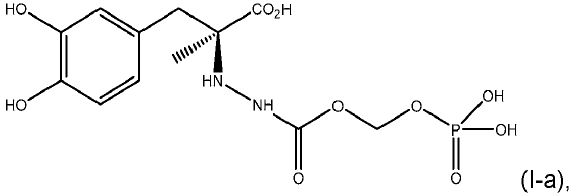 or

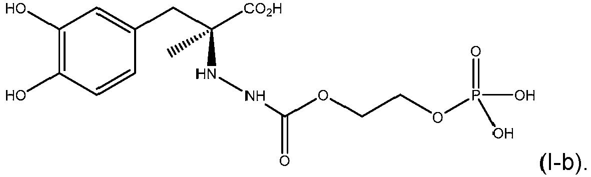

Embodiment 3. The pharmaceutical combination of Embodiment 1 or 2, wherein the second compound is 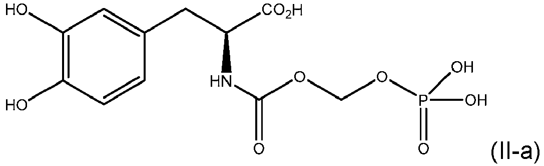 or

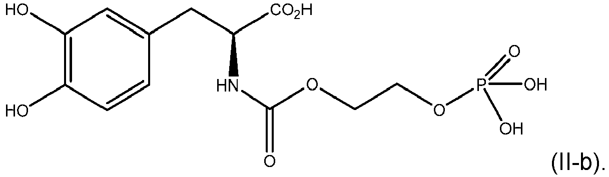

Embodiment 4. The pharmaceutical combination of any one of Embodiments 1-3, wherein the first compound or pharmaceutically acceptable salt thereof, and the second compound or pharmaceutically acceptable salt thereof are present in separate pharmaceutical compositions or are both present in the same pharmaceutical composition.

Embodiment 5. The pharmaceutical combination of any one of Embodiments 1-4, wherein a weight ratio of the first compound or pharmaceutically acceptable salt thereof to the second compound or pharmaceutically acceptable salt thereof is about 1:1 to about 1:50, preferably about 1:2 to about 1:15, preferably about 1:4 to about 1:10, and more preferably about 1:4.

Embodiment 6. The pharmaceutical combination of any one of Embodiments 1-5, wherein the first compound or pharmaceutically acceptable salt thereof has a solubility of at least about 200 mg/ml in aqueous solution at about neutral pH, and the second compound or pharmaceutically acceptable salt thereof has a solubility of at least about 400 mg/ml in aqueous solution at about neutral pH.

Embodiment 7. The pharmaceutical combination of any one of Embodiments 1-6, wherein the combination is an aqueous combination suitable for intragastric, subcutaneous, intramuscular, intrajejunum, oral, intranasal or intravenous administration.

Embodiment 8. The pharmaceutical combination of any one of Embodimentss 1-7, wherein the combination is an aqueous combination suitable for subcutaneous administration.

Embodiment 9. The pharmaceutical combination of any one of Embodiments 1-8, wherein the first compound is a compound corresponding in structure to Formula (I-a): 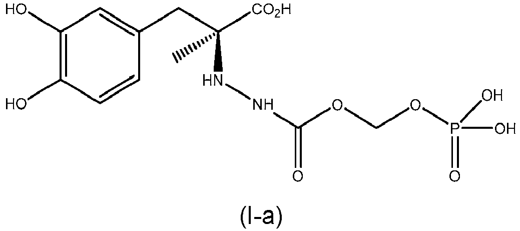 or a pharmaceutically acceptable salt thereof; and the second compound is a compound corresponding in structure to Formula (II-a): 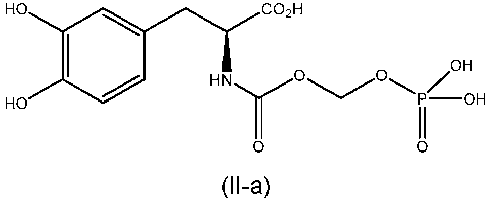 or a pharmaceutically acceptable salt thereof.

Embodiment 10. The pharmaceutical combination of any one of Embodiments 1-9, wherein the first compound is a compound corresponding in structure to Formula (I-a): 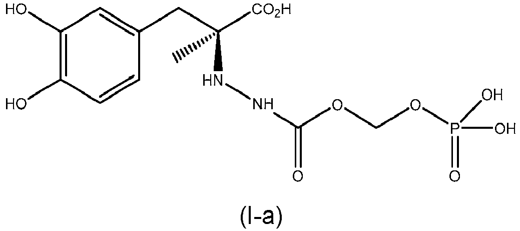 or a pharmaceutically acceptable salt thereof; and the second compound is a compound corresponding in structure to Formula (II-b): 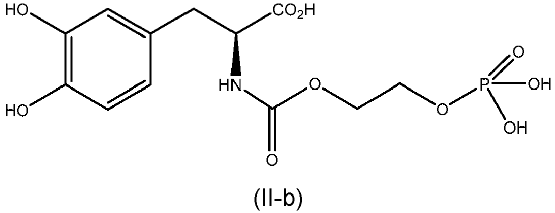 or a pharmaceutically acceptable salt thereof.

Embodiment 11. The pharmaceutical combination of any one of Embodiments 1-10, wherein the first compound is a compound corresponding in structure to Formula (I-b): 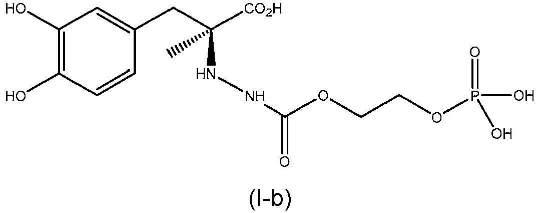 or a pharmaceutically acceptable salt thereof; and the second compound is a compound corresponding in structure to Formula (II-a): 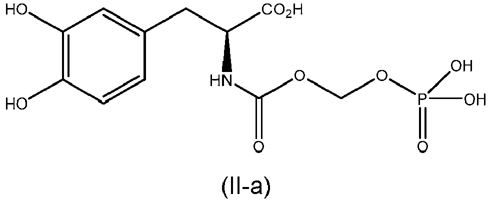 or a pharmaceutically acceptable salt thereof.

Embodiment 12. The pharmaceutical combination of any one of Embodiments 1-11, wherein the first compound is a compound corresponding in structure to Formula (I-b): 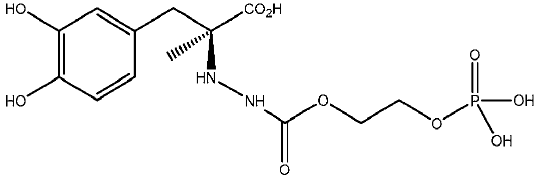 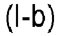 or a pharmaceutically acceptable salt thereof; and the second compound is a compound corresponding in structure to Formula (II-b): 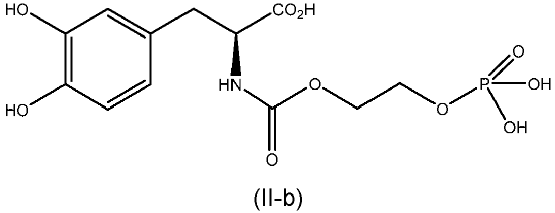 or a pharmaceutically acceptable salt thereof.

Embodiment 13. A method of treating Parkinson's disease in a subject in need thereof and/or a method of providing rescue therapy in a subject having Parkinson's disease, the method comprising administering to the subject a therapeutically effective amount of the pharmaceutical combination according to any one of Embodiments 1-12.

Embodiment 14. The method of Embodiment 13, wherein the first compound and the second compound are administered in separate pharmaceutical compositions to the subject, or the first compound and the second compound are administered to the subject in the same pharmaceutical composition comprising the first compound and the second compound.

Embodiment 15. The method of Embodiment 13 or 14, wherein the method comprises intragastric, subcutaneous, intrajejunum, oral, intranasal, intramuscular or intravenous administration of the first compound and the second compound.

Embodiment 16. The method of any one of Embodiments 13-15, wherein the method comprises subcutaneous administration of the first compound and the second compound.

Embodiment 17. The method of any one of Embodiments 13-16, wherein the method comprises substantially continuous administration of the first compound and the second compound over a period of at least about 12 hours.

Embodiment 18. The method of any one Embodiments 13-17, wherein the weight ratio of the first compound administered to the second compound administered is from about 1:1 to about 1:50.

Embodiment 19. The method of any one of Embodiments 13-18, wherein the weight ratio of the first compound administered to the second compound administered is from about 1:2 to about 1:15.

Embodiment 20. The method of any one of Embodiments 13-19, wherein the weight ratio of the first compound administered to the second compound administered is from about 1:4 to about 1:10.

Embodiment 21. The method of any one of Embodiments 13-20, wherein the weight ratio of the first compound administered to the second compound administered is about 1:4.

Embodiment 22. The method of any one of Embodiments 13-21, wherein the weight ratio of the first compound administered to the second compound administered is about 1:7.5.

Embodiment 23. The method of any one of Embodiments 13-22, wherein the weight ratio of the first compound administered to the second compound administered is about 1:10.

Embodiment 24. The method of any one of Embodiments 13-23, wherein the first compound is selected from the group consisting of 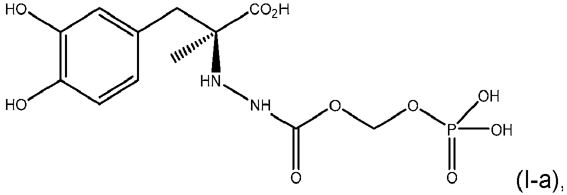 or 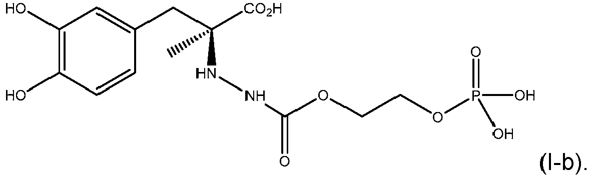 and the second compound is selected from the group consisting of 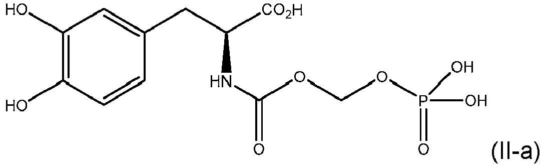 or

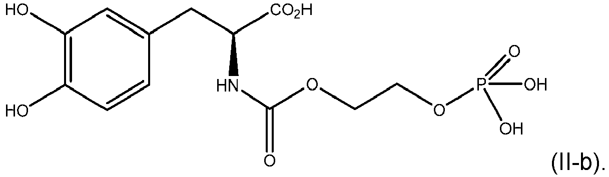

Embodiment 25. The method of any one of Embodiments 13-24 further comprising administering another anti-Parkinson's agent to the subject.

Embodiment 26. The method of any one of Embodiments 13-25, wherein the pharmaceutical combination is an aqueous combination.

Embodiment 27. The method of Embodiment 26, wherein the aqueous pharmaceutical combination is administered by intragastric, subcutaneous, intramuscular, intranasal, intrajejunum, oral or intravenous administration.

Embodiment 28. The method of Embodiments 26 or 27, wherein the aqueous pharmaceutical combination is administered by subcutaneous administration.

Embodiment 29. A compound corresponding in structure to Formula (I): 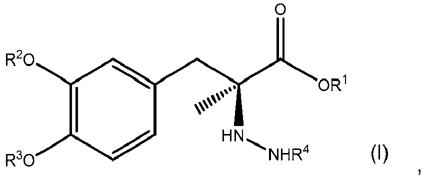 or a pharmaceutically acceptable salt thereof, wherein **R¹** is independently selected from the group consisting of hydrogen, 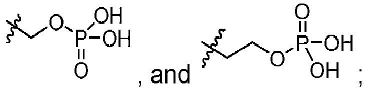 **R²** and **R³** are each independently selected from the group consisting of hydrogen, 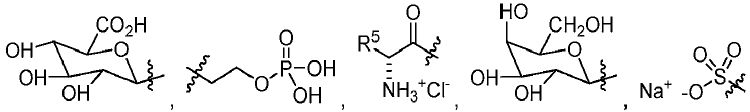 , and 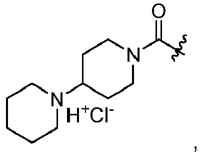 wherein **R⁵** is selected from the group consisting of hydrogen, methyl, and isopropyl; and **R⁴** is independently selected from the group consisting of hydrogen,

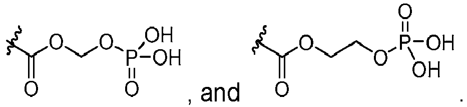

Embodiment 30. The compound or pharmaceutically acceptable salt of Embodiment 29, wherein **R¹** is hydrogen; **R²** and **R³** are each independently selected from the group consisting of hydrogen, 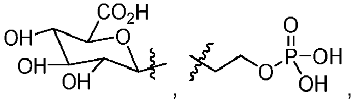 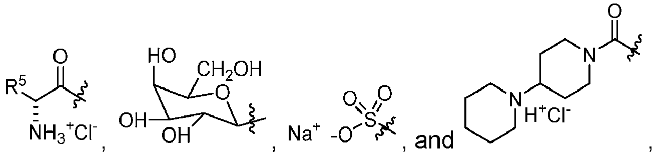 wherein **R⁵** is selected from the group consisting of hydrogen, methyl, and isopropyl; and **R⁴** is independently selected from the group consisting of hydrogen, 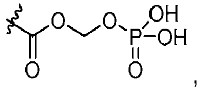 and

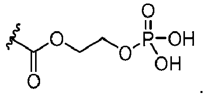

Embodiment 31. The compound or pharmaceutically acceptable salt of Embodiment 29 or 30, wherein **R¹** is hydrogen; **R²** and **R³** are hydrogen; and **R⁴** is independently selected from the group consisting of hydrogen, 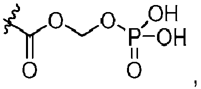 and

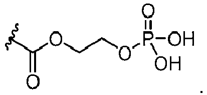

Embodiment 32. The compound or salt of any one of Embodiments 29-31, wherein the compound corresponds in structure to Formula (I-a):

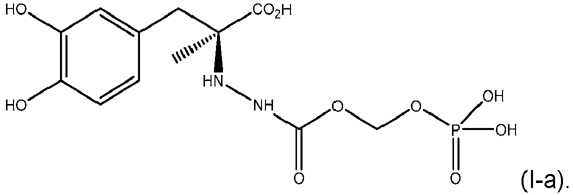

Embodiment 33. The compound or salt of any one of Embodiments 29-31, wherein the compound corresponds in structure to Formula (I-b):

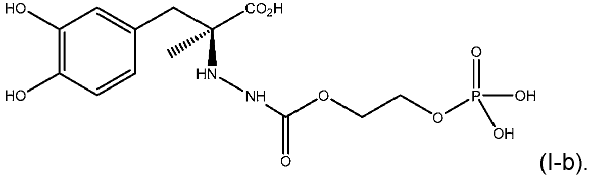

Embodiment 34. A compound corresponding in structure to Formula (II): 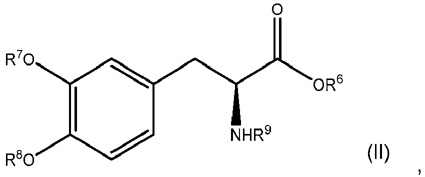 or a pharmaceutically acceptable salt thereof, wherein **R⁶** is independently selected from the group consisting of hydrogen, 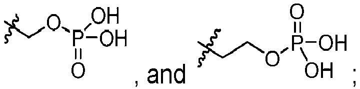 **R⁷** and **R⁸** are each independently selected from the group consisting of hydrogen, 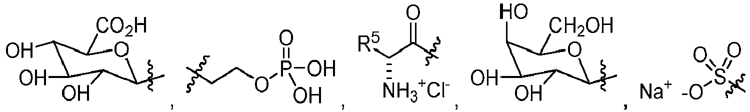 , and 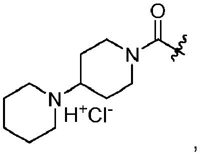 wherein **R⁵** is selected from the group consisting of hydrogen, methyl, and isopropyl; and **R⁹** is independently selected from the group consisting of hydrogen,

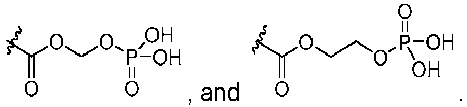

Embodiment 35. The compound or salt of Embodiment 34, wherein **R⁶** is hydrogen; **R⁷** and **R⁸** are each independently selected from the group consisting of hydrogen, 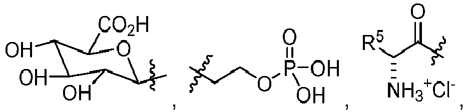 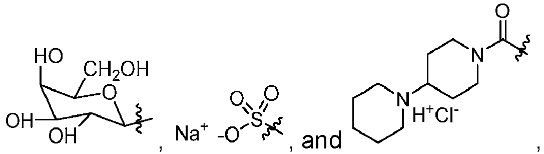 wherein **R⁵** is selected from the group consisting of hydrogen, methyl, and isopropyl; and **R⁹** is independently selected from the group consisting of hydrogen, 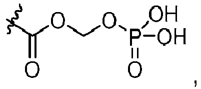 and

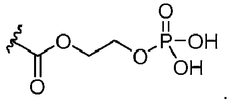

Embodiment 36. The compound or salt of Embodiment 34 or 35, wherein **R⁶** is hydrogen; **R⁷** and **R⁸** are hydrogen; and **R⁹** is independently selected from the group consisting of hydrogen, 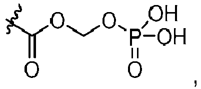 and

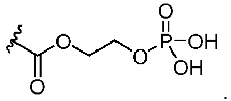

Embodiment 37. The compound or salt of any one of Embodiments 34-36, wherein the compound corresponds in structure to Formula (II-a):

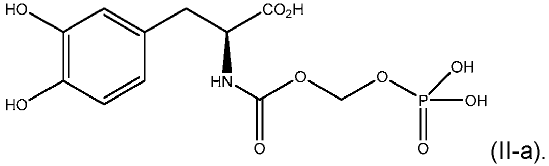

Embodiment 38. The compound or salt of any one of Embodiments 34-36, wherein the compound corresponds in structure to Formula (II-b):

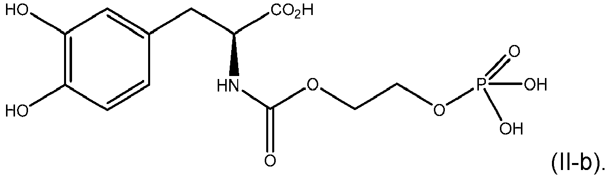

Embodiment 39. A pharmaceutical composition comprising a first compound corresponding in structure to Formula (I): 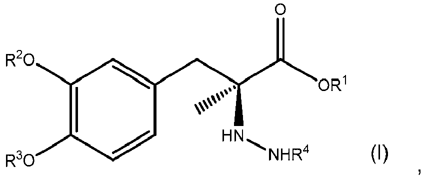 or a pharmaceutically acceptable salt thereof, wherein **R¹** is independently selected from the group consisting of hydrogen, 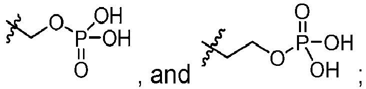 **R²** and **R³** are each independently selected from the group consisting of hydrogen, 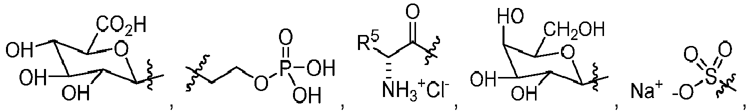 and 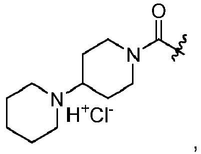 wherein **R⁵** is selected from the group consisting of hydrogen, methyl, and isopropyl; and **R⁴** is independently selected from the group consisting of hydrogen, 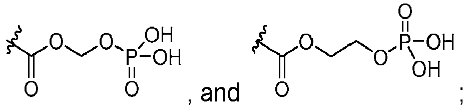 and a pharmaceutically acceptable carrier.

Embodiment 40. The pharmaceutical composition of Embodiment 39, wherein the first compound corresponds in structure to Formula (I-a):

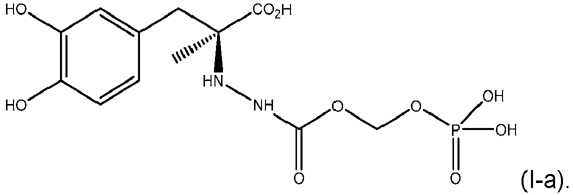

Embodiment 41. The pharmaceutical composition of Embodiment 39, wherein the first compound corresponds in structure to Formula (I-b):

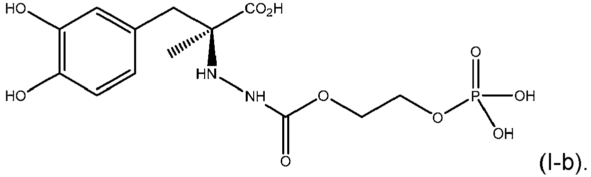

Embodiment 42. The pharmaceutical composition of any one of Embodiments 39-41, wherein the composition further comprises a second compound corresponding in structure to Formula (II): 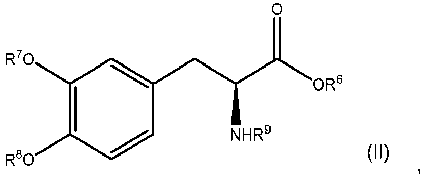 or a pharmaceutically acceptable salt thereof, wherein **R⁶** is independently selected from the group consisting of hydrogen, 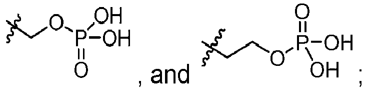 **R⁷** and **R⁸** are each independently selected from the group consisting of hydrogen, 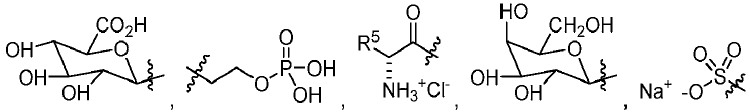 , and 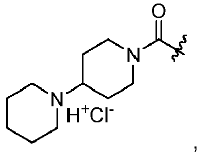 wherein **R⁵** is selected from the group consisting of hydrogen, methyl, and isopropyl; and **R⁹** is independently selected from the group consisting of hydrogen,

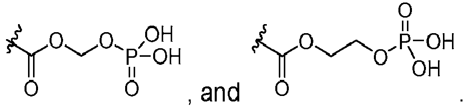

Embodiment 43. The pharmaceutical composition of Embodiment 42, wherein the second compound corresponds in structure to Formula (II-a):

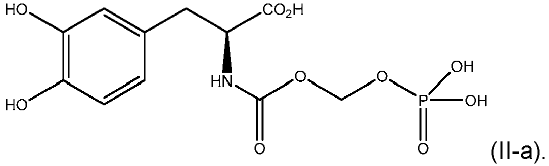

Embodiment 44. The pharmaceutical composition of Embodiment 42, wherein the second compound corresponds in structure to Formula (II-b):

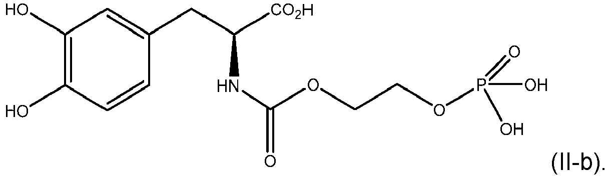

Embodiment 45. A pharmaceutical composition comprising a first compound corresponding in structure to Formula (II): 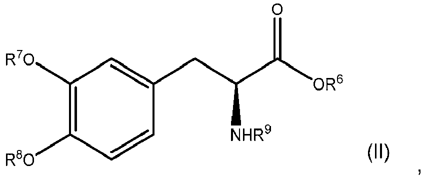 or a pharmaceutically acceptable salt thereof, wherein **R⁶** is independently selected from the group consisting of hydrogen, 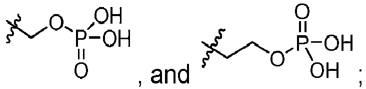 **R⁷** and **R⁸** are each independently selected from the group consisting of hydrogen, 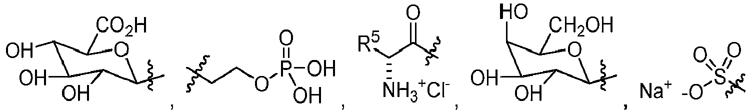 , and 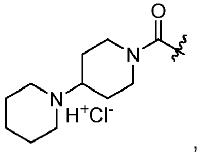 wherein **R⁵** is selected from the group consisting of hydrogen, methyl, and isopropyl; and **R⁹** is independently selected from the group consisting of hydrogen,

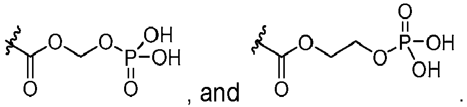

Embodiment 46. The pharmaceutical composition of Embodiment 45, wherein the first compound corresponds in structure to Formula (II-a):

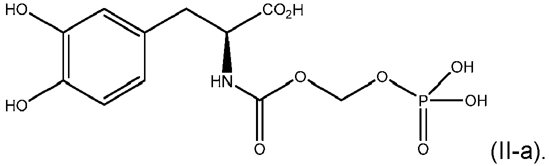

Embodiment 47. The pharmaceutical composition of Embodiment 45, wherein the second compound corresponds in structure to Formula (II-b):

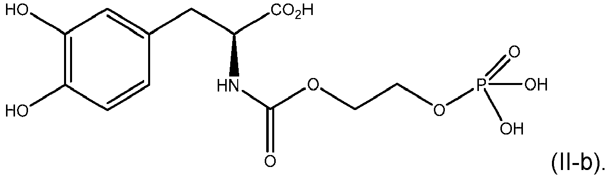

Embodiment 48. The pharmaceutical composition of any one of Embodiments 45-47, wherein the composition further comprises a second compound corresponding in structure to Formula (I): 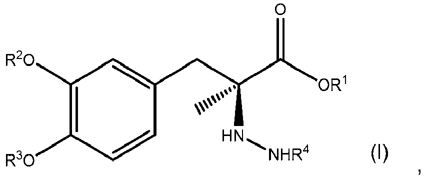 or a pharmaceutically acceptable salt thereof, wherein **R¹** is independently selected from the group consisting of hydrogen, 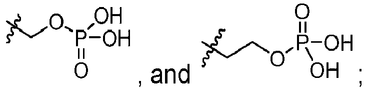 **R²** and **R³** are each independently selected from the group consisting of hydrogen, 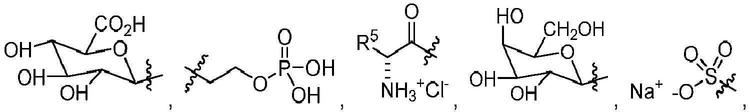 and 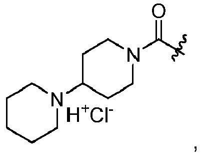 wherein **R⁵** is selected from the group consisting of hydrogen, methyl, and isopropyl; and **R⁴** is independently selected from the group consisting of hydrogen, 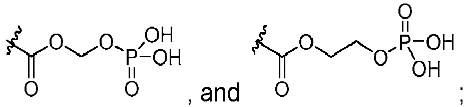 and a pharmaceutically acceptable carrier.

Embodiment 49. The pharmaceutical composition of Embodiment 48, wherein the second compound corresponds in structure to Formula (I-a):

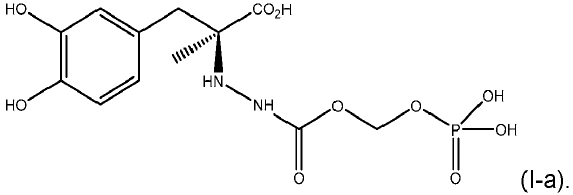

Embodiment 50. The pharmaceutical composition of Embodiment 48, wherein the second compound corresponds in structure to Formula (I-b):

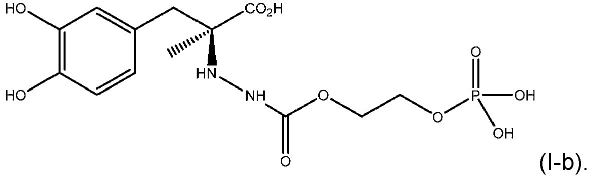

Embodiment 51. The pharmaceutical composition of any one of Embodiments 39-44, wherein the weight ratio of the first compound to the second compound is from about 1:1 to about 1:50, preferably from about 1:2 to about 1:15, even more preferably from about 1:4 to about 1:10.

Embodiment 52. The pharmaceutical composition of any one of Embodiments 39-51, wherein the weight ratio of the first compound to the second compound is about 1:4.

Embodiment 53. The pharmaceutical composition of any one of Embodiments 39-51, wherein the weight ratio of the first compound to the second compound is about 1:7.5.

Embodiment 54. The pharmaceutical composition of any one of Embodiments 39-51, wherein the weight ratio of the first compound to the second compound is about 1:10.

Embodiment 55. The pharmaceutical composition of any one of Embodiments 39-54, wherein the composition further comprises water and is suitable for infusion.

Embodiment 56. A kit comprising the pharmaceutical combination of any one of Embodiments 1-12.

Embodiment 57. A kit comprising the pharmaceutical composition of any one of Embodiments 39-55.

It is understood that the foregoing detailed description and accompanying examples are merely illustrative and are not to be taken as limitations upon the scope of the invention, which is defined solely by the appended claims and their equivalents.

Various changes and modifications to the disclosed embodiments will be apparent to those skilled in the art. Such changes and modifications, including without limitation those relating to the chemical structures, substituents, derivatives, intermediates, syntheses, compositions, formulations, or methods of use of the invention, may be made without departing from the spirit and scope thereof.

Further embodiments are set out in the following numbered items:
1. A pharmaceutical combination comprising a first compound corresponding in structure to Formula (I): 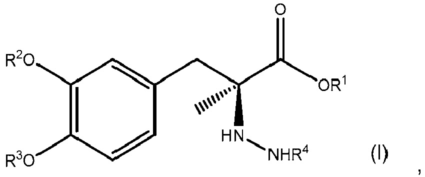 or a pharmaceutically acceptable salt thereof, wherein
   **R¹** is independently selected from the group consisting of hydrogen, 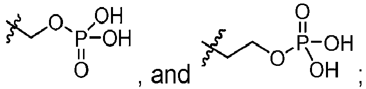
   R**²** and **R³** are each independently selected from the group consisting of hydrogen, 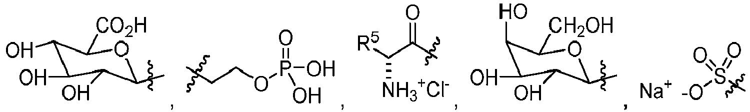 , and 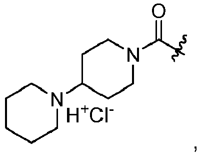 wherein **R⁵** is selected from the group consisting of hydrogen, methyl, and isopropyl; and
   **R⁴** is independently selected from the group consisting of hydrogen, 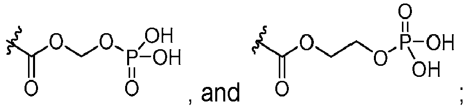 and
   a second compound corresponding in structure Formula (II): 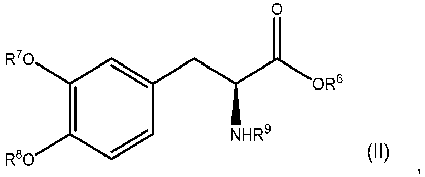 or a pharmaceutically acceptable salt thereof, wherein
   **R⁶** is independently selected from the group consisting of hydrogen, 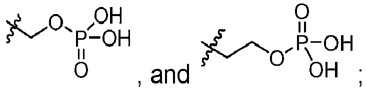
   **R⁷** and **R⁸** are each independently selected from the group consisting of hydrogen, 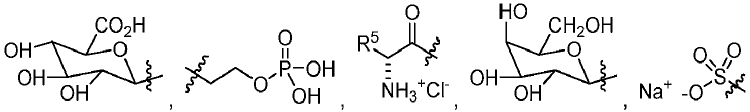 , and 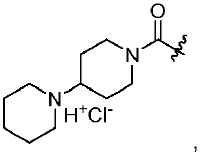 wherein **R⁵** is selected from the group consisting of hydrogen, methyl, and isopropyl; and
   **R⁹** is independently selected from the group consisting of hydrogen, 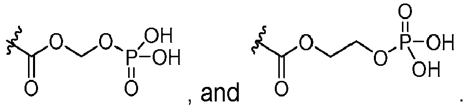
2. The pharmaceutical combination of item 1, wherein the first compound is 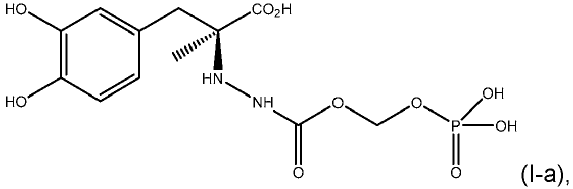 or 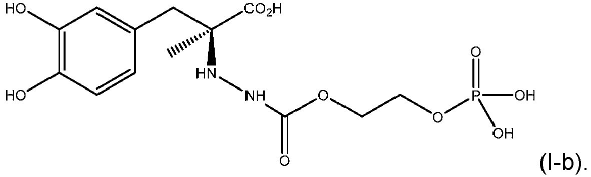
3. The pharmaceutical combination of item 1 or 2, wherein the second compound is 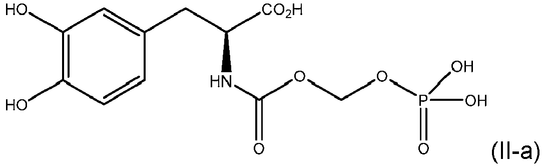 or 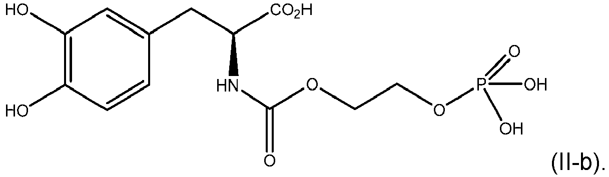
4. The pharmaceutical combination of any one of items 1-3, wherein the first compound or pharmaceutically acceptable salt thereof, and the second compound or pharmaceutically acceptable salt thereof are present in separate pharmaceutical compositions or are both present in the same pharmaceutical composition.
5. The pharmaceutical combination of any one of items 1-4, wherein a weight ratio of the first compound or pharmaceutically acceptable salt thereof to the second compound or pharmaceutically acceptable salt thereof is about 1:1 to about 1:50, preferably about 1:2 to about 1:15, preferably about 1:4 to about 1:10, and more preferably about 1:4.
6. The pharmaceutical combination of any one of items 1-5, wherein the first compound or pharmaceutically acceptable salt thereof has a solubility of at least about 200 mg/ml in aqueous solution at about neutral pH, and the second compound or pharmaceutically acceptable salt thereof has a solubility of at least about 400 mg/ml in aqueous solution at about neutral pH.
7. The pharmaceutical combination of any one of items 1-6, wherein the combination is an aqueous combination suitable for intragastric, subcutaneous, intramuscular, intrajejunum, oral, intranasal or intravenous administration.
8. The pharmaceutical combination of any one of items 1-7, wherein the combination is an aqueous combination suitable for subcutaneous administration.
9. The pharmaceutical combination of any one of items 1-8, wherein the first compound is a compound corresponding in structure to Formula (I-a): 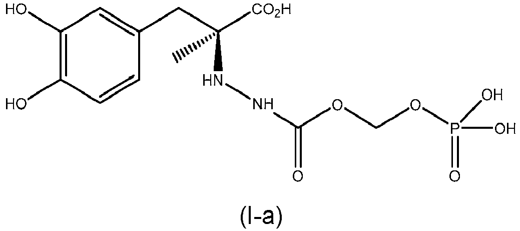 or a pharmaceutically acceptable salt thereof; and the second compound is a compound corresponding in structure to Formula (II-a): 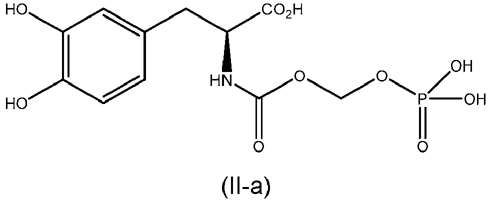 or a pharmaceutically acceptable salt thereof.
10. The pharmaceutical combination of any one of items 1-9, wherein the first compound is a compound corresponding in structure to Formula (I-a): 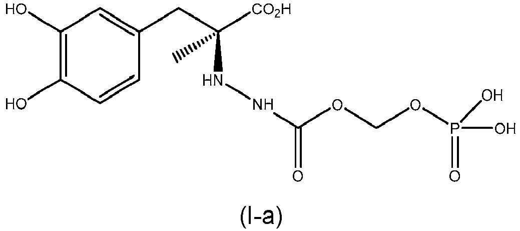 or a pharmaceutically acceptable salt thereof; and the second compound is a compound corresponding in structure to Formula (II-b): 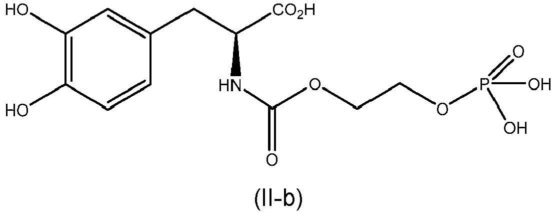 or a pharmaceutically acceptable salt thereof.
11. The pharmaceutical combination of any one of items 1-10, wherein the first compound is a compound corresponding in structure to Formula (I-b): 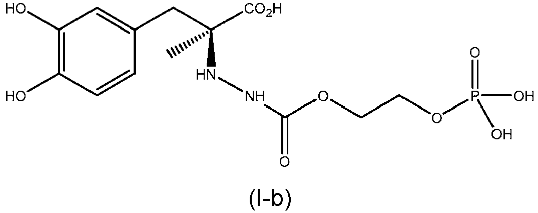 or a pharmaceutically acceptable salt thereof; and the second compound is a compound corresponding in structure to Formula (II-a): 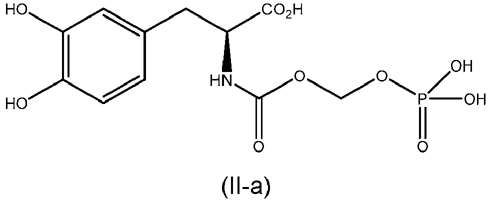 or a pharmaceutically acceptable salt thereof.
12. The pharmaceutical combination of any one of items 1-11, wherein the first compound is a compound corresponding in structure to Formula (I-b): 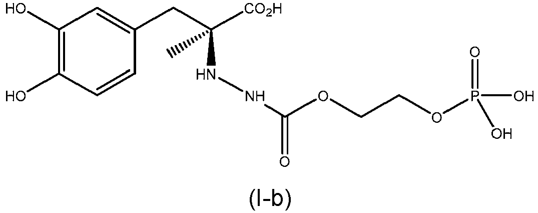 or a pharmaceutically acceptable salt thereof; and the second compound is a compound corresponding in structure to Formula (II-b): 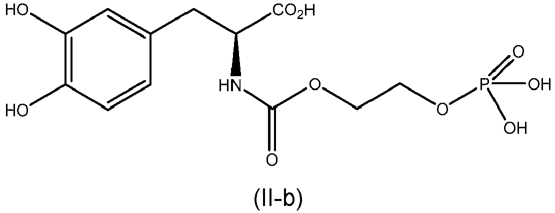 or a pharmaceutically acceptable salt thereof.
13. A method of treating Parkinson's disease in a subject in need thereof and/or a method of providing rescue therapy in a subject having Parkinson's disease, the method comprising administering to the subject a therapeutically effective amount of the pharmaceutical combination according to any one of items 1-12.
14. The method of item 13, wherein the first compound and the second compound are administered in separate pharmaceutical compositions to the subject, or the first compound and the second compound are administered to the subject in the same pharmaceutical composition comprising the first compound and the second compound.
15. The method of item 13 or 14, wherein the method comprises intragastric, subcutaneous, intrajejunum, oral, intranasal, intramuscular or intravenous administration of the first compound and the second compound.
16. The method of any one of items 13-15, wherein the method comprises subcutaneous administration of the first compound and the second compound.
17. The method of any one of items 13-16, wherein the method comprises substantially continuous administration of the first compound and the second compound over a period of at least about 12 hours.
18. The method of any one items 13-17, wherein the weight ratio of the first compound administered to the second compound administered is from about 1:1 to about 1:50.
19. The method of any one of items 13-18, wherein the weight ratio of the first compound administered to the second compound administered is from about 1:2 to about 1:15.
20. The method of any one of items 13-19, wherein the weight ratio of the first compound administered to the second compound administered is from about 1:4 to about 1:10.
21. The method of any one of items 13-20, wherein the weight ratio of the first compound administered to the second compound administered is about 1:4.
22. The method of any one of items 13-21, wherein the weight ratio of the first compound administered to the second compound administered is about 1:7.5.
23. The method of any one of items 13-22, wherein the weight ratio of the first compound administered to the second compound administered is about 1:10.
24. The method of any one of items 13-23, wherein the first compound is selected from the group consisting of 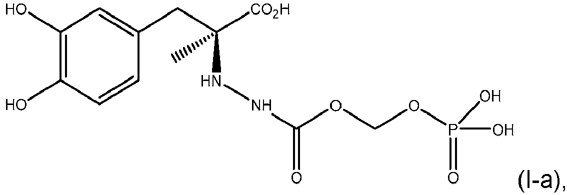 or 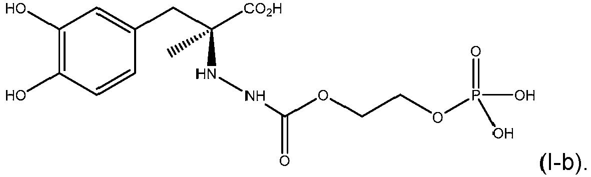 and the second compound is selected from the group consisting of 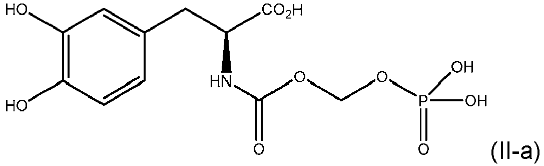 or 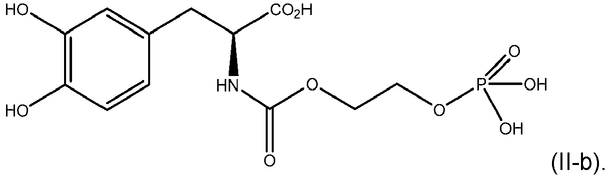
25. The method of any one of items 13-24 further comprising administering another anti-Parkinson's agent to the subject.
26. The method of any one of items 13-25, wherein the pharmaceutical combination is an aqueous combination.
27. The method of item 26, wherein the aqueous pharmaceutical combination is administered by intragastric, subcutaneous, intramuscular, intranasal, intrajejunum, oral or intravenous administration.
28. The method of items 26 or 27, wherein the aqueous pharmaceutical combination is administered by subcutaneous administration.
29. A compound corresponding in structure to Formula (I): 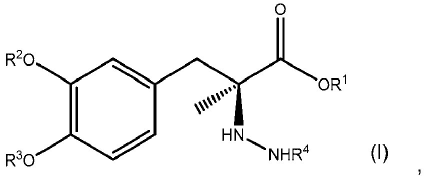 or a pharmaceutically acceptable salt thereof, wherein
   **R¹** is independently selected from the group consisting of hydrogen, 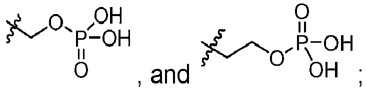
   R² and **R³** are each independently selected from the group consisting of hydrogen, 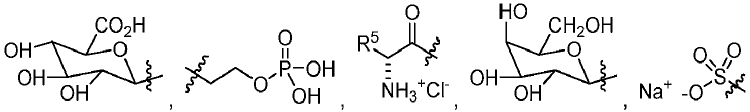 , and 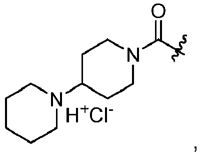 wherein **R⁵** is selected from the group consisting of hydrogen, methyl, and isopropyl; and
   **R⁴** is independently selected from the group consisting of hydrogen, 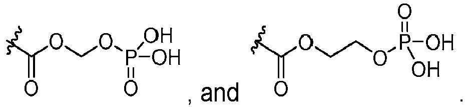
30. The compound or pharmaceutically acceptable salt of item 29, wherein **R¹** is hydrogen; **R²** and **R**³ are each independently selected from the group consisting of hydrogen, 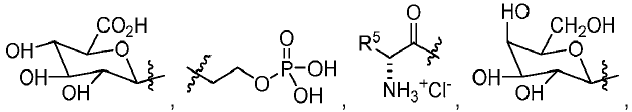 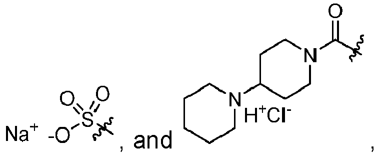 wherein **R⁵** is selected from the group consisting of hydrogen, methyl, and isopropyl; and **R⁴** is independently selected from the group consisting of hydrogen, 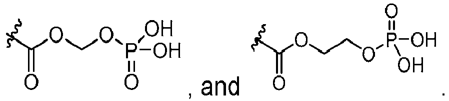
31. The compound or pharmaceutically acceptable salt of item 29 or 30, wherein **R¹** is hydrogen; **R²** and **R³** are hydrogen; and **R⁴** is independently selected from the group consisting of hydrogen, 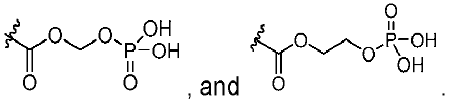
32. The compound or salt of any one of items 29-31, wherein the compound corresponds in structure to Formula (I-a): 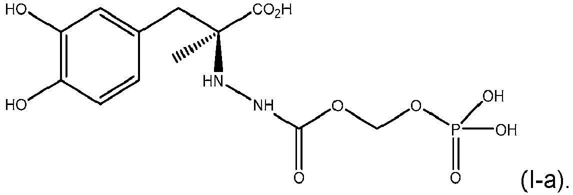
33. The compound or salt of any one of items 29-31, wherein the compound corresponds in structure to Formula (I-b): 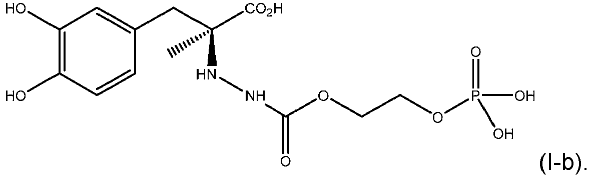
34. A compound corresponding in structure to Formula (II): 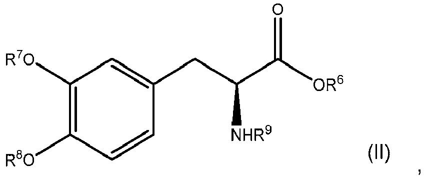 or a pharmaceutically acceptable salt thereof, wherein
   **R⁶** is independently selected from the group consisting of hydrogen, 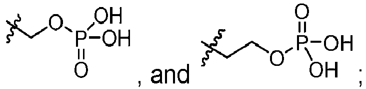
   **R⁷** and **R⁸** are each independently selected from the group consisting of hydrogen, 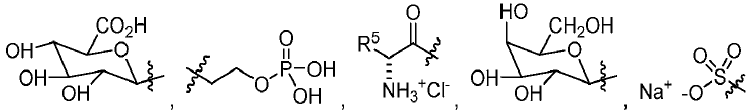 , and 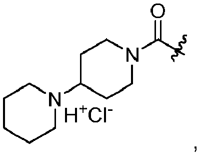 wherein **R⁵** is selected from the group consisting of hydrogen, methyl, and isopropyl; and
   **R⁹** is independently selected from the group consisting of hydrogen, 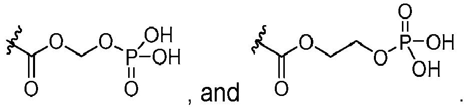
35. The compound or salt of item 34, wherein **R⁶** is hydrogen; **R⁷** and **R⁸** are each independently selected from the group consisting of hydrogen, 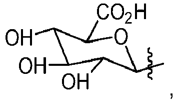 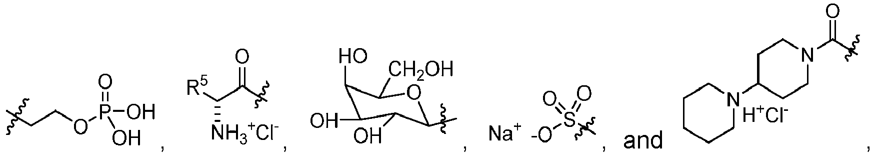 wherein **R⁵** is selected from the group consisting of hydrogen, methyl, and isopropyl; and **R⁹** is independently selected from the group consisting of hydrogen, 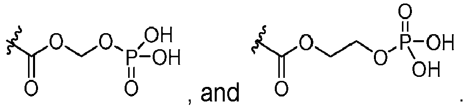
36. The compound or salt of item 34 or 35, wherein **R⁶** is hydrogen; **R⁷** and **R⁸** are hydrogen; and **R⁹** is independently selected from the group consisting of hydrogen, 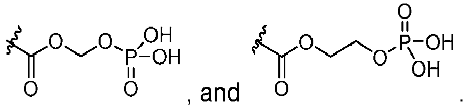
37. The compound or salt of any one of items 34-36, wherein the compound corresponds in structure to Formula (II-a): 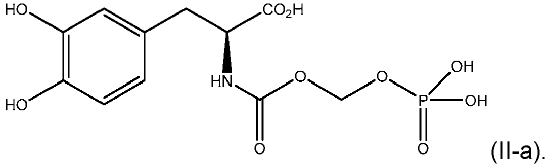
38. The compound or salt of any one of items 34-36, wherein the compound corresponds in structure to Formula (II-b): 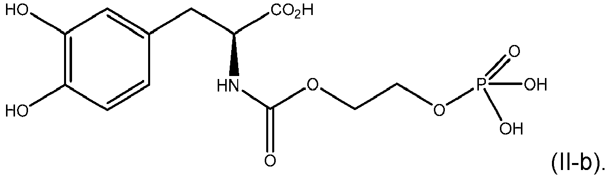
39. A pharmaceutical composition comprising a first compound corresponding in structure to Formula (I): 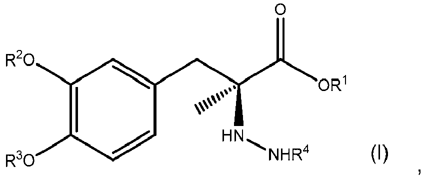 or a pharmaceutically acceptable salt thereof, wherein
   **R¹** is independently selected from the group consisting of hydrogen, 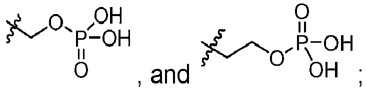
   **R²** and **R³** are each independently selected from the group consisting of hydrogen, 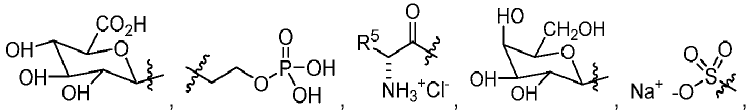 and 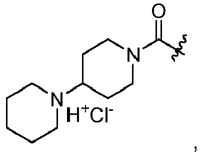 wherein **R⁵** is selected from the group consisting of hydrogen, methyl, and isopropyl; and
   **R⁴** is independently selected from the group consisting of hydrogen, 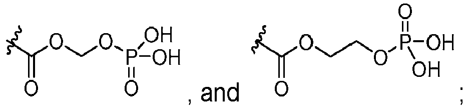 and a pharmaceutically acceptable carrier.
40. The pharmaceutical composition of item 39, wherein the first compound corresponds in structure to Formula (I-a): 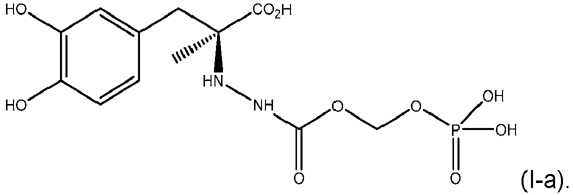
41. The pharmaceutical composition of item 39, wherein the first compound corresponds in structure to Formula (I-b): 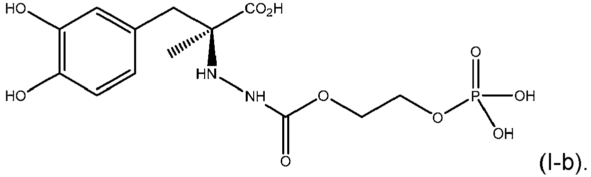
42. The pharmaceutical composition of any one of items 39-41, wherein the composition further comprises a second compound corresponding in structure to Formula (II): 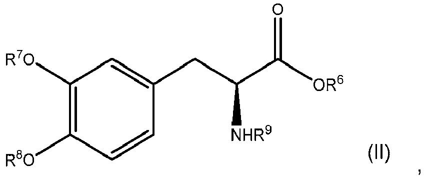 or a pharmaceutically acceptable salt thereof, wherein
   **R⁶** is independently selected from the group consisting of hydrogen, 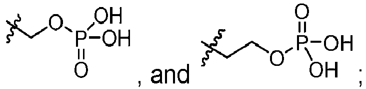
   **R⁷** and **R⁸** are each independently selected from the group consisting of hydrogen, 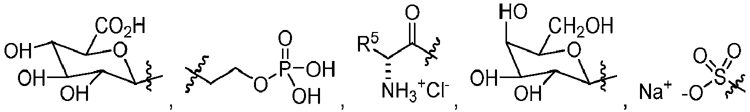 , and 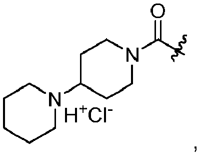 items wherein **R⁵** is selected from the group consisting of hydrogen, methyl, and isopropyl; and
   **R⁹** is independently selected from the group consisting of hydrogen, 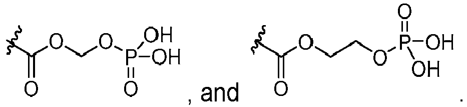
43. The pharmaceutical composition of item 42, wherein the second compound corresponds in structure to Formula (II-a): 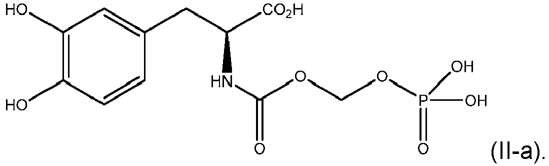
44. The pharmaceutical composition of item 42, wherein the second compound corresponds in structure to Formula (II-b): 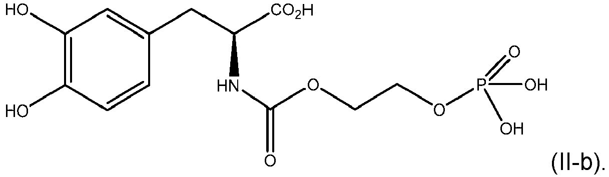
45. A pharmaceutical composition comprising a first compound corresponding in structure to Formula (II): 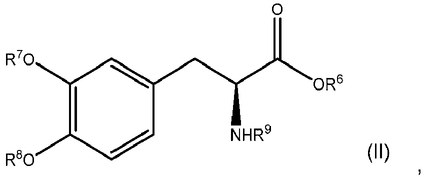 or a pharmaceutically acceptable salt thereof, wherein
   **R⁶** is independently selected from the group consisting of hydrogen, 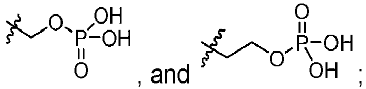
   **R⁷** and **R⁸** are each independently selected from the group consisting of hydrogen, 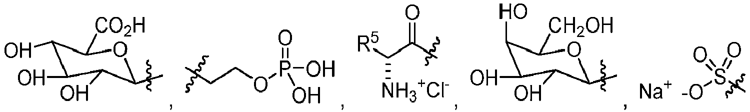 , and 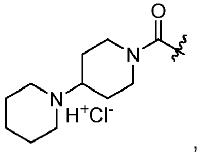 wherein **R⁵** is selected from the group consisting of hydrogen, methyl, and isopropyl; and
   **R⁹** is independently selected from the group consisting of hydrogen, 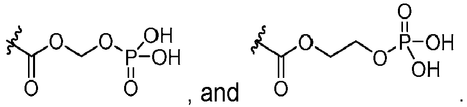
46. The pharmaceutical composition of item 45, wherein the first compound corresponds in structure to Formula (II-a): 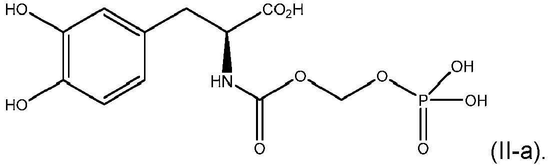
47. The pharmaceutical composition of item 45, wherein the second compound corresponds in structure to Formula (II-b): 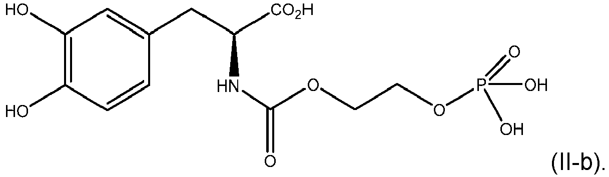
48. The pharmaceutical composition of any one of items 45-47, wherein the composition further comprises a second compound corresponding in structure to Formula (I): 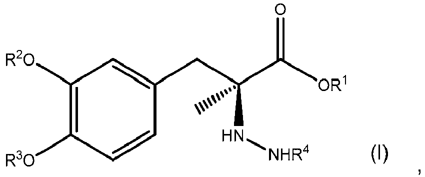 or a pharmaceutically acceptable salt thereof, wherein
   **R¹** is independently selected from the group consisting of hydrogen, 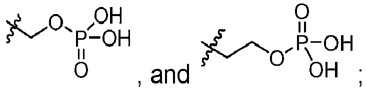
   **R**² and **R³** are each independently selected from the group consisting of hydrogen, 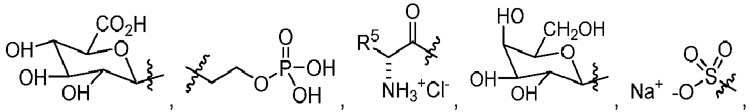 and 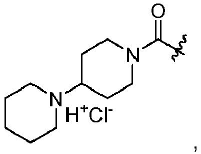 wherein **R⁵** is selected from the group consisting of hydrogen, methyl, and isopropyl; and
   **R⁴** is independently selected from the group consisting of hydrogen, 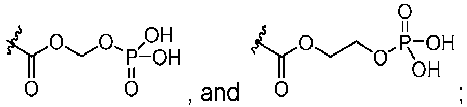 and a pharmaceutically acceptable carrier.
49. The pharmaceutical composition of item 48, wherein the second compound corresponds in structure to Formula (I-a): 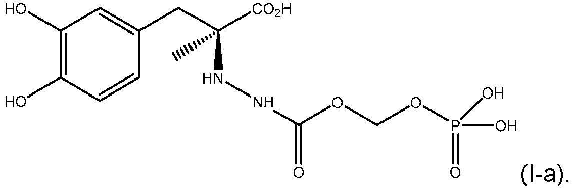
50. The pharmaceutical composition of item 48, wherein the second compound corresponds in structure to Formula (I-b): 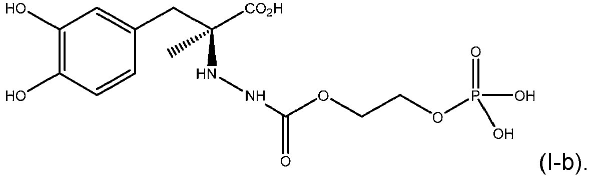
51. The pharmaceutical composition of any one of items 39-44, wherein the weight ratio of the first compound to the second compound is from about 1:1 to about 1:50, preferably from about 1:2 to about 1:15, even more preferably from about 1:4 to about 1:10.
52. The pharmaceutical composition of any one of items 39-51, wherein the weight ratio of the first compound to the second compound is about 1:4.
53. The pharmaceutical composition of any one of items 39-51, wherein the weight ratio of the first compound to the second compound is about 1:7.5.
54. The pharmaceutical composition of any one of items 39-51, wherein the weight ratio of the first compound to the second compound is about 1:10.
55. The pharmaceutical composition of any one of items 39-54, wherein the composition further comprises water and is suitable for infusion.
56. A kit comprising the pharmaceutical combination of any one of items 1-12.
57. A kit comprising the pharmaceutical composition of any one of items 39-55.

## Claims

1. A pharmaceutical combination comprising a first compound corresponding in structure to Formula (I): 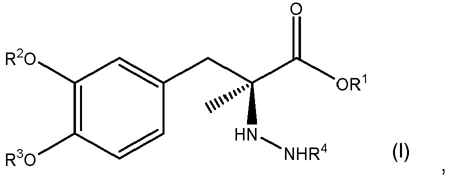 or a pharmaceutically acceptable salt thereof, wherein
**R¹** is independently selected from the group consisting of hydrogen, 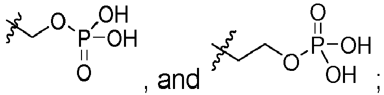
**R**² and **R³** are each independently selected from the group consisting of hydrogen, 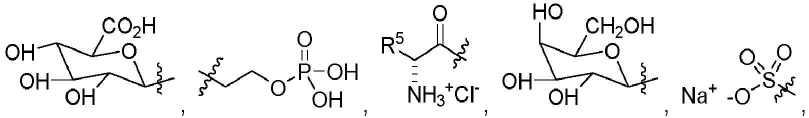 and 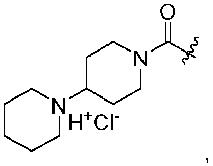 wherein **R⁵** is selected from the group consisting of hydrogen, methyl, and isopropyl; and
**R⁴** is independently selected from the group consisting of hydrogen, 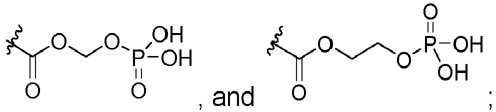 and
a second compound corresponding in structure Formula (II): 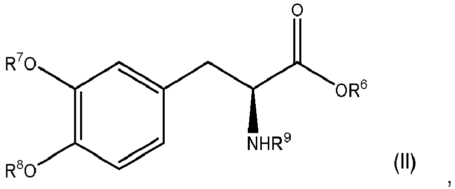 or a pharmaceutically acceptable salt thereof, wherein
**R⁶** is independently selected from the group consisting of hydrogen, 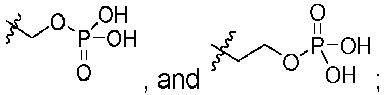
**R**⁷ and **R⁸** are each independently selected from the group consisting of hydrogen, 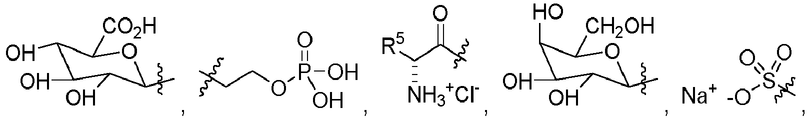 and 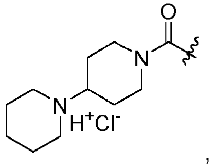 wherein **R⁵** is selected from the group consisting of hydrogen, methyl, and isopropyl; and
**R⁹** is independently selected from the group consisting of hydrogen,

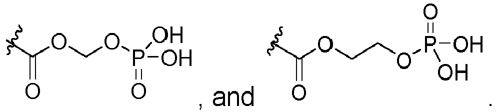

2. The pharmaceutical combination of Claim 1, wherein the first compound is 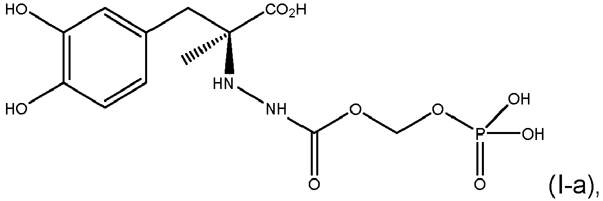 or

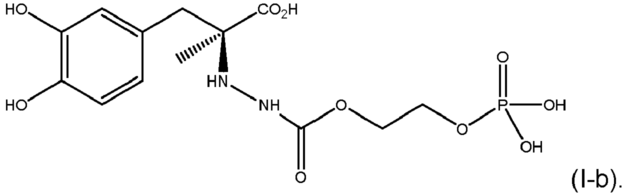

3. The pharmaceutical combination of Claim 1 or 2, wherein the second compound is 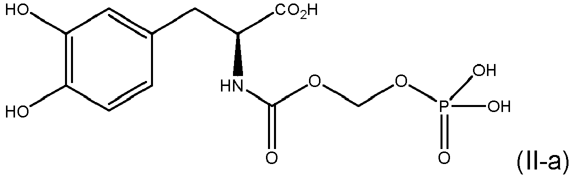 or

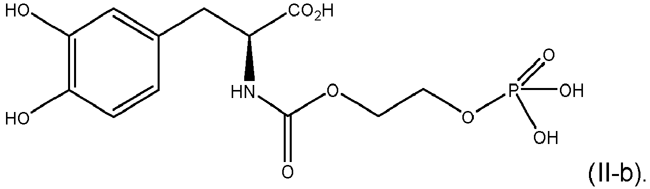

4. The pharmaceutical combination of any one of Claims 1-3, wherein the first compound or pharmaceutically acceptable salt thereof, and the second compound or pharmaceutically acceptable salt thereof are present in separate pharmaceutical compositions or are both present in the same pharmaceutical composition.

5. The pharmaceutical combination of any one of Claims 1-4, wherein a weight ratio of the first compound or pharmaceutically acceptable salt thereof to the second compound or pharmaceutically acceptable salt thereof is about 1:1 to about 1:50, preferably about 1:2 to about 1:15, preferably about 1:4 to about 1:10, and more preferably about 1:4.

6. The pharmaceutical combination of any one of Claims 1-5, wherein the first compound or pharmaceutically acceptable salt thereof has a solubility of at least about 200 mg/ml in aqueous solution at about neutral pH, and the second compound or pharmaceutically acceptable salt thereof has a solubility of at least about 400 mg/ml in aqueous solution at about neutral pH.

7. The pharmaceutical combination of any one of Claims 1-6, wherein the combination is an aqueous combination suitable for intragastric, subcutaneous, intramuscular, intrajejunum, oral, intranasal or intravenous administration.

8. The pharmaceutical combination of any one of Claims 1-7, wherein the combination is an aqueous combination suitable for subcutaneous administration.

9. The pharmaceutical combination of any one of Claims 1-8, wherein the first compound is a compound corresponding in structure to Formula (I-a): 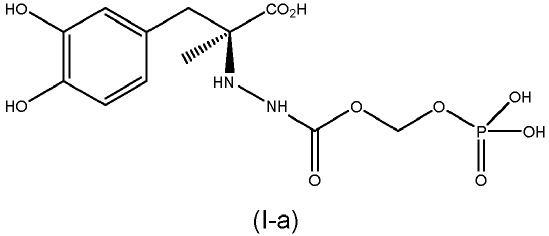 or a pharmaceutically acceptable salt thereof; and the second compound is a compound corresponding in structure to Formula (II-a): 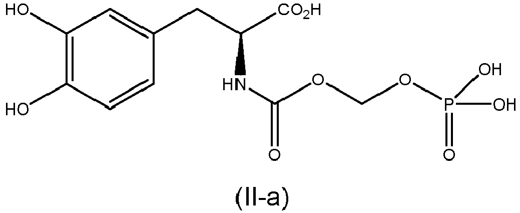 or a pharmaceutically acceptable salt thereof.

10. The pharmaceutical combination of any one of Claims 1-9, wherein the first compound is a compound corresponding in structure to Formula (I-a): 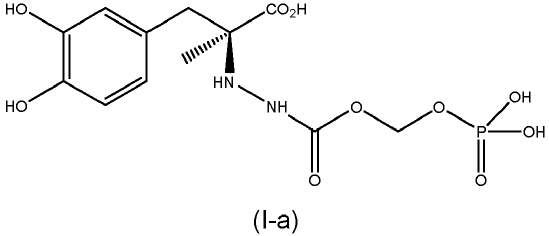 or a pharmaceutically acceptable salt thereof; and the second compound is a compound corresponding in structure to Formula (II-b): 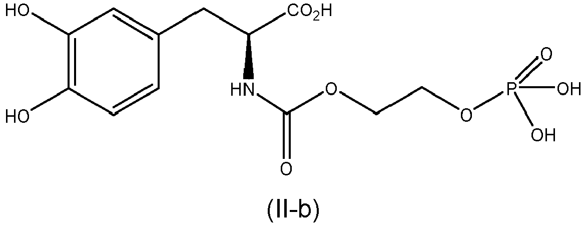 or a pharmaceutically acceptable salt thereof.

11. The pharmaceutical combination of any one of Claims 1-10, wherein the first compound is a compound corresponding in structure to Formula (I-b): 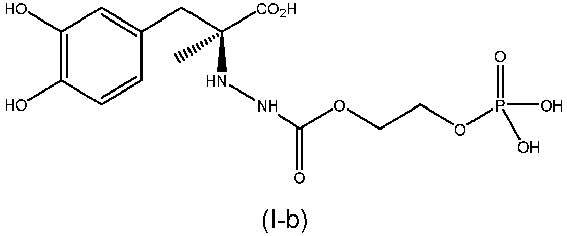 or a pharmaceutically acceptable salt thereof; and the second compound is a compound corresponding in structure to Formula (II-a): 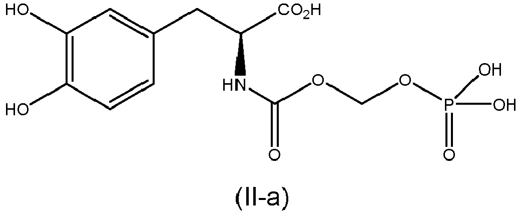 or a pharmaceutically acceptable salt thereof.

12. The pharmaceutical combination of any one of Claims 1-11, wherein the first compound is a compound corresponding in structure to Formula (I-b): 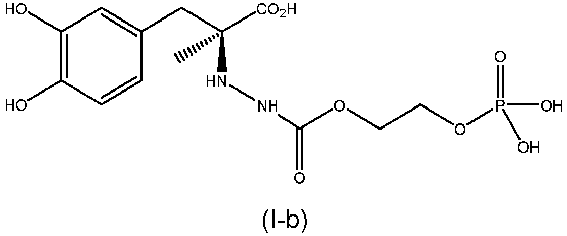 or a pharmaceutically acceptable salt thereof; and the second compound is a compound corresponding in structure to Formula (II-b): 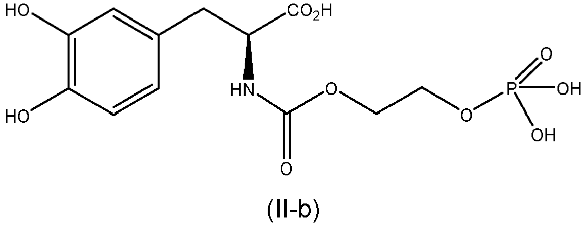 or a pharmaceutically acceptable salt thereof.
